# EUROPÄISCHE PATENTANMELDUNG

(11) **EP 4 091 599 A1**
(43) Veröffentlichungstag der Anmeldung: **23.11.2022**
(21) Anmeldenummer: 21174453.7
(22) Anmeldetag: 18.05.2021
(51) Int. Cl.: A61K 6/891, A61K 31/66, A61L 24/00, C07F 9/38, C08G 61/08, A61L 27/18

(54) **POLYMERISIERBARE ZUSAMMENSETZUNG AUF THIOL-EN-BASIS**

(71) Anmelder: Karl Leibinger Medizintechnik GmbH & Co. KG, 78570 Mühlheim/Donau (DE)
(72) Erfinder: Steinbauer, Patrick, 3910 Zwettl (AT); Baudis, Stefan, 1230 Wien (AT); Reinauer, Frank, 78576 Emmingen-Liptingen (DE); Wolfram, Tobias, 63303 Dreieich (DE)
(74) Vertreter: Ellmeyer, Wolfgang

(57) **Zusammenfassung**

Die vorliegende Erfindung betrifft die Verwendung von Verbindungen, die pro Molekül jeweils zumindest eine polymerisierbare C-C-Doppelbindung, zumindest eine haftungsvermittelnde Gruppierung und einen dazwischen liegenden zumindest zweiwertigen Spacer aufweisen, als Primer in einer polymerisierbaren Zusammensetzung auf Thiol-En-Basis, die weiters zumindest einen radikalischen Initiator, Monomere mit zumindest zwei polymerisierbaren C-C-Doppelbindungen pro Molekül und Polythiole mit zumindest zwei SH-Gruppen pro Molekül umfasst, mit dem Kennzeichen, dass die Primer als polymerisierbare Doppelbindungen 5-Norbornen-2-yl-Gruppen und aus Phosphonsäure-Gruppen und 3,4-Dihydroxyphenyl-Gruppen ausgewählte Gruppen als haftungsvermittelnde Gruppierungen umfassen; entsprechende Zusammensetzungen auf Thiol-En-Basis; entsprechende, für diesen Zweck geeignete Norbornen-Derivate; sowie konkrete, erstmalig synthetisierte Vertreter solcher Norbornen-Derivate.

## Beschreibung

Die vorliegende Erfindung betrifft neue polymerisierbare Zusammensetzungen auf Thiol-En-Basis sowie technische und medizinische Verwendungen dafür, z.B. in Dentalmaterialien oder als Materialien für die Osteosynthese, insbesondere als Knochenkleber. Weiters betrifft sie neue Norbornen-Derivate und deren Verwendung als Primer in solchen polymerisierbaren Zusammensetzungen.

### STAND DER TECHNIK

Trotz der unter den Wirbeltiergeweben einzigartigen Fähigkeit von Knochen zur Heilung durch Knochenneubildung kann dieser Vorgang nach schweren Schädigungen oder Erkrankungen nicht durchgeführt werden. Schwere Knochenbrüche oder -verletzungen aufgrund von Alter, Verkehrsunfällen, Knochentumorresektion usw. sind schwerwiegende Probleme in der Orthopädie, die zusätzliche chirurgische Eingriffe erfordern, um die Knochen zu fixieren und eine adäquate Heilung zu ermöglichen. Statistisch gesehen wird jeder zweite Mensch im Laufe seines Lebens mit einer Knochenfraktur konfrontiert. Sowohl die klassische als auch die chirurgische Frakturbehandlung kann jedoch immer zu Komplikationen oder Problemen mit der mechanischen Stabilität führen, weswegen ein dringender Bedarf an Knochen- und Gewebeklebern besteht (Heiss et al., Eur. J. Trauma 32, 141-148 (2006)).

Neben besonders im Vergleich zu Metallimplantaten vorteilhaften Eigenschaften von Klebstoffen bei der Fixierung von Knochenfrakturen, wie z.B. breiterer Anwendbarkeit, gleichmäßigerer Kräfteverteilung auf die Frakturfläche und besserer Fixierung im spongiösen Teil des Knochens, bereiten Knochenkleber doch bestimmte Schwierigkeiten, die von speziellen Anforderungen an Knochenkleber sowie unterschiedlichen Anwendungsgebieten herrühren. Einerseits müssen die polymerisierbaren Zusammensetzungen von Knochenklebern nichttoxisch und biokompatibel sein, um für die klinische Anwendung in der Trauma- und orthopädischen Chirurgie geeignet zu sein. Andererseits sollten sie das umliegende Gewebe nicht reizen und nach einer vorbestimmten Zeitspanne biologisch abbaubar sein, und die Wärmeentwicklung während der Polymerisation muss minimal sein. Zusätzlich muss der Klebstoff in feuchter Umgebung binden, weswegen eine hohe Haftfestigkeit in situ wichtig ist. Darüber hinaus sind einfache Herstellung, Praktikabilität und Anwendbarkeit, minimale Schrumpfung nach der Härtung, Lagerstabilität und Effizienz relevante Faktoren.

Im Handel erhältlich ist beispielsweise OsStic^{™} von PBC Biomed, ein biomimetischer Klebstoff für die Knochenreparatur, der aus Tricalciumphosphat und Phosphoserin besteht. Wässrige Lösungen dieser beiden Komponenten mit Silicat als zusätzlicher Komponente können auch als Weichgewebeklebstoffe eingesetzt werden (siehe z.B. WO 2018/060289 A1, US 2020/038545 A1). Diese injizierbaren Biokeramiken zeigten eine höhere Scherhaftfestigkeit ("shear bond strength", SBS) (2,5-4 MPa) als kommerzielle Cyanoacrylate (Pujari-Palmer et al., Materials 11(12), 2492 (2018)). Tetranite^{™} von LaunchPad Medical ist ein synthetischer, injizierbarer, biomimetischer und resorbierbarer Klebstoff auf Basis einer Kombination von Tetracalciumphosphat und Phosphoserin, der osteopromotive Eigenschaften aufweist und auf Knochen oder Metallen haftet (WO 2010/056811 A1). Er weist zudem eine 7,5-mal höhere Scherhaftfestigkeit (3±2 MPa) auf als nicht-resorbierbarer Poly(methylmethacrylat)-Knochenzement (Kirillova et al., Adv. Healthc. Mater. 7(17), 1800467 (2018)).

Aufgrund der Unfähigkeit biokompatibler und biologisch abbaubarer Matrixmaterialien, eine direkte Verbindung mit hartem Gewebe einzugehen, enthalten Kleberformulierungen häufig Haftvermittler- oder "Primer"-Moleküle. Diese sind von enormer Bedeutung für die schlussendliche Haftfestigkeit an feuchten Knochen und müssen daher auch gut in die Klebermatrix integriert sein. Das Primer-Molekül besteht in der Regel aus einer polymerisierbaren Gruppe (PG), die zur Reaktion mit anderen Monomeren der polymerisierbaren Zusammensetzung und zur Copolymerisation mit dem Matrixmaterial in der Lage ist, sowie einem Adhäsionsmotiv (AM) als weitere funktionelle Gruppe, die für die Ausbildung primärer chemischer Bindungen (kovalenter oder ionischer Bindungen) verantwortlich ist. In gängigen Dentalrestaurationsmaterialien besteht die PG des Primers aus (Meth)acrylaten und das AM aus Phosphonsäure. Durch die ätzende Wirkung der Phosphonsäure wird eine Reihe von zusätzlichen Effekten erzielt, wie z.B. die Reinigung von Rückständen aus dem Zahnschmelz, eine Vergrößerung der Schmelzoberfläche und die Erzeugung von Mikroporen. Zur Verbindung von PG und AM enthalten die Primer darüber hinaus eine Spacer-Gruppe. Anhand der Länge dieser Gruppe können bestimmte Eigenschaften wie etwa Flüchtigkeit, Löslichkeit, Viskosität, Flexibilität, Quellung und Hydrophilie gesteuert werden (Moszner et al., Dent. Mater. 21(10), 895-910 (2005)).

In der Patentliteratur werden etwa in EP 2.229.930 A1 und DE 10 2004 061 924 A1 Primer-Moleküle für Dentalrestaurationsmaterialien offenbart. Kommerziell erhältliche selbstätzende Zahnschmelz-Dentin-Adhäsive ("self-etching adhesives", SEA) bestehen aus einem selbstätzenden Haftvermittler, vernetzenden Monomeren, monofunktionellen Co-Monomeren und Additiven. Adhese^{®} Universal (Ivoclar Vivadent) aus einem Verbundwerkstoff, Methacrylat-Monomeren mit Methacryloyloxydecyldihydrogenphosphat (MDP) als Haftvermittler sowie Scotchbond^{™} Universal (3M) aus Methacrylaten, einem Silan und MDP als Primer sind weit verbreitete Dentalrestaurationsmaterialien. Diese Haftvermittler zeigten je nach Verbundwerkstoff Scherhaftfestigkeiten zwischen 25 und 28 MPa (Todd et al., Ivoclar Vivadent, Adhese Universal - Scientific Documentation, Schaan, 2015.).

Für das "Tissue Engineering" von Knochen sind (Meth)acrylate aufgrund hoher Zytotoxizität sowie ihrer Abbauprodukte jedoch nicht zu bevorzugen. Als Alternative beschreibt WO 2013/052328 A1 die Thiol-En-Polymerisation mit Vinylestern. Die radikalische Thiol-En-Polymerisation ist von großem Interesse für biomedizinische Anwendungen wie Gewebekleber, Hydrogele und Zahnersatzmaterialien. Der schrittweise Aufbau von Thiol-En-Netzwerken fördert homogene Strukturen, die für die mechanische Robustheit sehr vorteilhaft sind. Trotz einiger Versuche, die radikalische Thiol-En-Polymerisation in Adhäsiven für die Geweberestauration zu nutzen, waren die endgültigen Eigenschaften und die Leistung dieser Systeme bisher allerdings unzureichend. Somit besteht weiterhin ein Bedarf an Adhäsiven für die Knochenfixierung, wobei biomimetische Strategien unter Verwendung von Phosphonaten, Aminen und Catecholen vielversprechende grundlegende Ansätze zeigen (Böker et al., Materials 12(23), 3975 (2019)). Für Thiol-En-Klebstoffe sind der Mangel an mechanischer Steifigkeit und die Adhäsion an nassen Oberflächen auch weiterhin Herausforderungen, die es zu überwinden gilt.

WO 2009/029734 A2 beschreibt verschiedene Zusammensetzungen eines polymeren Knochenzements unter Einsatz von Thiol-En-Chemie (TEC). Die Verwendung des Knochenzements ist jedoch auf die Injektion in Knochenhohlräume beschränkt. WO 2011/048077 A2 offenbart diverse Zusammensetzungen für die Behandlung von Knochenbrüchen unter Einsatz von Thiol-En-Chemie (TEC), wobei als polymerisierbare Gruppen PG verschiedenste C-C-Doppelbindungen enthaltende Gruppen offenbart werden, die aus Vinyl-, Acrylat-, Methacrylat- und Allyl-Gruppen sowie Resten ungesättigter zyklischer Alkene, einschließlich von Norbornenen und N-Maleimiden ausgewählt sind. Als in einem ersten Schritt auf den Knochen aufzutragende Primer werden unter anderem 4-Hydroxy-3-methoxybenzaldehyd, 3,4-Dihydroxyphenethylamin, p-Ethylphenol, p-Vinylphenol, p-Methacrylatphenol, Bicyclo[2.2.1]hept-2-ensäure und diverse Polyhydroxystyrole angeführt.

Die WO 2018/077973 A1 sowie Granskog et al., Adv. Funct. Mater. 28(26), 1800372 (2018), beschreiben die Synthese von Primer-Molekülen und faserverstärkten Klebepflastern ("fiber-reinforced adhesive patch", FRAP) in einem Zwei-Komponenten-Klebersystem unter Verwendung einer Harzformulierung auf TEC-Basis mit Tris[2-(3-mercaptopropionyloxy)ethyl]isocyanurat (TEMPIC) als trifunktionellem Thiol und Triallylisocyanurat (TATATO) als trifunktionellem Monomer. Außerdem wird eine Erhöhung des Biegemoduls durch den Zusatz von Hydroxylapatit als Füllstoff gezeigt. Eine Formulierung mit Primer umfasst 2,2-Bis(allyloxymethyl)propansäure (BAPA), ethoxyliertes Trimethylolpropan-tris(3-mercaptopropionat) (ETTMP) sowie ein Phosphonsäure-hältiges Primer-Molekül, das eine oder zwei jeweils an BAPA gebundene Phosphonsäure-Gruppen umfasst, d.h. ((2,2-Bis(allyloxymethyl)propanamido)methyl)phosphonsäure (hierin als BAPA^{Phn} bezeichnet) oder ((((2,2-Bis(allyloxymethyl)propan-amido)ethyl)azandiyl)-bis(methylen))-bis(phosphonsäure) (hierin BAPA^{bisPhn}). Für eine Matrixformulierung mit Hydroxylapatit in Kombination mit BAPA^{bisPhn} in der Primer-Formulierung wird nach der Härtung auf Knochenmaterial eine Scherhaftfestigkeit von etwa 9 MPa berichtet.

Als Adhäsionsmotiv AM wurden anstatt Phosphonsäure-Gruppen auch verschiedenste Polyole, einschließlich phenolische OH-Gruppen enthaltender Gruppierungen wie etwa Brenzcatechin- oder Catechol-Gruppen in so genannten "biomimetischen Primern" (aufgrund ihrer strukturellen Verwandtschaft mit den Catecholaminen Adrenalin und Dopamin), sowie Amine und Silane eingesetzt, um die Haftung unter anderem unter nassen Bedingungen zu verbessern, wovon Silane häufig als Primer für Metallsubstrate eingesetzt werden. Siehe z.B. WO 2011/048077 A2; Granskog et al., s.o.; und Tomas Romson, "Triazine-based adhesive: An adherence study on clinically used metal surfaces", Dissertation, KTH Königliche Technische Hochschule (Royal Institute of Technology), Stockholm, Schweden, 2018). Bei Verwendung von Brenzcatechin-Gruppen als AM enthaltenden Primer-Molekülen für faserverstärkte Klebepflaster wird in der Literatur ein Scherhaftfestigkeitswert von 0,29 MPa angegeben (Olofsson et al., RSC Adv. 6(31), 26398-26405 (2016)).

All diesen unterschiedlichen Ansätzen und chemischen Konzepten bei der Herstellung von Knochenklebern und der teilweisen Überwindung von Problemen wie geringer Adhäsion an feuchten Oberflächen und geringer Biokompatibilität zum Trotz sind wesentliche Faktoren, wie z.B. einfache Anwendbarkeit und chirurgische Einsetzbarkeit, solcher Kleber unzureichend berücksichtigt worden. Ziel der Erfindung war vor diesem Hintergrund daher die Entwicklung einer polymerisierbaren Zusammensetzung auf Thiol-En-Basis, die sich unter anderem zur Verwendung als Knochenkleber eignet und mit der manche der weiterhin bestehenden Probleme zumindest teilweise gelöst werden können.

### OFFENBARUNG DER ERFINDUNG

Dieses Ziel erreicht die vorliegende Erfindung durch Bereitstellung einer polymerisierbaren Zusammensetzung auf Thiol-En-Basis, die zumindest einen radikalischen Initiator, Monomere mit zumindest zwei polymerisierbaren C-C-Doppelbindungen pro Molekül, Polythiole mit zumindest zwei SH-Gruppen pro Molekül sowie Primer zur Verbesserung der Haftung auf einem mit der Zusammensetzung zu beschichtenden Substrat umfasst, wobei die Primer pro Molekül jeweils zumindest eine polymerisierbare C-C-Doppelbindung, zumindest eine haftungsvermittelnde Gruppierung und eine dazwischen liegende, zumindest zweiwertige Kohlenwasserstoff-Gruppierung als Spacer aufweisen, wobei die erfindungsgemäße Zusammensetzung dadurch gekennzeichnet ist, dass
a) die polymerisierbaren C-C-Doppelbindungen der Primer 5-Norbornen-2-yl-Gruppen sind; und
b) die haftungsvermittelnden Gruppierungen der Primer aus Phosphonsäure-Gruppen (HO)₂P(=O)- und 3,4-Dihydroxyphenyl-Gruppen ausgewählt sind.

Anders formuliert stellt die vorliegende Erfindung die Verwendung von Verbindungen, die pro Molekül jeweils zumindest eine polymerisierbare C-C-Doppelbindung, zumindest eine haftungsvermittelnde Gruppierung und eine dazwischen liegende, zumindest zweiwertige Kohlenwasserstoff-Gruppierung als Spacer aufweisen, als Primer in einer polymerisierbaren Zusammensetzung auf Thiol-En-Basis zur Verbesserung der Haftung der Zusammensetzung auf einem damit zu beschichtenden Substrat bereit, wobei die Zusammensetzung weiters zumindest einen radikalischen Initiator, Monomere mit zumindest zwei polymerisierbaren C-C-Doppelbindungen pro Molekül und Polythiole mit zumindest zwei SH-Gruppen pro Molekül umfasst,
mit dem Kennzeichen, dass die Primer
a) als polymerisierbare C-C-Doppelbindungen 5-Norbornen-2-yl-Gruppen und
b) aus Phosphonsäure-Gruppen und 3,4-Dihydroxyphenyl-Gruppen ausgewählte Gruppen als haftungsvermittelnde Gruppierungen
   umfassen.

Die Erfinder haben nämlich überraschenderweise herausgefunden, dass ebendiese Kombinationen aus 5-Norbornen-2-yl-Gruppen als polymerisierbare Gruppen PG und Phosphonsäure- oder 3,4-Dihydroxyphenyl- (4-Brenzcatechyl-) Gruppen als Adhäsionsmotiv AM der Primermoleküle synergistische Wirkung zeigen und eine Reihe von Vorteilen nicht nur, aber speziell bei Verwendung der erfindungsgemäßen Zusammensetzung als Knochenkleber, bieten. Einerseits haben sich Norbornenyl-Gruppen als in durch Thiol-en-Additionspolymerisation härtbaren Zusammensetzungen im direkten Vergleich mit allen anderen, üblichen Primer-PG als am reaktivsten erwiesen. Und andererseits ist gleichzeitig die mit diesen Primern erzielte Haftung der gehärteten Zusammensetzung auf Substraten - sowohl auf Hydroxylapatit als auch auf Metalloberflächen - jener, die unter Verwendung herkömmlicher Primer in ansonsten identischen Testzusammensetzungen oder mit im Handel erhältlichen Knochenklebern erreicht wird, deutlich überlegen, wie in den späteren Beispielen anhand von Messungen der Scherhaftfestigkeit der gehärteten Zusammensetzungen - sowohl für Ein- als auch Zwei-Komponenten-Zusammensetzungen - belegt wird.

Ohne sich auf eine bestimmte Theorie festlegen zu wollen, nehmen die Erfinder an, dass auch die ausgezeichneten Ergebnisse in Bezug auf die Scherhaftfestigkeit auf die hohe Reaktivität der Norbornenyl-Gruppen während der Thiol-en-Additionspolymerisation zurückzuführen sind. Dadurch werden die Primermoleküle bereits unmittelbar nach Initiierung der Polymerisationsreaktion in die entstehende Polymermatrix eingebaut und sind daher imstande, die Haftung der Matrix am zu beschichtenden Substrat, wie z.B. Knochenmaterial, schon sehr früh zu fördern, d.h. schon lange bevor die Härtung der Zusammensetzung abgeschlossen ist.

In bevorzugten Ausführungsformen der Erfindung umfassen die Primer jeweils zumindest zwei 5-Norbornen-2-yl-Gruppen und/oder zumindest zwei haftungsvermittelnde Gruppierungen, was die obigen Effekte noch weiter verstärkt, noch bevorzugter aber nicht mehr als drei 5-Norbornen-2-yl-Gruppen und/oder nicht mehr als zwei haftungsvermittelnde Gruppierungen, da in diesen Fällen keine weitere Verbesserung der Wirkung zu erwarten ist und daher das Molekulargewicht der Primer und somit die Masse der Formulierung unnötigerweise erhöht wird. Besonders bevorzugt umfassen die Primer jeweils zwei 5-Norbornen-2-yl-Gruppen und eine oder zwei haftungsvermittelnde Gruppierungen, insbesondere Phosphonsäure-Gruppen (HO)₂P(=O)-.

Die haftungsvermittelnden Gruppierungen der Primer sind nämlich besonders bevorzugt Phosphonsäure-Gruppen, da diese in Scherhaftfestigkeitstests nach Beschichtung und Härtung von erfindungsgemäßen Zusammensetzungen auf Hydroxylapatit- und Titandioxid-Oberflächen - speziell auf Letzteren - noch besser abgeschnitten haben als 3,4-Dihydroxyphenyl- (4-Brenzcatechyl-) Gruppen als AM der Primer und zudem einfacher zu synthetisieren sind.

Die Spacer-Gruppierungen zur Verbindung der PG- und AM-Gruppen der Primer sind in der Regel Kohlenwasserstoff-Gruppierungen, deren Anzahl an Kohlenstoff- und gegebenenfalls auch Heteroatomen nicht speziell eingeschränkt ist, so dass auch oligomere oder polymere Spacer zum Einsatz kommen können, die daher mitunter auch eine zweistellige Anzahl an 5-Norbornen-2-yl-PG-Gruppen und/oder AM-Gruppierungen miteinander verbinden können. Andererseits können die Spacer auch nur ein oder zwei (Kohlenstoff-)Atome umfassen, um beispielsweise nur jeweils eine 5-Norbornen-2-yl-Gruppe und eine AM-Gruppierung zu verbinden. Gemäß vorliegender Erfindung wird jedoch bevorzugt, dass die Spacer-Gruppierung eine Kettenlänge von zumindest 3 oder zumindest 6 Kohlenstoffatomen aufweist, von denen eines oder mehrere gegebenenfalls durch jeweils ein aus O, S und N ausgewähltes Heteroatom ersetzt sind. Primer, die Spacer mit geringeren Kettenlängen enthalten, ergeben tendenziell niedrigere Scherhaftfestigkeiten, was von den Erfindern, ohne sich darauf festlegen zu wollen, auf eine geringere Flexibilität der nach der Härtung aus der Polymermatrix herausragenden Spacer-AM-Ketten zurückgeführt wird.

Spacer-Gruppierungen der Primer, die eine Anzahl an Kohlenstoffatomen, von denen eines oder mehrere gegebenenfalls durch jeweils ein aus O, S und N ausgewähltes Heteroatom ersetzt sind, von mehr als 40 aufweisen und/oder eine Kettenlänge, womit hierin die direkte lineare Verbindung zwischen den PG- und AM-Gruppen gemeint ist, von mehr als 10 oder mehr als 12 Kohlenstoff- bzw. Heteroatomen aufweisen, sind in der Zusammensetzung der Erfindung allerdings auch nicht zu bevorzugen, da diese keine zusätzliche Erhöhung der Scherhaftfestigkeit bewirken, aber wiederum das Molekulargewicht und folglich die Masse der Formulierung unerwünschterweise erhöhen.

Die Monomere sind erfindungsgemäß vorzugsweise aus Verbindungen mit zumindest zwei Allyl- oder Vinylester-Gruppen, noch bevorzugter aus 1,3,3-Triallyl-1,3,5-triazin-2,4,6-trion (TATATO), O,O'-(Hexahydrofuro[3,2-b]furan-3,6-diyl)divinyladipat (Glucitoldivinyladipat, GDVA) und Gemischen davon ausgewählt, da sich diese in bisherigen Tests der Erfinder als mit den erfindungsgemäßen Primern gut kompatibel erwiesen und gute Scherhaftfestigkeiten ergeben haben. Insbesondere sind oder umfassen die Monomere 1,3,3-Triallyl-1,3,5-triazin-2,4,6-trion (TATATO), das in den Tests noch besser abgeschnitten hat als O,O'-(Hexahydrofuro[3,2-b]furan-3,6-diyl)-divinyladipat (GDVA).

In weiteren bevorzugten Ausführungsformen der Erfindung sind die in der Zusammensetzung enthaltenen Polythiole aus Verbindungen mit zumindest drei SH-Gruppen pro Molekül, besonders bevorzugt aus Tris[2-(3-mercaptopropionyloxy)ethyl]isocyanurat (TEMPIC), Trimethylolpropan-tris(3-mercaptopropionat) (TMPMP) und Gemischen davon ausgewählt, da diese Polythiole besonders gute Testergebnisse geliefert haben, die bei Verwendung von Tris[2-(3-mercaptopropionyloxy)ethyl]isocyanurat (TEMPIC) alleine noch etwas besser waren als jene mit Trimethylolpropan-tris(3-mercaptopropionat) (TMPMP) alleine oder Gemischen der beiden.

Die polymerisierbare Zusammensetzung kann gemäß vorliegender Erfindung sowohl als Ein-Komponenten-Zusammensetzung vorliegen als auch als Zwei-Komponenten-Zusammensetzung, wovon nur die erste Komponente einen oder mehrere der oben definierten Primer umfasst, die bei Verwendung zuerst auf die zu beschichtende Oberfläche aufgetragen und vorgehärtet wird, bevor der Auftrag der zweiten Komponente auf die erste und anschließend die vollständige Härtung beider Schichten erfolgt. Je nach der speziellen Anwendung der Zusammensetzung kann entweder eine Ein- oder eine Zwei-Komponenten-Zusammensetzung zu bevorzugen sein.

Die Massenverhältnisse der in der polymerisierbaren Zusammensetzung enthaltenen Komponenten sind natürlich nicht speziell eingeschränkt. Ein durchschnittlicher Fachmann auf dem Gebiet der auf Thiol-En-Polyaddition ist anhand der hierin offenbarten Lehre ohne übermäßiges Experimentieren in der Lage, für eine spezielle Auswahl von Komponenten und zu beschichtenden Oberflächen optimale Mischungsverhältnisse zwischen Initiatoren, Monomeren, Polythiolen, Primern und etwaigen Additiven, wie z.B. Füllstoffen, Stabilisatoren und anderen auf dem Gebiet üblichen Zusatzstoffen, zu bestimmen - beispielsweise unter Durchführung der in den späteren Beispielen beschriebenen Testverfahren.

Allgemein kann die polymerisierbare Zusammensetzung in bevorzugten Ausführungsformen etwa 0,1 bis etwa 5 Gew.-% radikalischen Initiator, etwa 10 bis etwa 50 Gew.-% Monomere, etwa 40 bis etwa 80 Gew.-% Polythiole und etwa 5 bis etwa 25 Gew.-% Primer umfassen, deren Summe natürlich 100 Gew.-% ergeben muss. Im Falle einer Ein-Komponenten-Zusammensetzung kann diese beispielsweise etwa 0,5 bis etwa 2 Gew.-% radikalischen Initiator, etwa 40 bis etwa 60 Gew.-% Monomere, etwa 60 bis etwa 80 Gew.-% Polythiole und etwa 10 bis etwa 20 Gew.-% Primer jeweils in solchen Mengen umfassen, dass die Summe wiederum 100 Gew.-% ergibt. Zusätzliche Additive, Füllstoffe, Stabilisatoren etc. sind natürlich auch in diesen Fällen in beliebigen Mengen möglich, die mit den übrigen Komponenten verträglich sind.

Der radikalische Initiator ist in besonders bevorzugten Ausführungsformen der Erfindung ein radikalischer Photoinitiator, so dass die Zusammensetzung photopolymerisierbar ist. Dies ist speziell im Hinblick auf die Verwendung als Knochenkleber vorteilhaft, da die Anwendung der polymerisierbaren Zusammensetzung zur Behandlung oder Heilung in vivo erheblich vereinfacht wird, wenn die Härtung der auf eine Knochenbruchstelle aufgetragenen Zusammensetzung mittels einfacher Bestrahlung ausgelöst werden kann. Speziell in solchen Fällen handelt es sich bei der polymerisierbaren Zusammensetzung auch vorzugsweise um eine Ein-Komponenten-Zusammensetzung. Alternativ dazu können die Initiatoren freilich auch Redox- oder thermische Initiatoren sein, wobei Letztere für In-vivo-Anwendungen aber kaum in Frage kommen werden.

In einem weiteren Aspekt stellt die vorliegende Erfindung somit auch eine Zusammensetzung, speziell eine Ein-Komponenten-Zusammensetzung, wie oben definiert, zur Verwendung als Knochenkleber bereit, die von medizinischem Fachpersonal zur Behandlung von Knochenbrüchen oder -verletzungen einsetzbar ist.

Und in einem weiteren Aspekt stellt die vorliegende Erfindung ein Norbornen-Derivat der nachstehenden Formel

(Norb)ₙ-Sp-(AM)ₘ

bereit, worin
Norb für einen 5-Norbornen-2-yl-Rest steht;
AM jeweils unabhängig für eine Phosphonsäure-Gruppe (HO)₂P(=O)- oder eine 3,4-Dihydroxyphenyl-Gruppe steht;
n und m jeweils unabhängig für eine ganze Zahl ≥ 1 stehen; und
Sp für eine Spacer-Gruppe steht, die aus (n+m)-wertigen Kohlenwasserstoff-Resten mit 1 bis 40 Kohlenstoffatomen ausgewählt ist, von denen eines oder mehrere gegebenenfalls durch jeweils ein aus O, S und N ausgewähltes Heteroatom ersetzt sind.

Diese neuen chemischen Verbindungen mit einem oder mehreren 5-Norbornen-2-yl-Resten als polymerisierbare Gruppen und einer oder mehreren Phosphonsäure-Gruppen (HO)₂P(=O)- und/oder 3,4-Dihydroxyphenyl-Gruppen als haftungsvermittelnde Gruppierungen eignen sich sehr gut als Primer in polymerisierbaren Zusammensetzungen auf Thiol-En-Basis gemäß dem ersten Aspekt der vorliegenden Erfindung, wie anhand zahlreicher, nachfolgend angeführter Beispiele und Vergleichsbeispiele belegt wird.

Die Anzahl an polymerisierbaren 5-Norbornen-2-yl-Resten Norb und an Phosphonsäure- (HO)₂P(=O)- oder 3,4-Dihydroxyphenyl-Gruppierungen AM in den Norbornen-Derivaten gemäß vorliegender Erfindung ist nicht speziell eingeschränkt. Das bedeutet, dass in Abhängigkeit von der Kettenlänge des Spacers Sp jeweils nur eine oder jeweils mehrere Gruppen Norb und AM an diesen gebunden sein können, z.B. jeweils 2, 3, 4, 5 oder mehr Gruppen Norb und/oder AM. Wie oben erwähnt kann im Falle von oligomeren oder polymeren Spacern mitunter sogar jeweils eine zweistellige Anzahl an entsprechenden Gruppen Norb und/oder AM in den Norbornen-Derivaten enthalten sein. Das heißt, n und m können jeweils für 2, 3, 4, 5 oder mehr, mitunter sogar für eine Zahl > 10 stehen.

Wie ebenfalls bereits erwähnt, stehen n und m aber vorzugsweise für nicht mehr als 3, da bei einer größeren Anzahl der Gruppen Norb und AM keine weitere Verbesserung der Wirkung bei Verwendung der Norbornen-Derivate als Primer in polymerisierbaren Zusammensetzungen zu erwarten ist und daher das Molekulargewicht der Primer und somit die Masse der jeweiligen Formulierung unnötigerweise erhöht wird.

Besonders bevorzugt ist das erfindungsgemäße Norbornen-Derivat der obigen Formel dadurch gekennzeichnet, dass
a) n und m jeweils unabhängig für 1 oder 2 stehen; und/oder
b) S für einen (n+m)-wertigen Kohlenwasserstoff-Rest mit 3 bis 20 oder 6 bis 16 Kohlenstoffatomen steht, von denen eines oder mehrere gegebenenfalls durch jeweils ein aus O, S und N ausgewähltes Heteroatom ersetzt sind; und/oder
c) AM jeweils für eine Phosphonsäure-Gruppe (HO)₂P(=O)- steht;
wobei noch bevorzugter alle drei der Merkmale a) bis c) erfüllt sind, was die besten Ergebnisse geliefert hat.

Und insbesondere ist das Norbornen-Derivat der vorliegenden Erfindung aus den folgenden Verbindungen ausgewählt, die sich in den Beispielen bewährt haben:
N-(4-Hydroxy-4,4-diphosphonobutyl)-5-norbornen-2-carbonsäureamid-Natriumsalz (N-(4-Hydroxy-4,4-diphosphonobutyl)alendronsäureamid-Natriumsalz) (Alendronat-Norb) (B1): 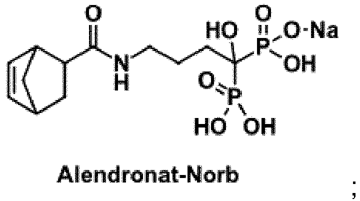
N-[2-(3,4-Dihydroxyphenyl)ethyl]-5-norbornen-2-carbonsäureamid (Catechol-2C-Norb) (B2): 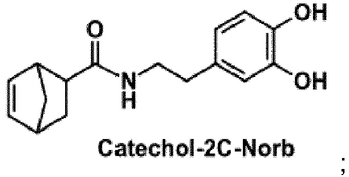
5-Norbornen-2-ylcarbonyloxymethylphosphonsäure (Phn-1C-Norb) (B3): 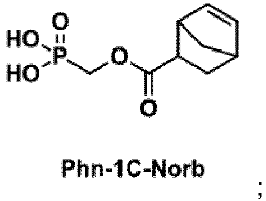
6-(5-Norbornen-2-ylcarbonyloxy)hexylphosphonsäure (Phn-6C-Norb) (B4): 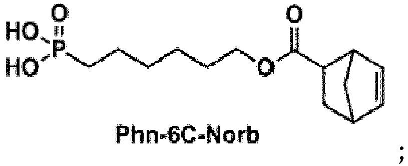
6-(2,3-Bis(5-norbornen-2-ylcarbonyloxy)propylthio)hexylphosphonsäure (Phn-6C-bisNorb) (B5): 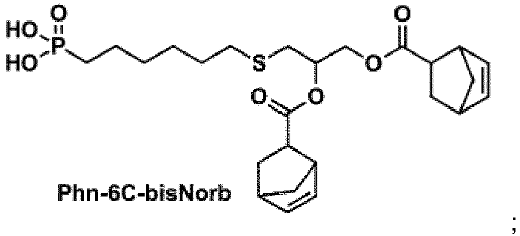
6-(2,3-Bis(5-norbornen-2-ylcarbonyloxy)propylthio)hexan-1,1-diyl-bis(phosphonsäure) (bisPhn-6C-bisNorb) (B6): 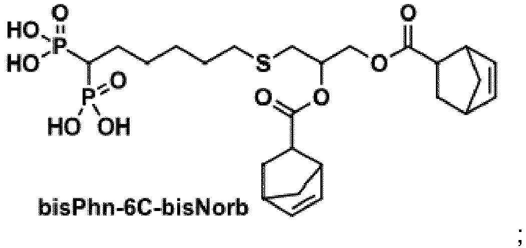
3-(3-(3,4-Dihydroxyphenyl)propylthio)propan-1,2-diyl-bis(norbornen-2-carboxylat) (Catechol-3C-bisNorb) (B7): 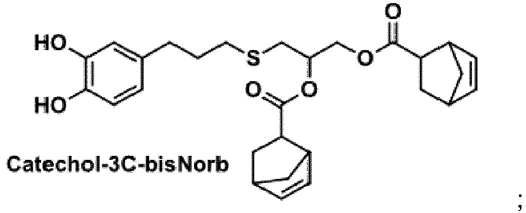
Ethylendiamintetraessigsäure-bis(2-(3,4-dihydroxyphenyl)ethyl)amid-bis(2-(5-norbornen-2-yl)ethyl)amid (bisCatechol-EDTA-bisNorb) (B8): 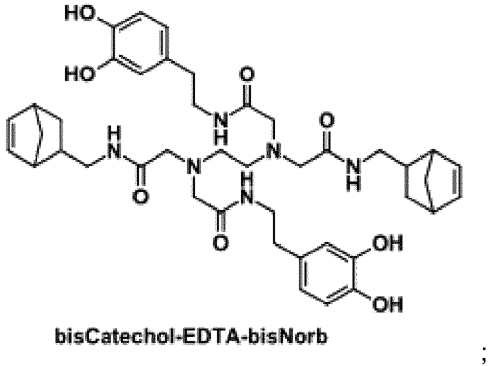
Diethylentriaminpentaessigsäure-bis(2-(5-norbornen-2-yl)ethyl)amid-tris(2-(3,4-dihydroxyphenyl)ethyl)amid (triCatechol-DTPA-bisNorb) (B9):

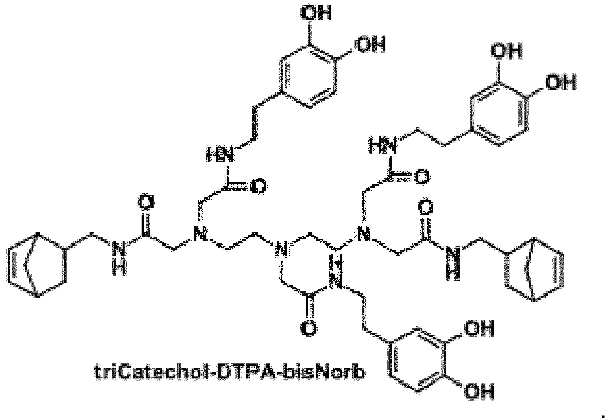

Von diesen neuen Verbindungen haben sich 6-(2,3-Bis(5-norbornen-2-ylcarbonyloxy)-propylthio)hexylphosphonsäure (Phn-6C-bisNorb) (B5) und 6-(2,3-Bis(5-norbornen-2-ylcarbonyloxy)propylthio)hexan-1,1-diyl-bis(phosphonsäure) (bisPhn-6C-bisNorb) (B6) als besonders wirksam erwiesen.

Aufgrund dieser guten Ergebnisse stellt die vorliegende Erfindung in einem letzten Aspekt auch die Verwendung aller obigen neuen Norbornen-Derivate als Primer in polymerisierbaren Zusammensetzungen gemäß dem ersten Aspekt bereit.

### KURZBESCHREIBUNG DER ZEICHNUNGEN

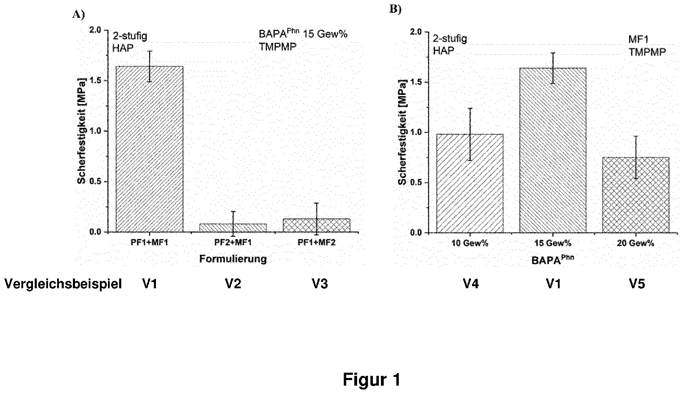

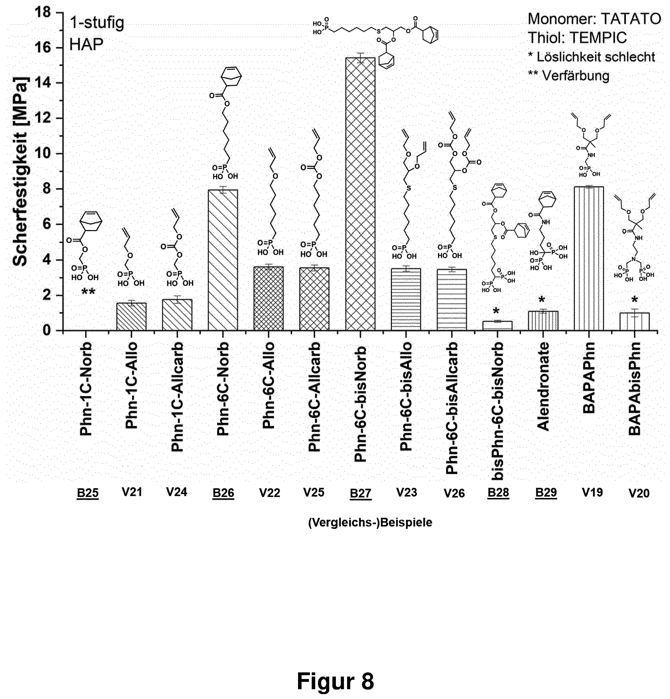

Die vorliegende Erfindung wird nachstehend anhand von konkreten Synthese-, Ausführungs- und Vergleichsbeispielen, die ausschließlich zu Illustrationszwecken dienen und nicht als Einschränkung zu verstehen sind, sowie unter Bezugnahme auf die beiliegenden Figuren näher beschrieben. Von diesen sind die Fig. 1 bis 7 grafische Darstellungen der Ergebnisse von Messungen der Scherhaftfestigkeit, die mit Zwei-Komponenten-Zusammensetzungen der Beispiele 1 bis 24 und Vergleichsbeispiele 1 bis 18 erzielt wurde, und die Fig. 8 bis 12 sind Darstellungen der mit Ein-Komponenten-Zusammensetzungen der Beispiele 25 bis 36 und der Vergleichsbeispiele 19 bis 30 erzielten Ergebnisse.

### BEISPIELE

Sofern hierin nicht anders angegeben, wurden alle in den nachstehenden Reaktionen umgesetzten Reagenzien aus kommerziellen Quellen syntheserein bezogen und ohne weitere Reinigung eingesetzt. Die Reinheit aller in den Beispielen erhaltenen Zwischenprodukte und Produkte wurde mittels Bestimmungen von physikalischen Daten wie Schmelzpunkt (Fp.) und Brechungsindex (RI), sowie mittels Kernresonanzspektren (¹H-, ¹³C- und ³¹P-NMR), hochauflösender Massenspektrometrie (HR-MS) und Elementaranalysen überprüft. Norbornenyl-hältige Verbindungen waren durchwegs racemische Gemische der jeweiligen endo- und exo-Isomere.

Die folgenden Synthesebeispiele sind in Abhängigkeit der eingeführten polymerisierbaren Gruppen PG und Adhäsionsmotiven AM zu Gruppen zusammengefasst.

### Synthesebeispiele

### Synthesebeispiele 1 bis 8 - Herstellung von Vergleichsprimern mit Allyloxy (Allo) oder Allyloxycarbonyloxy (Allcarb) als PG und Phosphonsäure (Phn) als AM

### Synthesebeispiel 1 - Herstellung von ((2,2-Bis(allyloxymethyl)propanamido)methyl)-phosphonsäure (BAPA^{Phn}) (S1)

### Stufe 1: Herstellung von 2,2-Bis(allyloxymethyl)propansäure (BAPA)

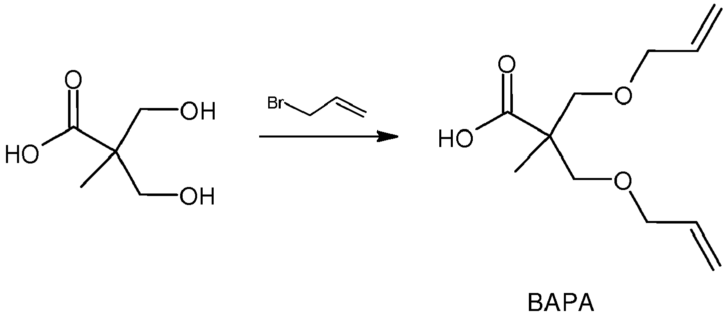

Die literaturbekannte Synthese erfolgte gemäß der Vorschrift in Antoni et al., Macromolecules 43, 6625-6631 (2010), durch Reaktion von 2,2-Bis(hydroxymethyl)propansäure mit Allylbromid.
DC (PE:EE, 9:1) R_{f} = 0,3; RI n_{D}^{20°C}: 1,4566; ¹H-NMR (400 MHz, CDCl₃) δ (ppm): 6,00-5,77 (m, 2H, 2x CH=CH₂), 5,22 (dd, 4H, 2x CH=CH₂), 4,01 (d, ³J_{HH} = 5,5, 1,5 Hz, 4H, 2x OCH₂CH), 3,58 (s, 4H, 2x CCH₂O), 1,25 (s, 3H, CCH₃); ¹³C-NMR (101 MHz, CDCl₃) δ (ppm): 180,14 (s, COOH), 136,7 (s, CH=CH₂), 117,08 (s, CH=CH₂), 72,55 (s, CCH₂O), 72,01 (s, OCH₂CH), 48,24 (s, C_{q}), 17,97 (s, CCH₃).

### Stufe 2 - Herstellung von ((2,2-Bis(allyloxymethyl)propanamido)methyl)phosphonsäure (BAPA^{Phn}) (S1)

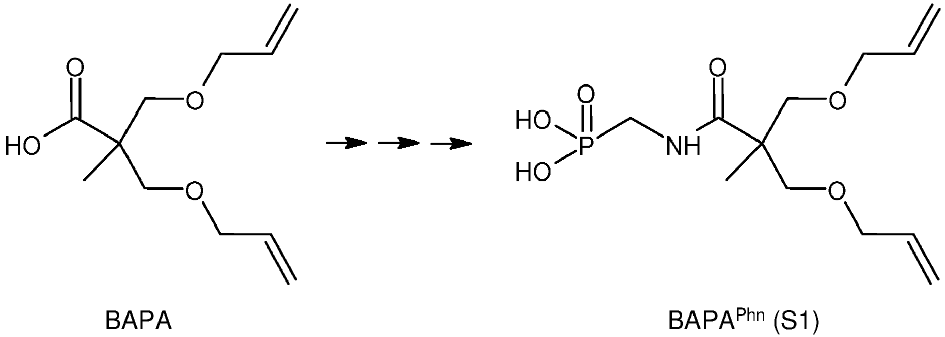

Die literaturbekannte Synthese erfolgte gemäß der Vorschrift in Granskog et al., s.o., durch Aktivierung von BAPA mit Carbonyldiimidazol (CDI), anschließende Reaktion des als Zwischenprodukt erhaltenen gemischten Anhydrids mit Aminoethylphosphonsäurediethylester und abschließende Esterspaltung mit Bromtrimethylsilan.
¹H-NMR (400 MHz, CDCl₃) δ (ppm): 6,02-5,73 (m, 2H, 2x CH=CH₂), 5,35-5,08 (m, 4H, 2x CH=CH₂), 4,01 (m, 4H, 2x CH₂CH=CH₂), 3,72-3,64 (m, 2H, NHCH₂P), 3,55 (s, 4H, 2x CCH₂O), 1,19 (s, 3H, CCH₃); ¹³C-NMR (101 MHz, CDCl₃) δ (ppm): 134,34 (s, CH=CH₂), 117,70 (s, CH=CH₂), 73,28 (s, CCH₂O), 72,62 (s, 2x CH₂CH=CH₂), 47,79 (s, C_{q}), 34,56 (d, ¹J_{CP} = 155,3 Hz, NHCH₂P), 18,38 (s, C_{q}CH₃); ³¹P-NMR (101 MHz, CDCl₃) δ (ppm): 19,22.

### Synthesebeispiel 2 - Herstellung von ((((2,2-Bis(allyloxymethyl)propanamido)ethyl)-azandiyl)-bis(methylen))-bis(phosphonsäure) (BAPA^{bisPhn}) (S2)

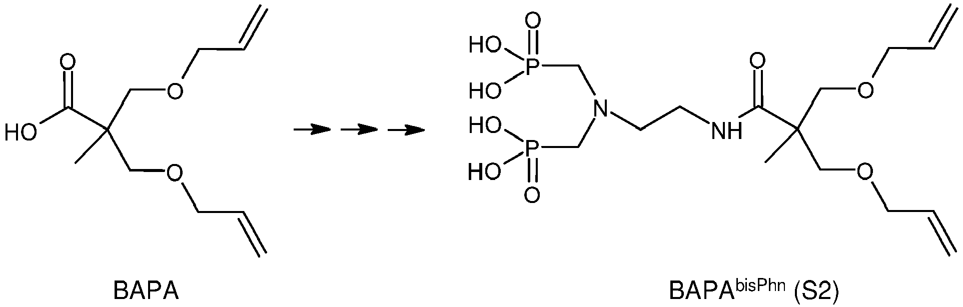

Die literaturbekannte Synthese erfolgte ebenfalls gemäß Granskog et al., s.o., wiederum durch anfängliche Aktivierung des in Synthesebeispiel, Stufe 1, erhaltenen BAPA mit Carbonyldiimidazol (CDI), Reaktion des gemischten Anhydrids mit Ethylendiamin zum Aminoethylamid, das mit je 2 mol p-Formaldehyd und Dimethylphosphit zum Bis(phosphonsäuredimethylester) umgesetzt wurde, und erneute abschließende Esterspaltung mit Bromtrimethylsilan.
¹H-NMR (400 MHz, CDCl₃) δ (ppm): 8,10 (s, 1H, NHCO), 6,12-5,81 (m, 2H, CH=CH₂), 5,21 (m, 4H, 2x CH=CH₂), 3,97 (d, 4H, ³J_{HH} = 12,9 Hz, 2x CH₂CH=CH₂), 3,80 (d, ³J_{HH} = 8,2 Hz, 4H, 2x NCH₂P), 3,70 (m, 4H, NHCH₂CH₂N), 3,54 (m, 4H, 2x CCH₂O), 1,21 (s, 3H, CCH₃); ¹³C-NMR (101 MHz, CDCl₃) δ (ppm): 178,9 (s, C=O), 136,2 (s, CH=CH₂), 117,40 (s, CH=CH₂), 73,80 (s, CCH₂O), 72,89 (s, 2x CH₂CH=CH₂), 59,4 (s, NHCH₂CH₂N), 52,9+51,5 (s, 2x NCH₂P), 48,98 (s, C_{q}), 36,71 (s, NHCH₂CH₂N), 18,29 (s, C_{q}CH₃); ³¹P-NMR (101 MHz, CDCl₃) δ (ppm): 9,66.

### Synthesebeispiel 3 - Herstellung von Allyloxymethylphosphonsäure (Phn-1C-Allo) (S3)

### Stufe 1: Herstellung von Allyloxymethylphosphonsäurediethylester (PhnEt-1C-Allo)

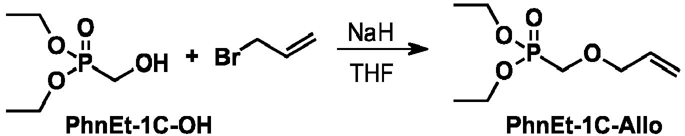

In einem Dreihalskolben wurde Natriumhydrid (1,9 Äqu., 0,7 g, 28,5 mmol) in 40 ml trockenem Tetrahydrofuran (THF) unter Argon suspendiert. Hydroxymethylphosphonsäurediethylester (PhnEt-1C-OH) (1 Äqu., 2,2 ml, 15 mmol) wurde in 10 ml THF verdünnt und bei 0 °C langsam mittels Septum zugegeben. Nach 1 h wurde Allylbromid (1,3 Äqu., 1,7 ml, 19,5 mmol) in den Kolben injiziert. Dann wurde das Eisbad entfernt und das Reaktionsgemisch über Nacht bei Raumtemperatur gerührt. Nach Abdampfen des Lösungsmittels und der nichtumgesetzten Edukte wurde das Rohprodukt mittels Säulenchromatographie (Ethylacetat, EE) gereinigt. 1,85 g (59 % der Theorie) des gewünschten Phosphonats PhnEt-1C-Allo wurden als farblose Flüssigkeit isoliert.
DC (EE) R_{f} = 0,52; RI n_{D}^{20°C}: 1,4357; ¹H-NMR (400 MHz, CDCl₃) δ (ppm): 5,96-5,77 (m, 1H, CH₂CH=CH₂), 5,33-5,18 (m, 2H, CH₂CH=CH₂), 4,25-4,03 (m, 6H, 2x OCH₂CH₃, CH₂CH=CH₂), 3,75 (d, ³J_{HH} = 8,7 Hz, 2H, PCH₂O), 1,39-1,29 (t, ³J_{HH} = 7,0 Hz, 6H, 2x OCH₂CH₃); ¹³C-NMR (101 MHz, CDCl₃) δ (ppm): 133,78 (s, OCH₂CHCH₂), 118,54 (s, OCH₂CHCH₂), 74,11 (d, J = 12,7 Hz, OCH₂CHCH₂), 63,81 (d, ²J_{CP} = 167,1 Hz, CH₂P C₁), 62,54 (d, ³J_{CP} = 6,3 Hz, OCH₂CH₃), 16,53 (d, ³J_{CP} = 6,0 Hz, OCH₂CH₃); ³¹P-NMR (CDCl₃) δ (ppm): 21,58.

### Stufe 2: Herstellung von Allyloxymethylphosphonsäure (Phn-1C-Allo) (S3)

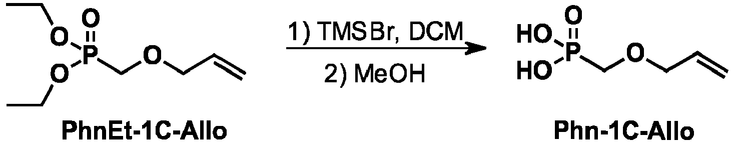

1,7 ml Bromtrimethylsilan (TMSBr) (12,5 mmol, 2,5 Äqu.) wurden zu einer Lösung von PhnEt-1C-Allo (1 Äqu., 1,1 g, 5 mmol) in 15 ml trockenem Methylenchlorid (DCM) zugesetzt. Nach 20 h Rühren bei 30 °C wurde das Gemisch unter reduziertem Druck eingeengt. Methanol (20 ml) wurde zugegeben, und die Lösung wurde 5 h lang bei Raumtemperatur gerührt. Das Lösungsmittel wurde abgedampft und das Produkt im Vakuum getrocknet. 0,75 g (99 % d. Th.) der gewünschten Phosphonsäure Phn-1C-Allo wurden als farblose Flüssigkeit isoliert.
RI n_{D}^{20°C}: 1,4773; ¹H-NMR (400 MHz, CDCl₃) δ (ppm): 9,78 (s, 2H, 2x OH), 5,97-5,78 (m, 1H, CH₂CH=CH₂), 5,41-5,21 (m, 2H, CH₂CH=CH₂), 4,15 (d, J = 5,7 Hz, 2H, CH₂CH=CH₂), 3,85 (d, J = 8,8 Hz, 2H, PCH₂O); ¹³C-NMR (101 MHz, CDCl₃) δ (ppm): 133,19 (s, OCH₂CHCH₂), 119,51 (s, OCH₂CHCH₂), 74,44 (d, J = 12,5 Hz, OCH₂CHCH₂), 63,81 (d, ²J_{CP} = 167,9 Hz, CH₂P C₁); ³¹P-NMR (CDCl₃) δ (ppm): 23,73; HR-MS (MeOH, ESI, m/z): ber. 152,09, gef. 152,07 [M].

### Synthesebeispiel 4 - Herstellung von 6-Allyloxyhexylphosphonsäure (Phn-6C-Allo) (S4)

### Stufe 1: Herstellung von 6-Allyloxyhexylphosphonsäurediethylester (PhnEt-6C-Allo)

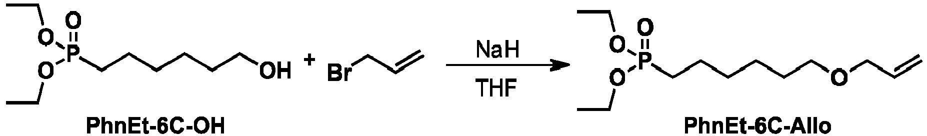

Die Synthese erfolgte analog zu Synthesebeispiel 3, Stufe 1, aus 6-Hydroxyhexylphosphonsäure (PhnEt-6C-OH) (1 Äqu., 3,1 g, 13 mmol) mit Allylbromid (1,5 Äqu., 1,7 ml, 19,5 mmol) und Natriumhydrid in Paraffinöl (1,9 Äqu., 0,6 g, 24,7 mmol). Mittels Säulenchromatographie (EE) wurden 1,4 g (49 % d. Th.) des gewünschten Phosphonats PhnEt-6C-Allo als farblose Flüssigkeit isoliert.
DC (EE/MeOH, 19:1) R_{f} = 0,47; RI n_{D}^{20°C}: 1,4461; ¹H-NMR (400 MHz, CDCl₃) δ (ppm): 5,98-5,81 (ddt, ¹J_{HH} = 17,3, ²J_{HH} = 10,4, ³J_{HH} = 5,6 Hz, 1H, CH=CH₂), 5,24 (dq, ¹J_{HH} = 17,2, ²J_{HH} = 1,7 Hz, 1H, CH=CH₂), 5,15 (dq, ¹J_{HH} = 10,4, ²J_{HH} = 1,4 Hz, 1H, CH=CH₂), 4,16-4,00 (m, 4H, 2x OCH₂CH₃), 3,94 (dt, ¹J_{HH} = 5,8, ²J_{HH} = 1,4 Hz, 2H, OCH₂CH), 3,40 (t, ²J_{HH} = 6,6 Hz, 2H, CH₂OCH₂CH), 1,77-1,48 (m, 6H, CH₂P C₁, CH₂CH₂O C₅, CH₂ C₄), 1,42-1,20 (m, 10H, 2x OCH₂CH₃, CH₂ C₃, CH₂CH₂P); ¹³C-NMR (101 MHz, CDCl₃) δ (ppm): 135,17 (s, CH₂CH=CH₂), 116,83 (s, CH₂CH=CH₂), 71,92 (s, CH₂CH=CH₂), 70,37 (s, CH₂O), 61,49 (d, ²J_{CP} = 6,6 Hz, OCH₂CH₃), 30,55 (d, ³J_{CP} = 16,8 Hz, CH₂CH₂CH₂P), 29,63 (s, CH₂CH₂O C₅), 25,84 (s, ⁴J_{CP} = 1,3 Hz, CH₂ C₄), 25,76 (d, ¹J_{CP} = 140,6 Hz, CH₂P C₁), 22,50 (d, ²J_{CP} = 5,1 Hz, PCH₂CH₂), 16,62 (d, ³J_{CP} = 6,0 Hz, OCH₂CH₃); ³¹P-NMR (CDCl₃) δ (ppm): 32,55.

### Stufe 2: Herstellung von 6-Allyloxyhexylphosphonsäure (Phn-6C-Allo) (S4)

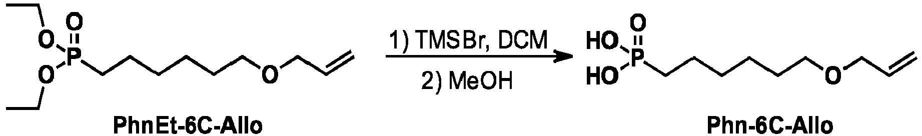

Die Synthese erfolgte analog zu Synthesebeispiel 3, Stufe 2, aus PhnEt-6C-Allo (1 Äqu., 1,4 g, 5 mmol) mit TMSBr (2,5 Äqu., 1,7 ml, 12,5 mmol), wobei 0,95 g (99 % d. Th.) der gewünschten Phosphonsäure Phn-6C-Allo als braune viskose Flüssigkeit isoliert wurden.
RI n_{D}^{20°C}: 1,4775; ¹H-NMR (400 MHz, CDCl₃) δ (ppm): 9,03-8,47 (m, 2H, 2x OH), 5,91 (ddt, ¹J_{HH} = 17,2, ²J_{HH} = 10,3, ³J_{HH} = 5,7 Hz, 1H, CH=CH₂), 5,27 (dq, ¹J_{HH} = 17,2, ²J_{HH} = 1,6 Hz, 1H, CH=CH₂), 5,18 (dq, ¹J_{HH} = 10,4, ²J_{HH} = 1,4 Hz, 1H, CH=CH₂), 3,98 (dt, ¹J_{HH} = 5,7, ²J_{HH} = 1,4 Hz, 2H, OCH₂CH), 3,45 (t, ³J_{HH} = 6,6 Hz, 2H, CH₂OCH₂CH), 1,88-1,73 (m, 2H, PCH₂), 1,62 (m, 4H, CH₂CH₂O C₅, CH₂ C₄), 1,40 (m, 4H, PCH₂CH₂ C₂, PCH₂CH₂CH₂ C₃); ¹³C-NMR (101 MHz, CDCl₃) δ (ppm): 134,70 (s, CH₂CH=CH₂), 117,38 (s, CH₂CH=CH₂), 71,97 (s, CH₂CH=CH₂), 70,43 (s, CH₂O), 30,15 (d, ³J_{CP} = 16,9 Hz, CH₂CH₂CH₂P), 29,35 (s, CH₂CH₂O C₅), 25,65 (s, CH₂C₄), 25,53 (d, ¹J_{CP} = 141 Hz, CH₂P C₁), 21,96 (d, ²J_{CP} = 5,1 Hz, PCH₂CH₂ C₂); ³¹P-NMR (CDCl₃) δ (ppm): 36,26; HR-MS (MeOH, ESI, m/z): ber. 222,22, gef. 222,20 [M].

### Synthesebeispiel 5 - Herstellung von 6-(2,3-Bis(allyloxy)propylthio)hexylphosphonsäure (Phn-6C-bisAllo) (S5)

### Stufe 1: Herstellung von 6-(2,3-Bis(allyloxy)propylthio)hexylphosphonsäurediethyl-ester (PhnEt-6C-bisAllo)

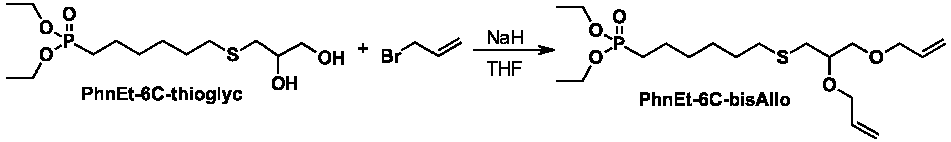

Die Synthese erfolgte analog zu Synthesebeispiel 3, Stufe 1, aus 6-(2,3-Dihydroxypropylthio)hexylphosphonsäurediethylester (PhnEt-6C-thioglyc) (1 Äqu., 3,1 g, 13 mmol) mit Allylbromid (1,5 Äqu., 1,7 ml, 19,5 mmol) und Natriumhydrid in Paraffinöl (1,9 Äqu., 0,6 g, 24,7 mmol). Mittels Säulenchromatographie (EE) wurden 1,4 g (49 % d. Th.) des gewünschten Phosphonats PhnEt-6C-bisAllo als farblose Flüssigkeit isoliert.
DC (EE) R_{f} = 0,3; RI n_{D}^{20°C}: 1,4775; ¹H-NMR (400 MHz, CDCl₃) δ (ppm): 5,99-5,82 (m, 2H, CH=CH₂), 5,32-5,09 (m, 4H, CH=CH₂), 4,23-3,95 (m, 8H, OCH₂CH₃, OCH₂), 3,74-3,49 (m, 3H, CHO, CH₂O), 2,80-2,61 (m, 2H, alkyl-CH₂SCH₂), 2,59-2,47 (m, 2H, alkyl-CH₂SCH₂), 1,79-1,50 (m, 6H, PCH₂, PCH₂CH₂, CH₂CH₂CH₂S), 1,38 (m, 4H, PCH₂CH₂CH₂, CH₂CH₂S), 1,31 (t, ³J_{HH} = 7,0 Hz, 6H, OCH₂CH₃); ¹³C-NMR (101 MHz, CDCl₃) δ (ppm): 134,93 (s, 2x CH=CH₂), 117,00 (s, 2x CH=CH₂), 78,2 (s, CHO), 72,35 (s, CH₂O), 71,18 (d, ³J_{CP} = 3,6 Hz, 2x OCH₂CH=CH₂), 61,42 (d, ³J_{CP} = 6,5 Hz, OCH₂CH₃), 33,61 (s, CH₂SCH₂), 33,05 (s, alkyl-CH₂SCH₂), 30,22 (d, ³J_{CP} = 16,8 Hz, PCH₂CH₂CH₂), 29,44 (s, CH₂CH₂CH₂S), 28,33 (s, CH₂CH₂S), 25,67 (d, ¹J_{CP} = 140,6 Hz, PCH₂), 22,36 (d, ²J_{CP} = 5,2 Hz, PCH₂CH₂), 16,45 (d, ³J_{CP} = 6,0 Hz, OCH₂CH₃); ³¹P-NMR (CDCl₃) δ (ppm): 32,40.

### Stufe 2: Herstellung von 6-(2,3-Bis(allyloxy)propylthio)hexylphosphonsäure (Phn-6C-bisAllo) (S5)

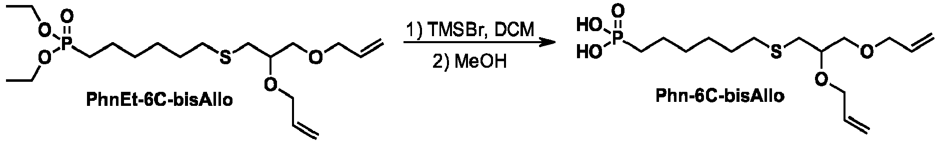

Die Synthese erfolgte analog zu Synthesebeispiel 3, Stufe 1, aus PhnEt-6C-bisAllo (1 Äqu., 0,8 g, 1,9 mmol) mit TMSBr (2,5 Äqu., 0,6 ml, 4,75 mmol), wobei 0,5 g (75 % d. Th.) der gewünschten Phosphonsäure Phn-6C-bisAllo als braune viskose Flüssigkeit isoliert wurden.
RI n_{D}^{20°C}: 1,5084; ¹H-NMR (400 MHz, CDCl₃) δ (ppm): 6,02-5,80 (m, 2H, CH=CH₂), 5,32-5,14 (m, 4H, CH=CH₂), 4,19-3,98 (m, 4H, OCH₂), 3,75-3,52 (m, 3H, CHO, CH₂O), 2,77-2,62 (m, 2H, alkyl-CH₂SCH₂), 2,60-2,50 (m, 2H, alkyl-CH₂SCH₂), 1,87-1,70 (m, 2H, PCH₂), 1,70-1,49 (m, 4H, PCH₂CH₂, CH₂CH₂CH₂S), 1,45-1,33 (m, 4H, PCH₂CH₂CH₂, CH₂CH₂S); ¹³C-NMR (101 MHz, CDCl₃) δ (ppm): 135,12 (s, CH=CH₂), 134,74 (s, CH=CH₂), 117,20 (s, 2x CH=CH₂), 78,10 (s, CHO), 72,51 (s, CH₂O), 71,30 (d, ³J_{CP} = 3,7 Hz, 2x OCH₂CH=CH₂), 33,67 (s, CH₂SCH₂), 33,13 (s, alkyl-CH₂SCH₂), 30,22 (d, ³J_{CP} = 17,1 Hz, PCH₂CH₂CH₂), 29,45 (s, CH₂CH₂CH₂S), 28,27 (s, CH₂CH₂S), 25,32 (d, ¹J_{CP} = 141 Hz, PCH₂), 21,95 (d, ²J_{CP} = 5,5 Hz, PCH₂CH₂); ³¹P-NMR (CDCl₃) δ (ppm): 37,32; HR-MS (MeOH, ESI, m/z): ber. 352,43, gef. 352,47 [M].

### Synthesebeispiel 6 - Herstellung von Allyloxycarbonyloxymethylphosphonsäure (Phn-1C-Allcarb) (S6)

### Stufe 1: Herstellung von Allyloxycarbonyloxymethylphosphonsäurediethylester (PhnEt-1C-Allcarb)

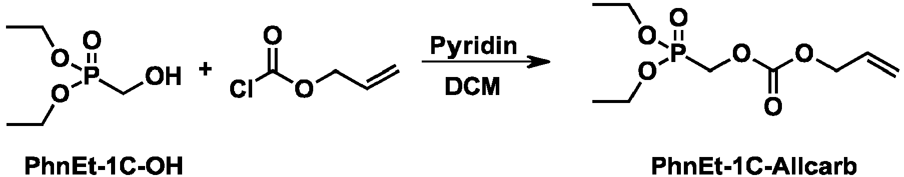

Ein Dreihalskolben wurde mit Hydroxymethylphosphonsäurediethylester (PhnEt-1C-OH) (1 Äqu., 2,2 ml, 15 mmol) und Pyridin (4 Äqu., 4,8 ml, 60 mmol) in 50 ml DCM unter Argon beschickt. Chlorameisensäureallylester (1,3 Äqu., 2,1 ml, 19,5 mmol) wurde bei 0 °C mittels Septum zugetropft. Nach 1 h Rühren bei 0 °C wurde das Eisbad entfernt und die Reaktion über Nacht bei Raumtemperatur gerührt. Das Rohprodukt wurde in EE gelöst und der Niederschlag (Pyridiniumsalze) abfiltriert. Nach Abdampfen des Lösungsmittels wurden 2,9 g (77 % d. Th.) des gewünschten Phosphonats PhnEt-1C-Allcarb als farblose Flüssigkeit isoliert.
DC (EE) R_{f} = 0,5; RI n_{D}^{20°C}: 1,4460; ¹H-NMR (400 MHz, CDCl₃) δ (ppm): 6,00-5,85 (m, 1H, CH₂CH=CH₂), 5,43-5,22 (m, 2H, CH₂CH=CH₂), 4,69-4,61 (m, 2H, CH₂CH=CH₂), 4,43 (d, ³J_{HH} = 8,5 Hz, 2H, PCH₂O), 4,25-4,10 (m, 4H, OCH₂CH₃), 1,35 (t, ³J_{HH} = 7,0 Hz, 6H, OCH₂CH₃); ¹³C-NMR (101 MHz, CDCl₃) δ (ppm): 154,71 (d, ³J_{CP} = 9,5 Hz, OC=OO), 131,26 (s, OCH₂CHCH₂), 119,42 (s, OCH₂CHCH₂), 69,31 (s, OCH₂CHCH₂), 63,07 (d, ²J_{CP} = 6,5 Hz, OCH₂CH₃), 60,32 (d, ¹J_{CP} = 168,5 Hz, CH₂P C₁), 16,49 (d, ³J_{CP} = 6,0 Hz, OCH₂CH₃); ³¹P-NMR (CDCl₃) δ (ppm): 17,74.

### Stufe 2: Herstellung von Allyloxycarbonyloxymethylphosphonsäure (Phn-1C-Allcarb) (S6)

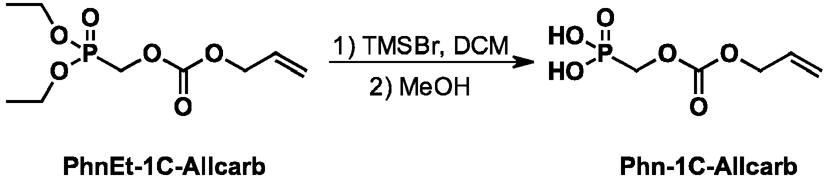

Die Synthese erfolgte analog zu Synthesebeispiel 3, Stufe 2, aus PhnEt-1C-Allcarb (1 Äqu., 2,5 g, 10 mmol) mit TMSBr (2,5 Äqu., 3,3 ml, 25 mmol), wobei 1,76 g (99 % d. Th.) der gewünschten Phosphonsäure Phn-1C-Allcarb als farblose Flüssigkeit isoliert wurden.
RI n_{D}^{20°C}: 1,4566; ¹H-NMR (400 MHz, CDCl₃) δ (ppm): 9,52 (s, 2H, OH), 6,00-5,81 (m, 1H, CH₂CH=CH₂), 5,43-5,22 (m, 2H, CH₂CH=CH₂), 4,65 (d, ³J_{HH} = 5,7 Hz, 2H, CH₂CH=CH₂), 4,46 (d, ³J_{HH} = 8,8 Hz, 2H, PCH₂O); ¹³C-NMR (101 MHz, CDCl₃) δ (ppm): 154,87 (d, ³J_{CP} = 9,5 Hz, OC=OO), 131,19 (s, OCH₂CHCH₂), 119,58 (s, OCH₂CHCH₂), 69,64 (s, OCH₂CHCH₂), 61,19 (d, ¹J_{CP} = 169,5 Hz, CH₂P C₁); ³¹P-NMR (CDCl₃) δ (ppm): 19,11; HR-MS (MeOH, ESI, m/z): ber. 196,09, gef. 196,06 [M].

### Synthesebeispiel 7 - Herstellung von 6-(Allyloxycarbonyloxy)hexylphosphonsäure (Phn-6C-Allcarb) (S7)

### Stufe 1: Herstellung von 6-(Allyloxycarbonyloxy)hexylphosphonsäurediethylester (PhnEt-6C-Allcarb)

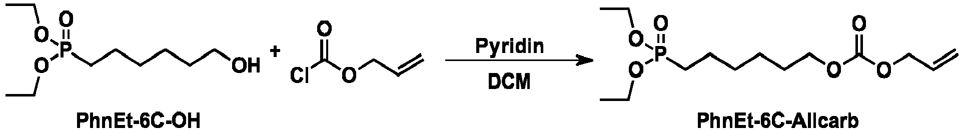

Die Synthese erfolgte analog zu Synthesebeispiel 6, Stufe 1, aus 6-Hydroxyhexylphosphonsäurediethylester (PhnEt-6C-OH) (1 Äqu., 2,4 g, 10 mmol) mit Chlorameisensäureallylester (1,5 Äqu., 1,6 ml, 15 mmol) und Pyridin (4 Äqu., 3,2 ml, 40 mmol), wobei nach dem Abdampfen des Lösungsmittels 2,2 g (68 % d. Th.) des gewünschten Phosphonats PhnEt-6C-Allcarb als gelbliche viskose Flüssigkeit isoliert wurden.
DC (EE/MeOH, 9:1) R_{f} = 0,51; RI n_{D}^{20°C}: 1,4463; ¹H-NMR (400 MHz, CDCl₃) δ (ppm): 5,98-5,82 (m, 1H, CH₂CH=CH₂), 5,39-5,20 (m, 2H, CH₂CH=CH₂), 4,64-4,53 (m, 2H, CH₂CH=CH₂), 4,15-3,94 (m, 6H, CH₂O C₆, OCH₂CH₃), 1,83-1,47 (m, 4H, CH₂P C₁, CH₂CH₂O C₅), 1,44-1,19 (m, 12H, CH₂ C₂-C₄, OCH₂CH₃); ¹³C-NMR (101 MHz, CDCl₃) δ (ppm): 155,15 (s, OC=OO), 131,67 (s, OCH₂CHCH₂), 118,93 (s, OCH₂CHCH₂), 68,41 (s, CH₂CH₂O C₆), 68,05 (s, OCH₂CHCH₂), 61,49 (d, ²J_{CP} = 6,4 Hz, OCH₂CH₃), 32,54 (s, CH₂CH₂O C₅), 30,21 (d, ³J_{CP} = 16,8 Hz, CH₂CH₂CH₂P C₃), 25,68 (d, ¹J_{CP} = 140,7 Hz, CH₂P C₁), 25,32 (d, ⁴J_{CP} = 1,2 Hz, CH₂ C₄), 22,40 (d, ²J_{CP} = 5,1 Hz, CH₂CH₂P C₂), 16,55 (d, ³J_{CP} = 6,0 Hz, OCH₂CH₃); ³¹P-NMR (CDCl₃) δ (ppm): 32,23.

### Stufe 2: Herstellung von 6-(Allyloxycarbonyloxy)hexylphosphonsäure (Phn-6C-Allcarb) (S7)

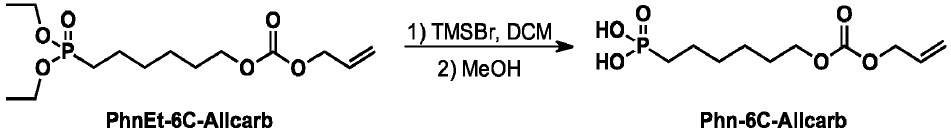

Die Synthese erfolgte analog zu Synthesebeispiel 3, Stufe 2, aus PhnEt-6C-Allcarb (1 Äqu., 2,1 g, 6,5 mmol) mit TMSBr (2,5 Äqu., 2,2 ml, 16,25 mmol), wobei 1,5 g (87 % d. Th.) der gewünschten Phosphonsäure Phn-6C-Allcarb als braune viskose Flüssigkeit isoliert wurden.
¹H-NMR (400 MHz, CDCl₃) δ (ppm): 6,02-5,84 (m, 1H, CH₂CH=CH₂), 5,40-5,21 (m, 2H, CH₂CH=CH₂), 4,67-4,53 (m, 2H, CH₂CH=CH₂), 4,13 (t, J = 6,6 Hz, 2H, CH₂O), 1,94-1,56 (m, 6H, CH₂P C₁, CH₂CH₂O C₅, CH₂C₄), 1,49-1,35 (m, 4H, CH₂CH₂P C₂, CH₂ C₃); ¹³C-NMR (101 MHz, CDCl₃) δ (ppm): 155,13 (s, OC=OO), 131,61 (s, OCH₂CHCH₂), 118,92 (s, OCH₂CHCH₂), 68,40 (s, CH₂O C₆), 68,01 (s, OCH₂CHCH₂), 30,02 (d, ³J_{CP} = 17,0 Hz, CH₂CH₂CH₂P C₃), 28,52 (s, CH₂CH₂O C₅), 25,45 (d, ¹J_{CP} = 141 Hz, CH₂P C₁), 25,31 (s, CH₂ C₄), 21,93 (d, ²J_{CP} = 5,0 Hz, PCH₂CH₂ C₂); ³¹P-NMR (CDCl₃) δ (ppm): 36,31; HR-MS (MeOH, ESI, m/z): ber. 238,26, gef. 238,31 [M].

### Synthesebeispiel 8 - Herstellung von 6-(2,3-Bis(allyloxycarbonyloxy)propylthio)hexylphosphonsäure (Phn-6C-bisAllcarb) (S8)

### Stufe 1: Herstellung von 6-(2,3-Bis(allyloxycarbonyloxy)propylthio)hexylphosphonsäurediethylester (PhnEt-6C-bisAllcarb)

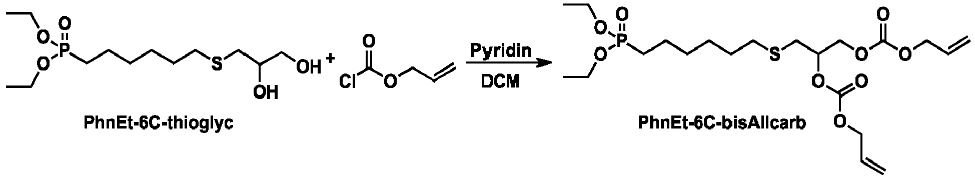

Die Synthese erfolgte analog zu Synthesebeispiel 6, Stufe 1, aus 6-(2,3-Dihydroxypropylthio)hexylphosphonsäurediethylester (PhnEt-6C-thioglyc) (1 Äqu., 3,3 g, 10 mmol) mit Allylchlorameisensäureester (4 Äqu., 4,3 ml, 40 mmol) und Pyridin (9 Äqu., 7,3 ml, 90 mmol). Mitrtels Säulenchromatographie (EE) wurden 3,38 g (68 % d. Th.) des gewünschten Phosphonats PhnEt-6C-bisAllcarb als braune viskose Flüssigkeit isoliert.
DC (EE) R_{f} = 0,4; RI n_{D}^{20°C}: 1,4774; ¹H-NMR (400 MHz, CDCl₃) δ (ppm): 6,00-5,85 (m, 2H, 2H, 2x CH₂CH=CH₂), 5,41-5,22 (m, 4H, 2x CH₂CH=CH₂), 5,01-4,92 (m, 1H, CHO), 4,67-4,58 (m, 4H, CH₂CH=CH₂), 4,48 (dd, J = 11,9, 3,2 Hz, 1H, CH₂OC=O), 4,30 (dd, J = 11,9, 5,9 Hz, 1H, CH₂OC=O), 4,18-3,97 (m, 4H, OCH₂CH₃), 2,83-2,69 (m, 2H, SCH₂CH), 2,60-2,52 (m, 2H, CH₂CH₂S C₆), 1,79-1,51 (m, 6H, PCH₂ C₁,_PCH₂CH₂ C₂, CH₂CH₂S C₅), 1,38 (m, 4H, CH₂ C₃-C₄), 1,31 (t, J = 7,1 Hz, 6H, OCH₂CH₃); ¹³C-NMR (101 MHz, CDCl₃) δ (ppm): 154,77 (s, OC=OO), 154,42 (s, OC=OO), 131,44 (s, OCH₂CHCH₂), 119,29 (s, OCH₂CHCH₂), 119,21 (s, OCH₂CHCH₂), 74,84 (s, CHO), 68,90 (d, J = 3,1 Hz, 2x OCH₂CHCH₂), 66,97 (s, CH₂O), 61,53 (d, ²J_{CP} = 6,5 Hz, OCH₂CH₃), 32,80 (s, SCH₂CH), 31,95 (s, CH₂CH₂S), 30,27 (d, ³J_{CP} = 16,7 Hz, CH₂CH₂CH₂P C₃), 29,33 (s, CH₂CH₂S), 28,34 (d, ⁴J_{CP} = 1,3 Hz, CH₂ C₄), 25,77 (d, ¹J_{CP} = 140,6 Hz, PCH₂ C₁), 22,47 (d, ²J_{CP} = 5,2 Hz, PCH₂CH₂ C₂), 16,61 (d, ³J_{CP} = 6,1 Hz, OCH₂CH₃); ³¹P-NMR (CDCl₃) δ (ppm): 32,33.

### Stufe 2: Herstellung von 6-(2,3-Bis(allyloxycarbonyloxy)propylthio)hexylphosphonsäure (Phn-6C-bisAllcarb) (S8)

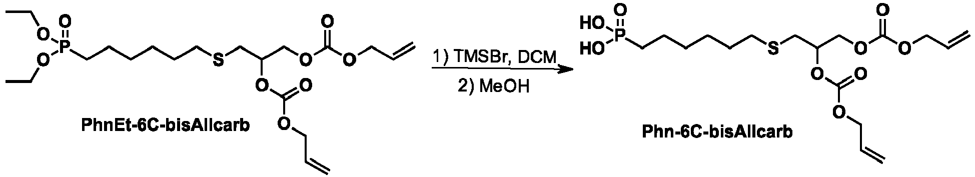

Die Synthese erfolgte analog zu Synthesebeispiel 3, Stufe 2, aus PhnEt-6C-bisAllcarb (1 Äqu., 1,9 g, 3,8 mmol) mit TMSBr (2,5 Äqu., 1,3 ml, 9,5 mmol), wobei 1,61 g (87 % d. Th.) der gewünschten Phosphonsäure Phn-6C-bisAllcarb als braune viskose Flüssigkeit isoliert wurden.
RI n_{D}^{20°C}: 1,4877; ¹H-NMR (400 MHz, CDCl₃) δ (ppm): 7,90 (s, 2H, 2x CH₂CH=CH₂), 6,01-5,84 (m, 2H), 5,43-5,23 (m, 4H, 2x CH₂CH=CH₂), 5,04-4,92 (m, 1H, CHO), 4,68-4,58 (m, 4H, 2x CH₂CH=CH₂), 4,48 (dd, J_{HH} = 11,9, 3,1 Hz, 1H, CH₂OC=O), 4,31 (dd, J_{HH} = 11,9, 6,0 Hz, 1H, CH₂OC=O), 2,80-2,72 (m, 2H, SCH₂CH), 2,62-2,52 (m, 2H, CH₂CH₂S C₆), 1,90-1,73 (m, 2H, PCH₂ C₁), 1,70-1,53 (m, 4H, PCH₂CH₂ C₂, CH₂CH₂S C₅), 1,47-1,35 (m, 4H, CH₂ C₃-C₄); ¹³C-NMR (101 MHz, CDCl₃) δ (ppm): 154,81 (s, OC=OO), 154,49 (s, OC=OO), 131,44 (s, OCH₂CHCH₂), 119,29 (s, OCH₂CHCH₂), 119,21 (s, OCH₂CHCH₂), 74,84 (s, CHO), 68,97 (d, J = 3,1 Hz, 2x OCH₂CHCH₂), 67,03 (s, CH₂O), 32,72 (s, SCH₂CH), 31,94 (s, CH₂CH₂S), 29,90 27 (d, ³J_{CP} = 17,1 Hz, CH₂CH₂CH₂P C₃), 29,21 (s, CH₂CH₂S), 28,15 (d, ⁴J_{CP} = 1,3 Hz, CH₂ C₄), 25,32 (d, ¹J_{CP} = 140,4 Hz, PCH₂ C₁), 21,88 (d, ²J_{CP} = 5,1 Hz, PCH₂CH₂ C₂); ³¹P-NMR (CDCl₃) δ (ppm): 37,05; HR-MS (MeOH, ESI, m/z): ber. 440,44, gef. 440,41 [M].

### Synthesebeispiele 9 und 10 - Herstellung von Vergleichsprimern mit Vinyl (Enyl) als PG und Phosphonsäure (Phn) als AM

### Synthesebeispiel 9 - Herstellung von Hex-5-en-1-ylphosphonsäure (Phn-6C-Enyl) (S9)

### Stufe 1: Herstellung von Hex-5-en-1-ylphosphonsäurediethylester (PhnEt-6C-Enyl)

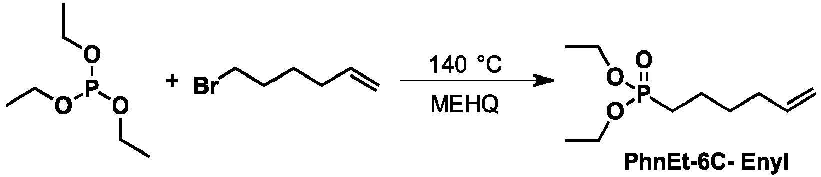

Eine mit einer Vigreux-Kolonne ausgestattete Destillationsapparatur wurde mit 9,8 g (1 Äqu., 60 mmol) Hex-5-en-1-ylbromid, 12,0 g (1,2 Äqu., 72 mmol), Triethylphosphit und 11 mg p-Methoxyphenol (Hydrochinonmonomethylether, MEHQ) unter ArgonAtmosphäre beschickt. Die Lösung wurde 24 h lang auf 140 °C erhitzt, wobei als Nebenprodukt Ethylbromid (Kp. 38 °C) gebildet wurde, das während der Reaktion abdestilliert wurde. Der Reaktionsfortschritt wurde mittels ³¹P-NMR überwacht. Nach vollständiger Reaktion wurde das Produkt im Hochvakuum (0,011 mbar) bei 75 °C fraktioniert abdestilliert, wobei wurden 8,99 g (68 % d. Th.) des gewünschten Phosphonats PhnEt-6C-Enyl als farblose Flüssigkeit erhalten wurden.
DC (PE/EE 1:2) R_{f} = 0,23; RI n_{D}^{20°C}: 1,4540; ¹H-NMR (400 MHz, CDCl₃) δ (ppm): 5,84-5,69 (m, 1H, CH=CH₂), 5,04-4,89 (m, 2H, CH=CH₂), 4,16-4,00 (m, 4H, OCH₂CH₃), 2,11-2,00 (m, 2H, CH₂CH), 1,78-1,53 (m, 4H, CH₂), 1,51-1,41 (m, 2H, PCH₂), 1,30 (t, ³J_{HH} = 7,1 Hz, 6H, OCH₂CH₃); ¹³C-NMR (101 MHz, CDCl₃) δ (ppm): 138,31 (s, CH=CH₂), 114,92 (s, CH=CH₂), 61,50 (d, J = 6,5 Hz, OCH₂CH₃), 33,31 (d, ⁴J_{CP} = 1,4 Hz, CH₂CH), 29,88 (d, ³J_{CP} = 17,0 Hz, PCH₂ CH₂CH₂), 26,72-24,62 (d, ¹J_{CP} = 140,6 Hz, PCH₂, 22,03 (d, ²J_{CP} = 5,2 Hz, PCH₂CH₂), 16,62 (d, ³J_{CP} = 6,1 Hz, OCH₂CH₃); ³¹P-NMR (CDCl₃) δ (ppm): 32,31.

### Stufe 2: Herstellung von Hex-5-en-1-ylphosphonsäure (Phn-6C-Enyl) (S9)

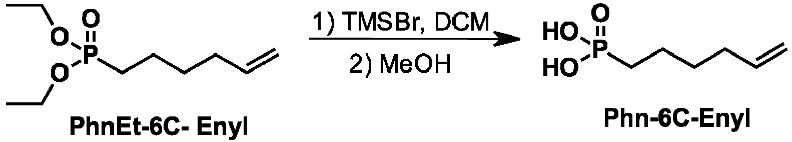

Die Synthese erfolgte analog zu Synthesebeispiel 3, Stufe 2, aus PhnEt-6C-Enyl (1 Äqu., 3,3 g, 15 mmol) mit TMSBr (3 Äqu., 6 ml, 45 mmol), wobei 2,51 g (84 % d. Th.) der gewünschten Phosphonsäure Phn-6C-Enyl als farbloser Feststoff isoliert wurden.
Fp.: 97,2-100,1 °C; ¹H-NMR (400 MHz, CDCl₃) δ (ppm): 9,47 (s, 2H, OH), 5,88-5,67 (m, 1H, CH=CH₂), 5,08-4,89 (m, 2H, CH=CH₂), 2,12-1,99 (m, 2H, CH₂CH), 1,85-1,55 (m, 4H, 2x CH₂), 1,55-1,41 (m, 2H, PCH₂); ¹³C-NMR (101 MHz, CDCl₃) δ (ppm): 138,17 (s, CH=CH₂), 114,88 (s, CH=CH₂), 33,16 (s, CH₂CH), 29,58 (d, ³J_{CP} = 17,0 Hz, PCH₂CH₂CH₂), 26,15-23,79 (d, ¹J_{CP} = 140,8 Hz, PCH₂), 21,49 (d, ²J_{CP} = 5,0 Hz, PCH₂CH₂); ³¹P-NMR (CDCl₃) δ (ppm): 37,41.

### Synthesebeispiel 10 - Herstellung von Undec-10-en-1-ylphosphonsäure (Phn-11C-Enyl) (S10)

### Stufe 1: Herstellung von Undec-10-en-1-ylphosphonsäurediethylester (PhnEt-11C-Enyl)

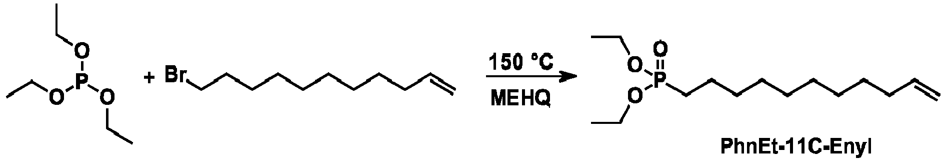

Die Synthese erfolgte analog zu Synthesebeispiel 8, Stufe 1, aus Undec-10-en-1-yl-bromid (1 Äqu., 8,2 g, 35 mmol), Triethylphosphit (1,3 Äqu., 8 ml, 45,5 mmol) und 20 mg MEHQ. Nach vollständiger Reaktion wurde das Produkt mittels Säulenchromatographie (EE) gereinigt, wobei 6,75 g (66 % d. Th.) des gewünschten Phosphonats PhnEt-11C-Enyl als gelbliche viskose Flüssigkeit isoliert wurden.
DC (EE) R_{f} = 0,38; RI n_{D}^{20°C}: 1,4462; ¹H-NMR (400 MHz, CDCl₃) δ (ppm): 5,86-5,71 (m, 1H, CH=CH₂), 5,01-4,93 (m, 1H, CH=CH₂), 4,93-4,87 (m, 1H, CH=CH₂), 4,16-3,99 (m, 4H, OCH₂CH₃), 2,09-1,94 (m, 2H, CH₂CH=CH₂), 1,78-1,64 (m, 2H, PCH₂), 1,63-1,51 (m, 2H, PCH₂CH₂), 1,41-1,20 (m, 18H, 2x OCH₂CH₃, 6x CH₂ C₃-C₈); ¹³C-NMR (101 MHz, CDCl₃) δ (ppm): 139,40 (s, CH=CH₂), 114,24 (s, CH=CH₂ C₁₁), 61,49 (d, J = 6,4 Hz, OCH₂CH₃), 33,90 (s, CH₂CH), 30,71 (d, ³J_{CP} = 17,0 Hz, PCH₂CH₂CH₂ C₃), 29,51 (s, CH₂ C₆), 29,42 (s, CH₂ C₇), 29, 19 (s, CH₂ C₅ C₈)), 29,02 (s, CH₂ C₄), 26,66-24,96 (d, ¹J_{CP} = 140,4 Hz, PCH₂ C₁), 22,51 (d, ²J_{CP} = 5,3 Hz, PCH₂CH₂ C₂), 16,60 (d, ³J_{CP} = 6,0 Hz, OCH₂CH₃); ³¹P-NMR (CDCl₃) δ (ppm): 32,65.

### Stufe 2: Herstellung von Undec-10-en-1-ylphosphonsäure (Phn-11C-Enyl) (S10)

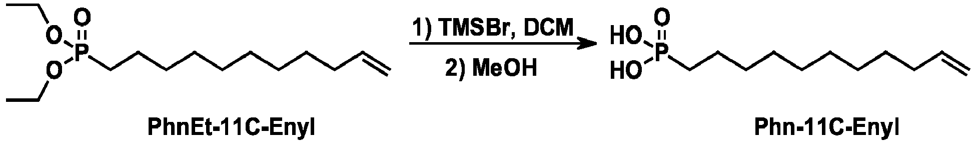

Die Synthese erfolgte analog zu Synthesebeispiel 3, Stufe 2, aus PhnEt-11C-Enyl (1 Äqu., 5,8 g, 20 mmol) mit TMSBr (3 Äqu., 8 ml, 60 mmol), wobei 4,65 g (99 % d. Th.) der gewünschten Phosphonsäure Phn-11C-Enyl als farbloser Feststoff isoliert wurden. Fp.: 74,2-76,9 °C; ¹H-NMR (400 MHz, CDCl₃) δ (ppm): 5,91-5,70 (m, 1H, CH=CH₂), 5,02-4,96 (m, 1H, CH=CH₂), 4,95-4,91 (m, 1H, CH=CH₂), 2,12-1,95 (m, 2H, CH₂CH=CH₂), 1,81-1,69 (m, 2H, PCH₂), 1,67-1,55 (m, 2H, PCH₂CH₂), 1,46-1,13 (m, 12H, 6x CH₂ C₃-C₈); ¹³C-NMR (101 MHz, CDCl₃) δ (ppm): 139,24 (s, CH=CH₂), 114,28 (s, CH=CH₂ C₁₁), 33,95 (s, CH₂CH), 30,56 (d, ³J_{CP} = 17,1 Hz, PCH₂CH₂CH₂ C₃), 29,56 (s, CH₂ C₆), 29,46 (s, CH₂ C₇), 29,24 (s, CH₂ C₈), 29,17 (s, CH₂ C₅), 29,07 (s, CH₂ C₄), 25,34 (d, ¹J_{CP} = 141 Hz, PCH₂ C₁), 22,12 (d, ²J_{CP} = 5,0 Hz, PCH₂CH₂ C₂); ³¹P-NMR (CDCl₃) δ (ppm): 37,52.

### Synthesebeispiel 11 - Herstellung eines Vergleichsprimers mit Vinyl (Enyl) als PG und 4-Brenzkatechyl (Catechol) als AM:

### 4-Allylbrenzkatechin (Catechol-3C-Enyl) (S11)

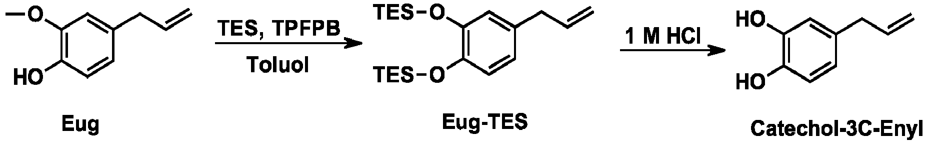

Die literaturbekannte Synthese erfolgte gemäß der Vorschrift in Donovan et al., RSC Adv. 4, 61927-61935 (2014), aus 4-Allyl-2-methoxyphenol (Eugenol, Eug) durch Silylierung mit Triethylsilan (TES) unter Abspaltung von CH₄ in Gegenwart von Tris(pentafluorphenyl)boran (TPFPB) und anschließende Hydrolyse des doppelt silylierten Zwischenprodukts Eug-TES mit 1 M wässriger HCl zum gewünschten Catechol-3C-Enyl. DC (PE:EE 4:1) R_{f} = 0,43; Fp.: 45,1-45,3 °C; ¹H-NMR (400 MHz, MeOD-d) δ (ppm): 6,89-6,53 (m, 3H, Ar-H), 6,03-5,80 (m, 1H, CH=CH₂), 5,13-5,06 (m, 1H, CH=CH₂), 5,02 (t, ²J_{HH} = 1,5 Hz, 1H, CH=CH₂), 3,28 (d, ³J_{HH} = 6,7 Hz, 2H, CH₂CH=CH₂); ¹³C-NMR (101 MHz, MeOD-d) δ (ppm): 143,39 (s, Ar-C_{q}-O), 141,66 (s, Ar-C_{q}-O), 137,61 (s, CH=CH₂), 133,31 (s, Ar-C_{q}), 121,05 (s, Ar-C), 115,72 (s, Ar-C), 115,62 (s, CH=CH₂), 115,36 (s, Ar-C), 39,51 (s, CH₂CH=CH₂).

### Beispiele 1 bis 9 - Herstellung von erfindungsgemäßen Primern mit Norbornenyl (Norb) als PG und Phosphonsäure (Phn) oder 4-Brenzkatechyl (Catechol) als AM

### Beispiel 1 - Herstellung von N-(4-Hydroxy-4,4-diphosphonobutyl)-5-norbornen-2-carbonsäureamid (N-(4-Hydroxy-4,4-diphosphonobutyl)alendronsäureamid) (Alendronat-Norb) (B1)

### Stufe 1: Herstellung von 5-Norbornen-2-carbonsäure-2,5-dioxopyrrolidinamid (Norb-NHS)

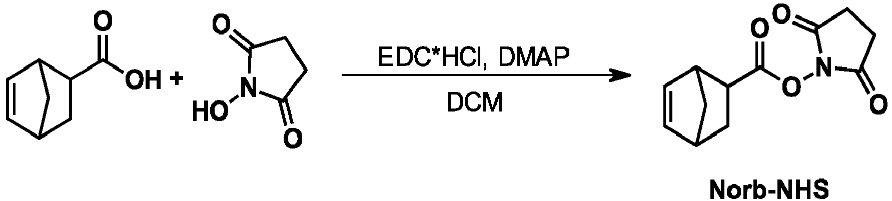

Die literaturbekannte Synthese erfolgte gemäß der Vorschrift in Werther et al., Chem. Eur. J. 23(72), 18216-18224 (2017), aus 5-Norbornen-2-carbonsäure und 1-Hydroxy-pyrrolidin-2,5-dion in Gegenwart von 1-Ethyl-3-(3-dimethylaminopropyl)carbodiimid-hydrochlorid (EDC·HCl) und 4-Dimethylaminopyridin (DMAP) in trockenem DCM.
DC (EE/PE, 3:1) R_{f} = 0,74; Fp.: 87,2-94,0 °C; ¹H-NMR (400 MHz, CDCl₃) δ (ppm): 6,21 (dd, ³J_{HH} = 3,0 Hz, ²J_{HH} = 5,3 Hz, 1H, CH=CH), 6,12 (dd, ³J_{HH} = 3,0 Hz, ²J_{HH} = 5,3 Hz, 1H, CH=CH), 3,43-3,35 (m, 1H, CH norb), 3,29-3,19 (m, 1H, CH norb), 2,98 (m, 1H, CHC=O), 2,81 (s, 4H, 2x CH₂), 2,09-1,95 (m, 1H, CH₂ norb), 1,57-1,40 (m, 3H, CH₂ Brücke + CH₂ norb), 1,37-1,29 (m, 1H, CH₂ Brücke); ¹³C-NMR (101 MHz, CDCl₃) δ (ppm): 171,75 (s, C=O), 170,08 (s, NC=O), 169,43 (s, NC=O), 138,24 (s, CH=CH), 135,38 (s, CH=CH), 49,76 (s, CH₂ Brücke endo), 47,24 (s, CH norb exo), 46,52 (s, CH norb endo), 46,50 (s, CH₂ Brücke exo), 42,63 (s, CH norb endo), 41,89 (s, CH norb exo), 40,72 (s, CHC=O endo), 40,40 (s, CHC=O exo), 31,08 (s, CH₂ norb exo) 29,68 (s, CH₂ norb endo), 25,72 (s, 2x CH₂).

### Stufe 2: Herstellung von N-(4-Hydroxy-4,4-diphosphonobutyl)-5-norbornen-2-carbonsäureamid-Natriumsalz (Alendronat-Norb) (B1)

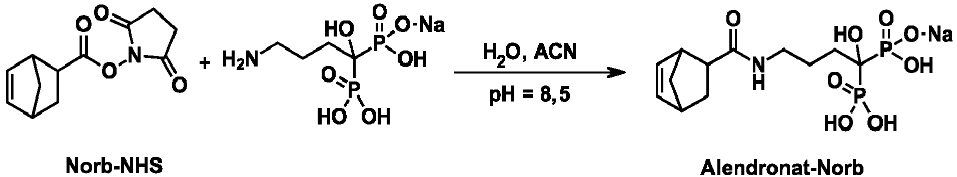

Alendronsäuremononatriumsalz-trihydrat (1 Äqu., 2,6 g, 8 mmol) wurde in 60 ml entionisiertem Wasser gelöst und der pH-Wert mit 1 M NaOH-Lösung auf 8,5 eingestellt. Norb-NHS (2 Äqu., 3,8 g, 16 mmol) wurde in einem zweiten Kolben in 60 ml Acetonitril gelöst und die organische Lösung wurde portionsweise zur wässrigen Alendronsäure-Lösung zugesetzt, wobei der pH-Wert nach jeder Portion gemessen und bei Bedarf wieder auf 8,5 eingestellt wurde. Nach 24 h Rühren bei Raumtemperatur wurden die Lösungsmittel abgedampft, und das Rohprodukt wurde aus Ethanol umkristallisiert, wobei 3,5 g (99 % d. Th.) der gewünschten Verbindung Alendronat-Norb als farbloser Feststoff isoliert wurden.
Fp.: 212,3 °C (Zers.); ¹H-NMR (400 MHz, D₂O) δ (ppm): 6,30-5,93 (m, 2H, CH=CH), 3,21 (t, ³J_{HH} = 6,9 Hz, 1H, CH norb), 3,18-3,13 (m, 2H, CH₂NH), 3,03-2,96 (m, 1H, CH norb), 2,95-2,90 (m, 1H, C_{q}OH), 2,67 (s, 3H, 3x POH), 2,49-2,35 (m, 1H, CHC=O), 1,99-1,87 (m, 3H, C_{q}CH₂, CH₂ norb), 1,84-1,71 (m, 2H, CH₂ norb, CH₂ Brücke), 1,41-1,35 (m, 1H, CH₂ Brücke), 1,35-1,30 (m, 2H, CH₂CH₂NH); ¹³C-NMR (101 MHz, D₂O) δ (ppm): 178,77 (s, C=O), 138,32 (s, CH=CH), 132,08 (s, CH=CH), 73,88 (t, ¹J_{CP} = 132,2 Hz, C_{q}), 49,50 (s, CH₂ Brücke endo), 46,43 (m, CH norb endo/exo), 45,97 (s, CH₂ Brücke exo), 44,12 (s, CH norb endo), 43,97 (s, CH norb exo), 42,56 (s, CHC=O endo), 41,30 (s, CHC=O exo), 40,04 (s, CqCH₂CH₂CH₂NH), 31,19 (s, CqCH₂CH₂CH₂NH), 29,12 (s, CH₂ norb exo), 28,75 (s, CH₂ norb endo), 23,60 (t, ³J_{CP} = 6,4 Hz, CqCH₂CH₂CH₂NH); ³¹P-NMR (D₂O) δ (ppm): 18,31; Elementaranalyse: ber. für C₁₂H₂₀NNaO₈P₂: C-36,84, H-5,15, N-3,58, Na-5,88, 0-32,72, P-15,83; gef.: C-36,85, H-4,86, N-3,62, Na-5,01, 0-33,32, P-15,79.

### Beispiel 2 - Herstellung von N-[2-(3,4-Dihydroxyphenyl)ethyl]-5-norbornen-2-carbonsäureamid (Catechol-2C-Norb) (B2)

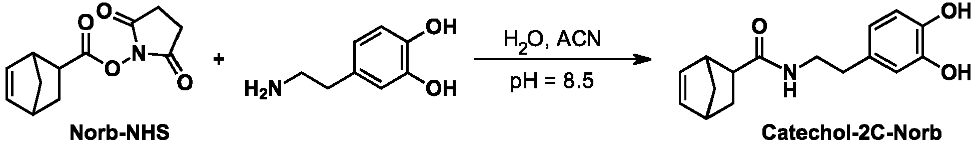

Die Synthese erfolgte analog zu Synthesebeispiel 12, Stufe 2, aus Norb-NHS (1,5 Äqu., 2,3 g, 10 mmol) und Dopamin-Hydrochlorid (1 Äqu., 2,4 g, 15 mmol) in einem Wasser/Acetonitril-Gemisch bei pH 8,5. Das durch Abdampfen der Lösungsmittel erhaltene Rohprodukt wurde mittels Säulenchromatographie (Etthylacetat:Petrolether, EE:PE, 1:1-3:1) gereinigt, was 1,4 g (60 % d. Th.) der gewünschten Verbindung Catechol-2C-Norb als farblose viskose Flüssigkeit ergab.
DC (EE:PE, 3:1) R_{f} = 0,42; ¹H-NMR (400 MHz, CDCl₃) δ (ppm): 6,82 (d, ³J_{HH} = 8,0 Hz, 1H, CH Ar), 6,73 (d, ³J_{HH} = 2,0 Hz, 1H, CH Ar), 6,54 (dd, ³J_{HH} = 8,1 Hz, ⁴J_{HH} = 2,0 Hz, 1H, CH Ar), 6,17 (dd, ³J_{HH} = 5,7, ⁴J_{HH} = 3,1 Hz, 1H, CH=CH), 5,82 (dd, ³J_{HH} = 5,7, ⁴J_{HH} = = 2,8 Hz, 1H, CH=CH), 5,63 (t, ³J_{HNCH} = 5,8 Hz, 1H, NH), 3,42 (q, ³J_{HH} = 6,7 Hz, 2H, CH₂NH), 3,06 (s, 1H, CH norb), 2,95-2,76 (m, 2H, CH norb, CHC=O norb), 2,64 (t, ³J_{HH} = 7,0 Hz, 2H, C_{Ar}CH₂), 2,03-1,82 (m, 1H, CH norb), 1,50-1,34 (m, 1H, CH₂ Brücke), 1,34-1,09 (m, 1H, CH₂ Brücke); ¹³C-NMR (101 MHz, CDCl₃) δ (ppm): 176,47 (s, C=O), 145,04 (s, C-OH), 143,39 (s, C-OH), 138,31 (s, CH=CH), 132,26 (s, CH=CH), 129,83 (s, C_{Ar}CH₂), 120,50 (s, CH Ar), 115,70 (s, CH Ar), 115,41 (s, CH Ar), 50,22 (s, CH₂ Brücke), 46,42 (s, CH norb), 45,11 (s, CH norb), 42,89 (CHC=O norb), 41,10 (s, CH₂NH), 35,67 (s, C_{Ar}CH₂), 30,43 (s, CH₂ norb); Elementaranalyse: ber. für C₁₆H₁₉NO₃: C-70,31, H-7,01, N-5,12, O-17,56; gef.: C-70,58, H-7,05, N-4,91, O-18,35.

### Beispiel 3 - Herstellung von 5-Norbornen-2-ylcarbonyloxymethylphosphonsäure (Phn-1C-Norb) (B3)

### Stufe 1: Herstellung von 5-Norbornen-2-carbonsäurechlorid (Norb-Cl)

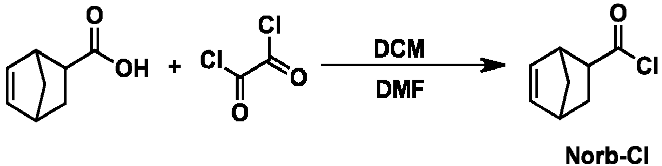

Die literaturbekannte Synthese erfolgte gemäß der Vorschrift in Chae et al., Macromolecules 51, 3458-3466 (2018), aus 5-Norbornen-2-carbonsäure und Oxalylchlorid in trockenem DCM in Gegenwart katalytischer Mengen an Dimethylformamid (DMF), wobei das gewünschte Norb-Cl durch Destillation als farblose Flüssigkeit erhalten wurde.
¹H-NMR (400 MHz, CDCl₃) δ (ppm): 6,32-6,17 (m, 1H, CH=CH endo/exo), 6,17-5,99 (m, 1H, CH=CH exo/endo), 3,49-2,71 (m, 3H, CH), 2,07-1,87 (m, 1H, CH₂, CH₂ Brücke), 1,57-1,38 (m, 2H, CH₂, CH₂ Brücke), 1,37-1,28 (m, 1H, CH₂, CH₂ Brücke); ¹³C-NMR (101 MHz, CDCl₃) δ (ppm): 175,19 (s, C=O), 139,17 (s, CH=CH exo), 138,83 (s, CH=CH endo), 135,02 (s, CH=CH exo), 131,75 (s, CH=CH endo), 56,56 (s, CHC=O endo), 56,45 (s, CHC=O exo), 49,36 (s, CH₂ Brücke), 47,29 (s, CH endo), 47,03 (s, CH exo), 46,43 (s, CH₂ Brücke), 43,00 (s, CH endo), 42,00 (s, CH exo), 31,29 (s, CH₂ exo), 30,21 (s, CH₂ endo).

### Stufe 2: Herstellung von 5-Norbornen-2-ylcarbonyloxymethylphosphonsäurediethyl-ester (PhnEt-1C-Norb)

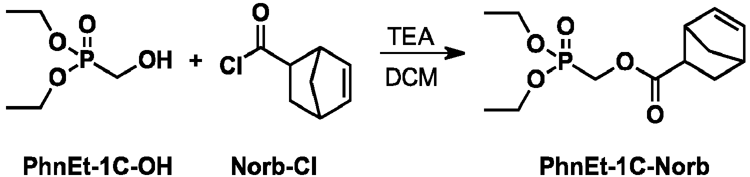

Phosphonsäurediethyl(hydroxymethyl)ester PhnEt-1C-OH (1 Äqu., 2,2 ml, 15 mmol) wurde unter Rühren langsam (binnen 15 min) zu einem Gemisch aus Norb-Cl (1,5 Äqu., 3,5 g, 22,5 mmol) und Triethylamin (TEA) (1,8 Äqu., 3,7 ml, 27 mmol) in 50 ml trockenem DCM zugesetzt, wonach das Reaktionsgemisch 1 h lang bei 0 °C und 24 h lang bei Raumtemperatur gerührt wurde. Das Lösungsmittel und die nichtumgesetzten Edukte wurden abgedampft, und das Rohprodukt wurde erneut in 50 ml DCM gelöst. Die Lösung wurde mit Kochsalzlösung (3x 25 ml) gewaschen und die organische Phase über MgSO₄ getrocknet. Das Lösungsmittel wurde abgedampft und das Produkt im Vakuum bis zur Gewichtskonstanz getrocknet, was 4,3 g (99 % d. Th.) des gewünschten PhnEt-1C-Norb als braune viskose Flüssigkeit ergab.
DC (EE) R_{f}= 0,53; ¹H-NMR (400 MHz, CDCl₃) δ (ppm): 6,26-5,90 (m, 2H, CH=CH), 4,47-4,23 (m, 2H, PCH₂O), 4,22-4,10 (m, 4H, OCH₂CH₃), 3,27-2,84 (m, 3H, 2x CH norb, CHC=O), 2,00-1,87 (m, 1H, CH₂ norb), 1,57-1,23 (m, 9H, CH₂ norb, CH₂ Brücke, 2x OCH₂CH₃); ¹³C-NMR (101 MHz, CDCl₃) δ (ppm): 175,47 (s, exo C=O), 173,89 (s, endo C=O), 138,34 (s, exo CH=CH), 137,99 (s, endo CH=CH), 135,69 (s, exo CH=CH), 132,42 (s, endo CH=CH), 62,89-62,73 (m, OCH₂CH₃), 56,85 (d, ¹J_{CP} = 168,5 Hz, CH₂P C₁ endo), 56,61 (d, ¹J_{CP} = 168,7 Hz, CH₂P C₁ exo), 49,73 (s, CH₂ Brücke), 46,75 (s, exo CH norb), 46,44 (s, CH₂ Brücke), 45,89 (s, endo CH norb), 43,15 (s, endo CH norb), 42,93 (s, exo CH norb), 42,63 (s, endo CH norb), 41,77 (s, exo CH norb), 30,57 (s, exo CH₂ norb), 29,51 (s, endo CH₂ norb), 16,53 (d, ³J_{CP} = 5,9 Hz, OCH₂CH₃); ³¹P-NMR (CDCl₃) δ (ppm): 19,47; HR-MS (MeOH, ESI, m/z): ber. 288,28, gef. 288,41 [M].

### Stufe 3: Herstellung von 5-Norbornen-2-ylcarbonyloxymethylphosphonsäure (Phn-1C-Norb) (B3)

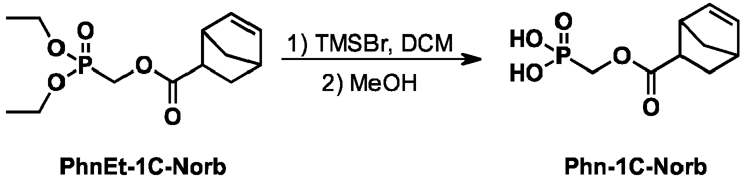

Die Synthese erfolgte analog zu Synthesebeispiel 3, Stufe 2, aus PhnEt-1C-Norb (1 Äqu., 5,2 g, 18 mmol) mit TMSBr (3 Äqu., 6 ml, 45 mmol), wobei 4,17 g (99 % d. Th.) der gewünschten Phosphonsäure Phn-1C-Norb als braune viskose Flüssigkeit isoliert wurden.
¹H-NMR (400 MHz, CDCl₃) δ (ppm): 8,17 (s, 2H), 6,23-5,87 (m, 2H, CH=CH), 4,51-4,27 (m, 2H, PCH₂O), 3,30-3,14 (m, 1H, CH norb), 3,12-2,99 (m, 1H, CH norb), 2,97-2,87 (m, 1H, CHC=O), 2,01-1,86 (m, 1H, CH₂ norb), 1,62-1,19 (m, 3H, CH₂ norb, CH₂ Brücke); ¹³C-NMR (101 MHz, CDCl₃) δ (ppm): 176,13 (s, exo C=O), 174,86 (s, endo C=O), 138,34 (s, exo CH=CH), 137,99 (s, endo CH=CH), 135,69 (s, exo CH=CH), 132,42 (s, endo CH=CH), 57,63 (d, ¹J_{CP} = 169,3 Hz, CH₂P C₁ exo), 56,96 (d, ¹J_{CP} = 169,2 Hz, CH₂P C₁ endo), 49,78 (s, CH₂ Brücke), 46,80 (s, exo CH norb), 46,48 (s, CH₂ Brücke), 46,03 (s, endo CH norb), 43,20 (s, endo CH norb), 42,99 (s, exo CH norb), 42,72 (s, endo CH norb), 41,79 (s, exo CH norb), 30,61 (s, exo CH₂ norb), 29,51 (s, endo CH₂ norb); ³¹P-NMR (CDCl₃) δ (ppm): 20,93; HR-MS (MeOH, ESI, m/z): ber. 288,28, gef. 288,28 [M].

### Beispiel 4 - Herstellung von 6-(5-Norbornen-2-ylcarbonyloxy)hexylphosphonsäure (Phn-6C-Norb) (B4)

### Stufe 1: Herstellung von 6-(5-Norbornen-2-ylcarbonyloxy)hexylphosphonsäurediethyl-ester (PhnEt-6C-Norb)

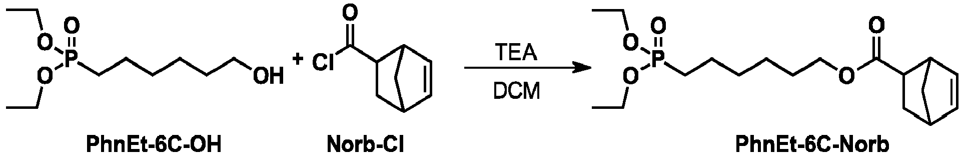

Die Synthese erfolgte analog zu Beispiel 3, Stufe 2, aus Phosphonsäurediethyl(6-hydroxyhexyl)ester PhnEt-6C-OH (1 Äqu., 2,9 g, 12 mmol) mit Norb-Cl (2,5 Äqu., 4,7 g, 30 mmol) und TEA (2,8 Äqu., 4,7 ml, 33 mmol) in DCM, wobei mittels Säulenchromatographie (PE:EE, 1:1) 3,01 g (70 % d. Th.) des gewünschten Esters PhnEt-6C-Norb als braune viskose Flüssigkeit isoliert wurden.
DC (EE/MeOH, 19:1) R_{f}= 0,5; ¹H-NMR (400 MHz, CDCl₃) δ (ppm): 6,21-5,84 (m, 2H, CH=CH), 4,15-3,90 (m, 6H, OCH₂CH₃, CH₂OC=O), 3,21-3,13 + 3,04-2,98 (m, 1H, CH norb), 2,95-2,81 + 2,25-2,13 (m, 2H, CH norb, CHC=O), 1,94-1,79 (m, 1H, CH₂ norb), 1,77-1,52 (m, 5H, CH₂ norb, CH₂ Brücke, CH₂P), 1,44-1,20 (m, 14H, 2x OCH₂CH₃, 4x CH₂ alkyl); ¹³C-NMR (101 MHz, CDCl₃) δ (ppm): 176,37 (exo C=O), 174,87 (endo C=O), 138,12 (s, exo CH=CH), 137,83 (s, endo CH=CH), 135,85 (s, exo CH=CH), 132,42 (s, endo CH=CH), 64,28 (d, J = 24,4 Hz, CH₂OC=O), 61,50 (d, ²J_{CP} = 6,5 Hz, OCH₂CH₃), 49,71 (s, CH₂ Brücke), 46,69 (s, exo CH norb), 46,44 (s, CH₂ Brücke), 45,80 (s, endo CH norb), 43,44 (s, endo CH norb), 43,28 (s, exo CH norb), 42,62 (s, endo CH norb), 41,71 (s, exo CH norb), 30,40 (s, exo CH₂ norb), 30,27 (d, ³J_{CP} = 17,0 Hz, CH₂CH₂CH₂P C₃), 29,27 (s, CH₂CH₂O C₅), 28,53 (s, endo CH₂ norb), 25,72 (d, ¹J_{CP}= 140,8 Hz, CH₂P C₁), 25,60 (d, ⁴J_{CP} = 1,4 Hz, CH₂C₄), 22,45 (d, ²J_{CP} = 5,1 Hz, PCH₂CH₂ C₂), 16,57 (d, ³J_{CP} = 5,9 Hz, OCH₂CH₃); ³¹P-NMR (CDCl₃) δ (ppm): 32,29.

### Stufe 2: Herstellung von 6-(5-Norbornen-2-ylcarbonyloxy)hexylphosphonsäure (Phn-6C-Norb) (B4)

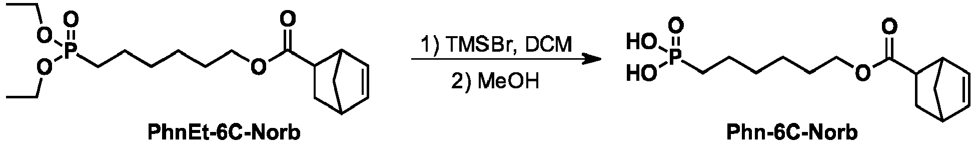

Die Synthese erfolgte analog zu Synthesebeispiel 3, Stufe 2, aus PhnEt-6C-Norb (1 Äqu., 2,9 g, 8 mmol) mit TMSBr (2 Äqu., 2,1 ml, 16 mmol), wobei 2,37 g (99% d. Th.) der gewünschten Phosphonsäure Phn-6C-Norb als braune viskose Flüssigkeit isoliert wurden.
¹H-NMR (400 MHz, CDCl₃) δ (ppm): 8,27 (s, 2H, OH), 6,24-5,84 (m, 2H, CH=CH), 4,12-3,95 (m, 2H, CH₂OC=O alkyl), 3,24-3,14 (m, 1H, CH norb), 3,07-2,84 (m, 2H, CH norb, CHC=O), 2,25-2,15 (m, 1H, CH₂ norb), 1,95-1,11 (m, 13H, CH₂ norb, CH₂ Brücke, CH₂P, 4x CH₂ alkyl); ¹³C-NMR (101 MHz, CDCl₃) δ (ppm): 176,60 (s, exo C=O), 175,15 (s, endo C=O), 138,17 (s, exo CH=CH), 137,92 (s, endo CH=CH), 135,90 (s, exo CH=CH), 132,46 (s, endo CH=CH), 64,39 (d, J = 24,3 Hz, CH₂OC=O), 49,77 (s, CH₂ Brücke), 46,75 (s, exo CH norb), 46,51 (s, CH₂ Brücke), 45,87 (s, endo CH norb), 43,52 (s, endo CH norb), 43,35 (s, exo CH norb), 42,67 (s, endo CH norb), 41,77 (s, exo CH norb), 30,48 (s, exo CH₂ norb), 30,04 (d, ³J_{CP} = 17,0 Hz, CH₂CH₂CH₂P), 29,36 (s, CH₂CH₂O C₅), 28,51 (s, endo CH₂ norb), 25,54 (s, CH₂C₄), 25,43 (d, ¹J_{CP} = 141 Hz, CH₂P C₁), 22,07 (d, ²J_{CP} = 5,0 Hz, PCH₂CH₂ C₂); ³¹P-NMR (CDCl₃) δ (ppm): 36,87; HR-MS (MeOH, ESI, m/z): ber. 302,35, gef. 302,35 [M].

### Beispiel 5- Herstellung von 6-(2,3-Bis(5-norbornen-2-ylcarbonyloxy)propylthio)hexyl-phosphonsäure (Phn-6C-bisNorb) (B5)

### Stufe 1: Herstellung von 6-(2,3-Dihydroxypropylthio)hexylphosphonsäurediethylester (PhnEt-6C-thioglyc)

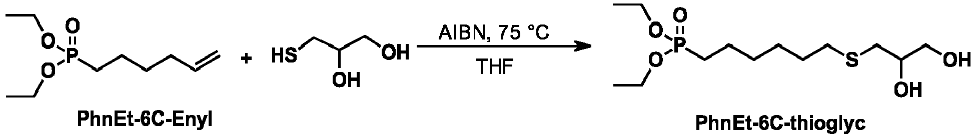

Die literaturbekannte Synthese erfolgte gemäß der Vorschrift in Catel et al., Macromol. Mater. Eng. 300, 1010-1022 (2015), aus Hex-5-en-1-ylphosphonsäurediethylester (PhnEt-6C-Enyl) aus Synthesebeispiel 9, Stufe 1, durch Addition von Thioglykol in Gegenwart von Azobis(isobutyronitril) (AIBN) in THF.
DC (EE/MeOH, 9:1) R_{f}= 0,9; RI n_{D}^{20°c}: 1,4880; ¹H-NMR (400 MHz, CDCl₃) δ (ppm): 4,17-4,00 (m, 4H, OCH₂CH₃), 3,83-3,68 (m, 2H, CHOH, CH₂OH), 3,63-3,54 (m, 1H, CH₂OH), 2,71 (dd, ²J_{HH} = 13,7, ³J_{HH} = 5,0 Hz, 1H, SCH₂CH), 2,62 (dd, ²J_{HH} = 13,7, ³J_{HH} = 7,8 Hz, 1H, SCH₂CH), 2,56 (t, ³J_{HH} = 7,4 Hz, 2H, CH₂CH₂S), 1,81-1,68 (m, 2H, PCH₂), 1,68-1,54 (m, 4H, PCH₂CH₂, CH₂CH₂CH₂S), 1,48-1,39 (m, 4H, PCH₂CH₂CH₂, CH₂CH₂S), 1,33 (t, ³J_{HH} = 7,1 Hz, 6H, OCH₂CH₃); ¹³C-NMR (101 MHz, CDCl₃) δ (ppm): 70,32 (s, CHOH), 65,54 (s, CH₂OH), 61,75 (d, ³J_{CP} = 6,7 Hz, OCH₂CH₃), 35,78 (s, SCH₂CH), 32,30 (s, CH₂CH₂S), 29,95 (d, ³J_{CP} = 16,8 Hz, PCH₂CH₂CH₂), 29,30 (s, CH₂CH₂CH₂S), 28,11 (s, CH₂CH₂S), 26,64-24,44 (d, ¹J_{CP} = 140,8 Hz, PCH₂), 22,21 (d, ²J_{CP} = 5,1 Hz, PCH₂CH₂), 16,51 (d, ³J_{CP} = 6,1 Hz, OCH₂CH₃); ³¹P-NMR (CDCl₃) δ (ppm): 32,60.

### Stufe 2: Herstellung von 6-(2,3-Bis(5-norbornen-2-ylcarbonyloxy)propylthio)hexyl-phosphonsäurediethylester (PhnEt-6C-bisNorb)

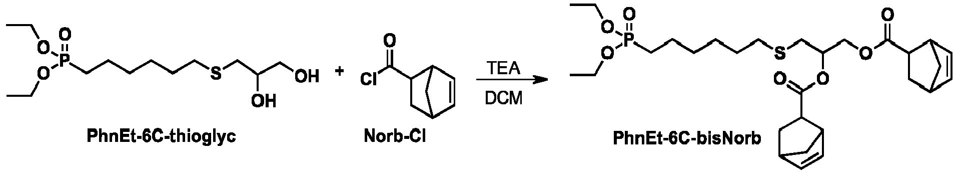

Die Synthese erfolgte analog zu Beispiel 3, Stufe 2, aus PhnEt-6C-thioglyc (1 Äqu., 3,3 g, 10 mmol) mit Norb-Cl (2,3 Äqu., 3,6 g, 23 mmol) und TEA (2,5 Äqu., 3,5 ml, 25 mmol) in trockenem DCM, wobei mittels Säulenchromatographie (EE) 3,0 g (73 % d. Th.) des gewünschten Esters PhnEt-6C-bisNorb als rote viskose Flüssigkeit isoliert wurden.
DC (EE) R_{f} = 0,37; ¹H-NMR (400 MHz, CDCl₃) δ (ppm): 6,24-5,87 (m, 4H, 2x CH=CH), 5,23-4,98 (m, 1H, CHO), 4,46-4,15 (m, 2H, CH₂O), 4,09 (m, 4H, OCH₂CH₃), 3,25-3,16 (m, 2H, 2x CH norb), 3,12-2,87 (m, 4H, 2x CH norb, SCH₂CH), 2,74-2,62 (m, 2H, CH₂SCH₂ C₆), 2,60-2,49 (m, 2H, CHC=O), 1,99-1,84 (m, 2H, CH₂ norb), 1,79-1,22 (m, 22H, 2x CH₂ Brücke, 5x CH₂ C₁-C₅, CH₂ norb, 2x OCH₂CH₃); ¹³C-NMR (101 MHz, CDCl₃) δ (ppm): 174,14 (m, 2x C=O), 138,14 (s, CH=CH), 137,80 (s, CH=CH), 135,67 (s, CH=CH), 132,39 (s, CH=CH), 70,55-70,34 (m, CHO), 64,48-62,74 (m, CHCH₂O), 61,42 (d, J = 6,5 Hz, OCH₂CH₃), 49,64 (s, CH₂ Brücke), 46,35 (s, CH₂ norb), 45,82-45,70 (m, CH norb), 43,42-43,25 (m, CHC=O), 42,59-42,49 (m, CH norb), 32,58 (s, SCH₂CH), 32,34 (s, CH₂SCH₂), 30,18 (d, ³J_{CP} = 17,0 Hz, PCH₂CH₂CH₂), 29,24 (s, CH₂CH₂CH₂S), 28,26 (s, CH₂CH₂S), 25,65 (d, ¹J_{CP} = 140,7 Hz, PCH₂), 22,36 (d, ²J_{CP} = 5,2 Hz, PCH₂CH₂), 16,52 (d, ³J_{CP} = 6,0 Hz, OCH₂CH₃); ³¹P-NMR (CDCl₃) δ (ppm): 32,34.

### Stufe 3: Herstellung von 6-(2,3-Bis(5-norbornen-2-ylcarbonyloxy)propylthio)hexyl-phosphonsäure (Phn-6C-bisNorb) (B5)

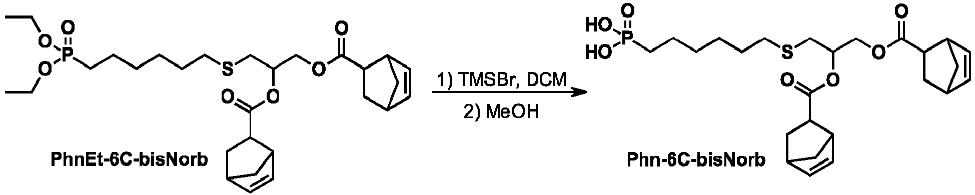

Die Synthese erfolgte analog zu Synthesebeispiel 3, Stufe 2, aus PhnEt-6C-bisNorb (1 Äqu., 2,3 g, 4 mmol) mit TMSBr (2,3 Äqu., 1,2 ml, 9,2 mmol), wobei 1,86 g (91 % d. Th.) der gewünschten Phosphonsäure Phn-6C-bisNorb als braune viskose Flüssigkeit isoliert wurden.
¹H-NMR (400 MHz, CDCl₃) δ (ppm): 6,24-5,86 (m, 4H, 2x CH=CH), 5,25-4,98 (m, 1H, CHO), 4,46-4,08 (m, 2H, CH₂O), 3,26-3,14 (m, 2H, 2x CH norb), 3,12-2,87 (m, 4H, 2x CH norb, SCH₂CH), 2,77-2,63 (m, 2H, CH₂SCH₂ C₆), 2,60-2,50 (m, 2H, CHC=O), CH₂ norb), 1,99-1,21 (m, 18H, 2x CH₂ Brücke, 5x CH₂ C₁-C₅, 2x CH₂ norb); ¹³C-NMR (101 MHz, CDCl₃) δ (ppm): 175,99-174,17 (m, 2x C=O), 138,28 (s, CH=CH), 137,99 (s, CH=CH), 135,79 (s, CH=CH), 132,47 (s, CH=CH), 70,80-70,39 (m, CHO), 64,19-63,51 (m, CHCH₂O), 50,78 (s, CH₂ norb), 49,74 (s, CH₂ Brücke), 45,82-45,70 (m, CH norb), 43,42-43,25 (m, CHC=O), 42,59-42,49 (m, CH norb), 32,64 (s, SCH₂CH), 32,41 (s, CH₂SCH₂), 30,18 (d, ³J_{CP} = 17,0 Hz, PCH₂CH₂CH₂), 29,34 (s, CH₂CH₂CH₂S), 28,25 (s, CH₂CH₂S), 25,58 (s, ¹J_{CP} = 140,9 Hz, PCH₂, 22,08 (d, ²J_{CP} = 5,2 Hz, PCH₂CH₂); ³¹P-NMR (CDCl₃) δ (ppm): 36,46; HR-MS (MeOH, ESI, m/z): ber. 512,62, gef. 512,62 [M].

### Beispiel 6- Herstellung von 6-(2,3-Bis(5-norbornen-2-ylcarbonyloxy)propylthio)hexan-1,1-diyl-bis(phosphonsäure) (bisPhn-6C-bisNorb) (B6)

### Stufe 1: Herstellung von 6-(2,3-Dihydroxypropylthio)hexan-1,1-diyl-bis(phosphon-säurediethylester) (bisPhnEt-6C-thioglyc)

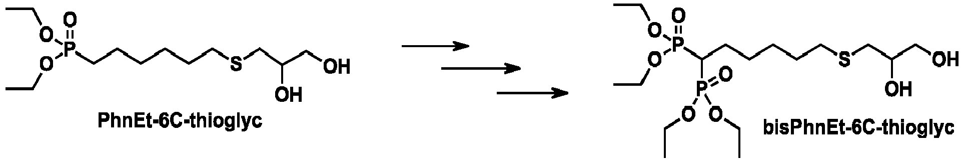

Die literaturbekannte Synthese erfolgte gemäß der Vorschrift in Catel et al., Macromol. Mater. Eng. 300, 1010-1022 (2015), aus 6-(2,3-Dihydroxypropylthio)hexylphosphonsäurediethylester (PhnEt-6C-thioglyc) durch schützen des Diols mit 2,2-Dimethoxypropan, anschließender Einführung einer zweiten Phosphonsäurediethylester-Gruppierung mittels Diethylchlorphosphat und gefolgt vom Entschützen des Diols.
DC (EE) R_{f}= 0,18; ¹H-NMR (400 MHz, CD₃OD) δ (ppm): 4,23-4,11 (m, 8H, 4 x OCH₂CH₃), 3,75-3,67 (m, 1H, CHO), 3,59 (dd, ³J_{HH} = 4,7 Hz, ²J_{HH} = 11,2 Hz, 1H, CHCH₂O), 3,53 (dd, ³J_{HH} = 5,8 Hz, ²J_{HH} = 11,2 Hz, 1H, CHCH₂O), 2,71 (dd, ³J_{HH} = 5,7 Hz, ²J_{HH} = 13,5 Hz, 1H, SCH₂CH), 2,64-2,45 (m, 4H, P₂CH C₁ + CH₂SCH₂ C₆ + SCH₂CH), 1,96-1,84 (m, 2H, CH₂ C₂), 1,61 (m, 4H, CH₂CH₂S Cs, CH₂ C₄), 1,48-1,40 (m, 2H, CH₂ C₃), 1,35 (t, ³J_{HH} = 7,1 Hz, 12H, 4x OCH₂CH₃); ¹³C-NMR (101 MHz, CD₃OD) δ (ppm): 72,86 (s, CHCH₂O), 65,96 (s, CHCH₂O), 64,18 (d, ²J_{CP} = 6,7 Hz, 4 x OCH₂CH₃), 36,28 (t, ¹J_{CP} = 133,3 Hz, P₂CH C₁), 33,37 (s, SCH₂CH), 30,31 (s, CH₂SCH₂ C₆), 29,38 (s, CH₂CH₂CH₂S C₄), 25,39 (t, ²J_{CP} = 5,0 Hz, P₂CHCH₂ C₂), 16,74 (d, ³J_{CP} = 6,0 Hz, 2 x OCH₂CH₃), 16,70 (d, ³J_{CP} = 6,0 Hz, 2 x OCH₂CH₃); ³¹P-NMR (CD₃OD) δ (ppm): 24,07.

### Stufe 2: Herstellung von 6-(2,3-Bis(5-norbornen-2-ylcarbonyloxy)propylthio)hexan-1,1-diyl-bis(phosphonsäurediethylester) (bisPhnEt-6C-bisNorb)

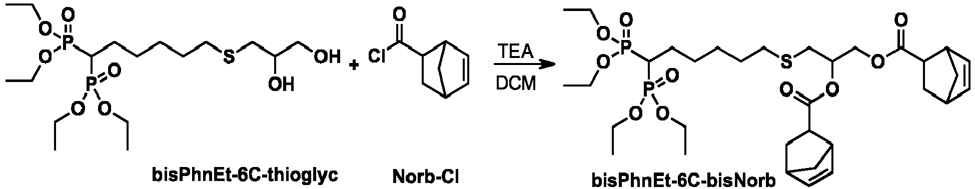

Die Synthese erfolgte analog zu Beispiel 3, Stufe 2, aus bisPhn-6C-thioglyc (1 Äqu., 9,9 g, 22 mmol) mit Norb-Cl (2,5 Äqu., 8,6 g, 55 mmol) und TEA (2,8 Äqu., 6,2 g, 61,6 mmol) in trockenem DCM, wobei mittels Säulenchromatographie (EE) 11,24 g (73 % d. Th.) des gewünschten Esters bisPhnEt-6C-bisNorb als rote viskose Flüssigkeit isoliert wurden.
DC (EE) R_{f} = 0,53; ¹H-NMR (400 MHz, CDCl₃) δ (ppm): 6,26-5,90 (m, 2H, CH=CH), 4,47-4,23 (m, 2H, PCH₂O), 4,22-4,10 (m, 4H, OCH₂CH₃), 3,27-2,84 (m, 3H, 2x CH norb, CHC=O), 2,00-1,87 (m, 1H, CH₂ norb), 1,57-1,23 (m, 9H, CH₂ norb, CH₂ Brücke, 2x OCH₂CH₃); ¹³C-NMR (101 MHz, CDCl₃) δ (ppm): 175,47 (s, exo C=O), 173,89 (s, endo C=O), 138,34 (s, exo CH=CH), 137,99 (s, endo CH=CH), 135,69 (s, exo CH=CH), 132,42 (s, endo CH=CH), 62,89-62,73 (m, OCH₂CH₃), 56,85 (d, ¹J_{CP} = 168,5 Hz, CH₂P C₁ endo), 56,61 (d,¹J_{CP} = 168,7 Hz, CH₂P C₁ exo), 49,73 (s, CH₂ Brücke), 46,75 (s, exo CH norb), 46,44 (s, CH₂ Brücke), 45,89 (s, endo CH norb), 43,15 (s, endo CH norb), 42,93 (s, exo CH norb), 42,63 (s, endo CH norb), 41,77 (s, exo CH norb), 30,57 (s, exo CH₂ norb), 29,51 (s, endo CH₂ norb), 16,53 (d, ³J_{CP} = 5,9 Hz, OCH₂CH₃); ³¹P-NMR (CDCl₃) δ (ppm): 19,47; HR-MS (MeOH, ESI, m/z): ber. 288,28, gef. 288,41 [M].

### Stufe 3: Herstellung von 6-(2,3-Bis(5-norbornen-2-ylcarbonyloxy)propylthio)hexan-1,1-diyl-bis(phosphonsäure) (bisPhn-6C-bisNorb) (B6)

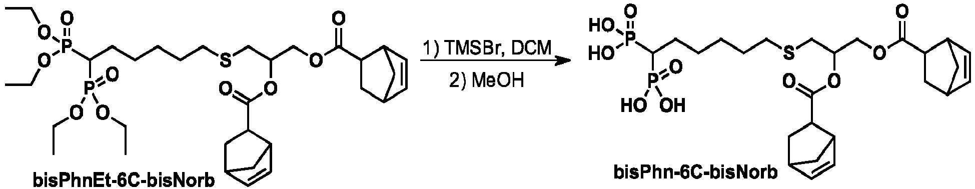

Die Synthese erfolgte analog zu Synthesebeispiel 3, Stufe 2, aus bisPhnEt-6C-bisNorb (1 Äqu., 10,6 g, 4 mmol) mit TMSBr (2,3 Äqu., 1,2 ml, 9,2 mmol), wobei 8,8 g (99 % d. Th.) der gewünschten Phosphonsäure bisPhn-6C-bisNorb als tiefrote viskose Flüssigkeit isoliert wurden.
¹H-NMR (400 MHz, CD₃OD) δ (ppm): 6,22-5,84 (m, 4H, 2x CH=CH), 5,22-4,94 (m, 1H, CHO), 4,51-4,23 (m, 1H, CH norb), 4,23-4,01 (m, 3H, CHCH₂O, CH norb), 3,22-3,15 (m, 1H, CH norb), 3,10-2,84 (m, 4H, CH norb, SCH₂CH), 2,75-2,60 (m, 2H, CH₂CH₂S), 2,60-2,49 (m, 2H, 2x CHC=O), 2,33 (tt, ³J_{HH} = 24,2, ²J_{HP} = 6,1 Hz, 1H, P₂CHCH₂), 2,28-2,13 (m, 2H, PCHCH₂), 1,98-1,85 (m, 4H, 2x CH₂ norb), 1,66-1,44 (m, 4H, 2x CH₂ Brücke norb), 1,44-1,34 (m, 4H, CH₂CH₂CH₂S), 1,28-1,20 (m, 2H, PCHCH₂CH₂); ¹³C-NMR (101 MHz, CD₃OD) δ (ppm): 176,00 (m, C=O), 174,36 (m, C=O), 138,42-138,14 (m, CH=CH), 138,03-137,75 (m, CH=CH), 135,76 (m, CH=CH), 132,64-132,26 (m, CH=CH), 71,24-70,21 (m, CHO), 63,93 (m, CHCH₂O), 49,71 (s, CH₂ Brücke), 46,72 (s, CH₂ norb), 46,49 (s, CH₂ norb), 45,82 (m, CHC=O), 43,80-43,02 (m, CH norb), 42,62 (m, CH norb), 41,75 (m, CH norb), 37,94 (t, ¹J_{CP} = 128,7 Hz, P₂CH), 32,90-32,13 (m, SCH₂CH), 30,44 (s, CH₂SCH₂), 29,63-28,96 (m, CH₂CH₂S), 28,53 (t, ³J_{CP} = 6,6 Hz, PCHCH₂CH₂), 28,30 (m, CH₂CH₂CH₂S), 25,23 (t, ²J_{CP} = 4,8 Hz, P₂CHCH₂); ³¹P-NMR (CD₃OD) δ (ppm): 22,73; HR-MS (MeOH, ESI, m/z): ber. 592,58, gef. 592,58.

### Beispiel 7 - Herstellung von 3-(3-(3,4-Dihydroxyphenyl)propylthio)propan-1,2-diyl-bis(norbornen-2-carboxylat) (Catechol-3C-bisNorb) (B7)

### Stufe 1: Herstellung von 5-Allyl-2,2-dimethyl-1,3-benzodioxol (Catechol^{prot}-3C-Enyl)

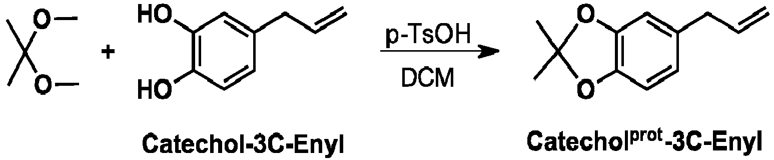

Ein Gemisch aus Catechol-3C-Enyl (1 Äqu., 9,8 ml, 57 mmol) aus Synthesebeispiel 11, 2,2-Dimethoxypropan (2 Äqu., 9,9 ml, 114 mmol) und p-Toluolsulfonsäure (p-TsOH) (0,06 Äqu., 0,2 g, 1,14 mmol) in 350 ml DCM wurde 20 h lang bei Raumtemperatur gerührt, wonach die Lösung mit gesättigter NaHCO₃-Lösung (3 x 100 ml) und die wässrigen Phasen mit Ethylacetat (3 x 80 ml) gewaschen wurden. Die vereinigten organischen Phasen wurden über MgSO₄ getrocknet und eingeengt, wobei 16,3 g (94 % d. Th.) der gewünschten Verbindung Catechol^{prot}-3C-Enyl als braune viskose Flüssigkeit erhalten wurden.
DC (PE) R_{f} = 0,27; ¹H-NMR (400 MHz, CDCl₃) δ (ppm): 6,87-6,52 (m, 3H, CH Ar), 6,07-5,80 (m, 1H, CH₂CH=CH₂), 5,15-4,97 (m, 2H, CH₂CH=CH₂), 3,37-3,19 (m, 2H, CH₂CH=CH₂), 1,67 (s, 6H, CH₃); ¹³C-NMR (101 MHz, CDCl₃) δ (ppm): 147,62 (s, CO), 145,77 (s, CO), 137,91 (s, CH=CH₂), 133,35 (s, C_{q}CH₂CH), 120,77 (s, CH Ar), 115,7 (s, CH=CH₂), 115,66 (s, C_{q}(CH₃)₂), 109,02 (s, CH Ar), 108,11 (s, CH Ar), 40,11 (s, C_{q}CH₂CH), 25,97 (s, C_{q}(CH₃)₂).

### Stufe 2: Herstellung von 3-(3-(2,2-Dimethyl-1,3-benzodioxol-5-yl)propylthio)propan-1,2-diol (Catechol^{prot}-3C-thioglyc)

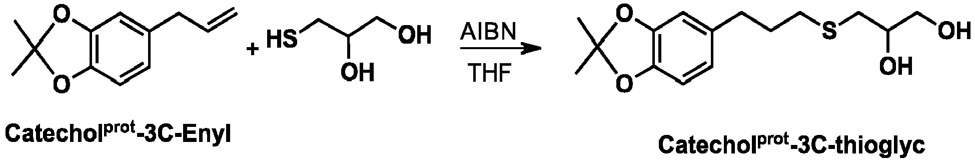

Catechol^{prot}-3C-Enyl (1 Äqu., 15 g, 79 mmol) und Thioglycerin (1,5 Äqu., 12,8 g, 118,5 mmol) wurden unter Argon in 250 ml trockenem THF gelöst. Das Gemisch wurde 45 min lang mit Argon entgast, wonach 0,2 g AIBN (0,02 Äqu., 0,92 mmol) zugesetzt wurden und das Reaktionsgemisch. 24 h lang bei 75 °C gerührt wurde, wobei der Reaktionsfortschritt mittels DC und ³¹P-NMR überwacht wurde. Anschließend wurde die Lösung unter reduziertem Druck eingeengt und das erhaltene Rohprodukt mittels Säulenchromatographie (EE:PE, 50:50) gereinigt wurde, wobei 7,26 g (31 % d. Th.) des gewünschten geschützten Produkts Catechol^{prot}-3C-thioglyc als gelbe viskose Flüssigkeit erhalten wurden.
DC (EE:PE 80:20) R_{f}= 0,54; ¹H-NMR (400 MHz, CDCl₃) δ (ppm): 6,71-6,48 (m, 3H, Ar-H), 3,87-3,63 (m, 2H, CH₂OH), 3,63-3,42 (m, 1H, CHOH), 2,70-2,40 (m, 6H, C_{q}CH₂CH₂CH₂SCH₂), 1,97-1,73 (m, 2H, C_{q}CH₂CH₂), 1,65 (s, 6H, C_{q}(CH₃)₂); ¹³C-NMR (101 MHz, CDCl₃) δ (ppm): 147,58 (s, CO), 145,74 (s, CO), 134,46 (s, C_{q}CH₂CH₂), 120,67 (s, CH Ar), 117,72 (s, C_{q}(CH₃)₂), 108,74 (s, CH Ar), 108,07 (s, CH Ar), 70,02 (s, CHOH), 65,51 (s, CH₂OH), 35,83 (SCH₂CH), 34,55 (s, C_{q,Ar}CH₂), 31,66 (s, C_{q,Ar}CH₂CH₂), 31,48 (s, CH₂SCH₂CH, 25,95 (s, C_{q}(CH₃)₂).

### Stufe 3: Herstellung von 3-(3-(2,2-Dimethyl-1,3-benzodioxol-5-yl)propylthio)propan-1,2-diyl-bis(norbornen-2-carboxylat) (Catechol^{prot}-3C-bisNorb)

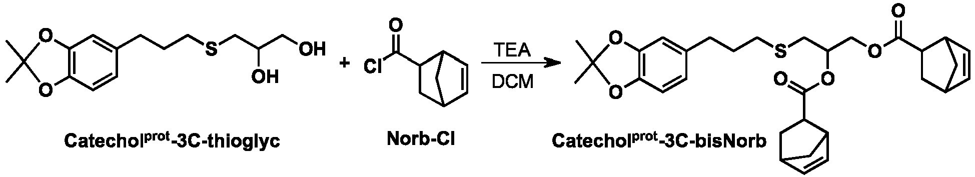

Die Synthese erfolgte analog zu Beispiel 3, Stufe 2, aus Catechol^{prot}-3C-thioglyc (1 Äqu., 6,0 g, 20 mmol) mit Norb-Cl (3 Äqu., 12,3 g, 60 mmol) und TEA (3,2 Äqu., 8,9 ml, 64 mmol), wobei mittels Säulenchromatographie (PE:EE 95:5) 4,5 g (42 % d. Th.) des gewünschten geschützten Produkts Catechol^{prot}-3C-bisNorb als braune viskose Flüssigkeit isoliert wurden.
DC (EE:PE, 1:9) R_{f} = 0,54; ¹H-NMR (400 MHz, CDCl₃) δ (ppm): 6,68-6,48 (m, 3H, 3x Ar-H), 6,23-5,86 (m, 4H, 2x CH=CH), 5,24-5,00 (m, 1H, CHO), 4,50-4,19 (m, 2H, CH₂O), 3,28-2,86 (m, 4H, (m, 4x CH norb), 2,77-2,47 (m, 6H, C_{q}CH₂CH₂CH₂SCH₂), 2,22 (m, 2H, 2x CHCO norb) 1,99-1,77 (m, 3H, CH₂ norb, C_{q}CH₂CH₂), 1,65 (s, 6H, C_{q}(CH₃)₂), 1,58-1,32 (m, 5H, CH₂ norb, 2x CH₂ Brücke); ¹³C-NMR (101 MHz, CDCl₃) δ (ppm): 175,81 (s, C=O), 175,48 (s, C=O), 147,54 (s, C-OC_{q}), 145,69 (s, C-OC_{q}), 138,21 (s, CH=CH), 135,74 (s, CH=CH), 134,52 (s, C_{q}CH₂CH₂), 120,63 (s, CH Ar), 117,59 (s, C_{q}(CH₃)₂), 108,72 (s, CH Ar), 108,01 (s, CH Ar), 70,59 (s, CHO), 63,92 (s, CH₂O), 49,69 (s, CH₂ Brücke), 46,45 (s, CH norb), 45,85 (s, CH norb), 43,51-43,10 (m, CHC=O norb), 42,82-42,42 (m, CH norb), 41,73 (s, CH norb), 34,48 (s, SCH₂CH), 32,38 (s, C_{q,Ar}CH₂), 32,01 (s, CH₂SCH₂CH), 30,43 (s, CH₂ norb), 29,31 (s, C_{q,Ar}CH₂CH₂), 25,91 (s, C_{q}(CH₃)₂).

### Stufe 4: Herstellung von 3-(3-(3,4-Dihydroxyphenyl)propylthio)propan-1,2-diyl-bis(norbornen-2-carboxylat) (Catechol-3C-bisNorb) (B7)

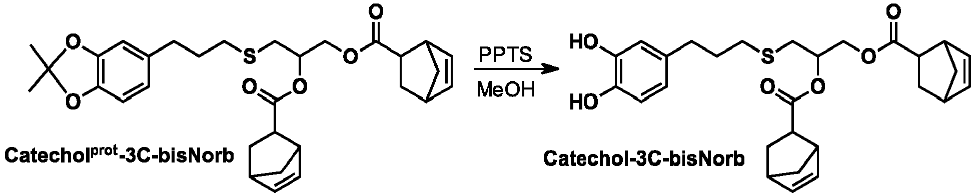

Ein Gemisch aus Catechol^{prot}-3C-bisNorb (1 Äqu., 2,9 g, 5,4 mmol) und Pyridinium-p-toluolsulfonat PPTS (0,1 Äqu., 0,14 g, 0,54 mmol) in 30 ml trockenem Methanol wurde über Nacht unter Rückfluss gerührt. Das Lösungsmittel wurde abgedampft und das Rohprodukt in 50 ml DCM gelöst. Die organische Phase wurde mit NaHCO₃ (3 x 75 ml) gewaschen und über MgSO₄ getrocknet, wobei 1,73 g (64 % d. Th.) des gewünschten Produkts Catechol-3C-bisNorb als braune viskose Flüssigkeit isoliert wurden.
DC (EE:PE, 1:1) R_{f} = 0,67; ¹H-NMR (400 MHz, CDCl₃) δ (ppm): 6,91-6,51 (m, 3H, 3x Ar-H), 6,27-5,87 (m, 4H, 2x CH=CH), 5,25-5,06 (m, 1H, CHO), 4,59-4,33 (m, 1H, CH₂O), 4,30-4,06 (m, 1H, CH₂O), 3,27-2,86 (m, 4H, 4x CH norb), 2,83-2,41 (m, 6H, C_{q}CH₂CH₂CH₂SCH₂), 2,40-2,16 (m, 2H, 2x CHCO norb), 2,03-1,73 (m, 3H, CH₂ norb, C_{q}CH₂CH₂), 1,57-1,33 (m, 5H, CH₂ norb, 2x CH₂ Brücke); ¹³C-NMR (101 MHz, CDCl₃) δ (ppm): 176,47 (s, C=O), 175,09 (s, C=O), 143,63 (s, C-OC_{q}), 142,35 (s, C-OC_{q}), 138,28 (s, CH=CH), 135,74 (s, CH=CH), 133,77 (s, C_{q}CH₂CH₂), 120,89 (s, CH Ar), 115,61 (s, CH Ar), 115,35 (s, CH Ar), 70,85 (s, CHO), 64,26 (s, CH₂O), 49,76 (s, CH₂ Brücke), 46,68 (s, CH norb), 46,51 (s, CH₂ Brücke), 45,96 (s, CH norb), 43,53 (s, CHC=O norb), 43,44 (s, CHC=O norb), 43,27 (s, CH norb), 42,68 (s, CH norb), 41,75 , 33,44 (SCH₂CH), 32,58 (s, C_{q,Ar}CH₂), 31,73 (s, CH₂SCH₂CH), 30,81 (s, 2x CH₂ norb), 29,38 (s, C_{q,Ar}CH₂CH₂); Elementaranalyse: ber. für C₂₈H₃₄O₆S: C-67,44, H-6,87, O-19,25, S-6,43, gef.: C-67,28, H-6,32, O-19,28, S-5,96.

### Beispiel 8 - Herstellung von Ethylendiamintetraessigsäure-bis(2-(3,4-dihydroxyphenyl)ethyl)amid-bis(2-(5-norbornen-2-yl)ethyl)amid (bisCatechol-EDTA-bisNorb) (B8)

### Stufe 1: Herstellung von Ethylendiamintetraessigsäuredianhydrid (EDTA-Dianhydrid)

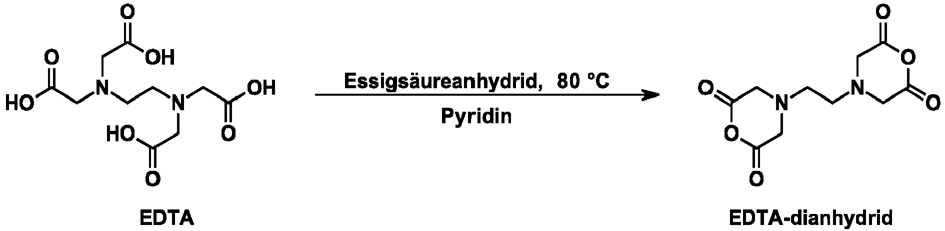

Die literaturbekannte Synthese erfolgte gemäß der Vorschrift in Peng et al., Org. Biomol. Chem. 15(30), 6441-6446 (2017), durch Reaktion von Ethylendiamintetraessigsäure (EDTA) mit Essigsäureanhydrid in Pyridin bei 80 °C.
DC (PE:EE, 2:1) R_{f}= 0,43; Fp.: 192-193 °C; ¹H-NMR (400 MHz, DMSO-d) δ (ppm): 3,71 (s, 8H, 4x (CH₂)₂N), 2,66 (s, 4H, NCH₂CH₂N); ¹³C-NMR (101 MHz, DMSO-d) δ (ppm): 172,20 (s, 4x C=O), 54,71 (s, 4x (CH₂)₂N), 51,37 (s, NCH₂CH₂N).

### Stufe 2: Herstellung von Ethylendiamintetraessigsäure-bis(2-(5-norbornen-2-yl)ethyl)-amid (EDTA-bisNorb)

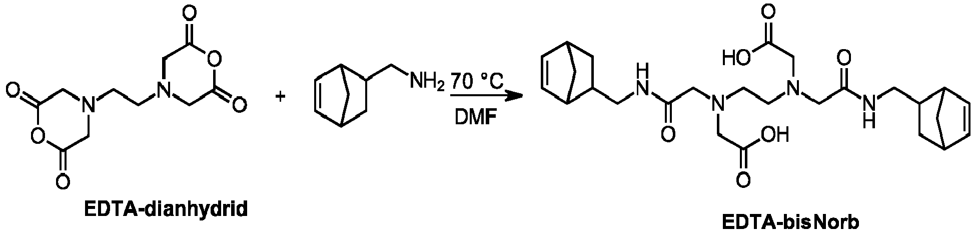

EDTA-Dianhydrid (1 Äqu., 4,6 g, 18 mmol) wurde in 46 ml DMF gelöst und 10 min lang gerührt, wonach 5-Norbornen-2-ylmethylamin (2,04 Äqu., 4,5 g, 36,7 mmol) langsam zugegeben und das Reaktionsgemisch 24 h lang bei 70 °C gerührt wurde. Das braune Gemisch wurde eingeengt, filtriert und mit 13 ml Chloroform versetzt. Bei Zusatz von 250 ml Diethylether (Et₂O) fiel ein weißer Feststoff aus, wonach der Überstand entfernt wurde. Der Niederschlag wurde zweimal mit 150 ml Et₂O trituriert, abfiltriert und im Vakuum getrocknet, was 8,9 g (98 % d. Th.) des gewünschten EDTA-Diamids EDTA-bisNorb als weißen Feststoff ergab.
Fp.: 148,1-153,2 °C (Zers.); ¹H-NMR (400 MHz, DMSO-d) δ (ppm): 8,06-7,91 (m, 2H, 2x NH), 6,21-5,91 (m, 4H, 2x CH=CH), 3,35 (s, 4H, 2x NCH₂COOH), 3,20 (s, 4H, 2x NHCOCH₂), 2,88-2,81 (m, 2H, 2x CH₂NH), 2,79-2,73 (m, 6H, 2x CH₂NH, 4x CH norb), 2,71 (s, 4H, NCH₂CH₂N), 2,25-2,15 (m, 2H, 2x CHCH₂NH norb), 1,81-1,70 (m, 2H, 2x CH₂ norb), 1,36-1,27 (m, 2H, 2x CH₂ norb), 1,25-1,02 (m, 4H, 2x CH₂ Brücke); ¹³C-NMR (101 MHz, DMSO-d) δ (ppm): 172,76 (s, 2x COOH), 170,07 (s, 2x NHC=O), 136,94 (s, 2x CH=CH), 132,57 (s, 2x CH=CH), 64,97 (s, 2x NCH₂COOH), 57,81 (s, 2x NCH₂C=0), 55,78 (s, NCH₂CH₂N), 52,50 (s, 2x CH₂ Brücke), 49,00 (s, 2x NHCH₂CH), 43,73 (s, 2x CH norb), 41,91 (s, 2x CH norb), 38,47 (s, 2x CH norb), 29,76 (s, 2x CH₂ norb); Elementaranalyse: ber. für C₂₆H₃₈N₄O₆: C-62,13, H-7,62, N-11,15, O-19,10; gef.: C-62,23, H-7,78, N-10,57, O-19,01.

### Stufe 3: Herstellung von Ethylendiamintetraessigsäure-bis(2-(3,4-dihydroxyphenyl)-ethyl)amid-bis(2-(5-norbornen-2-yl)ethyl)amid (bisCatechol-EDTA-bisNorb) (B8)

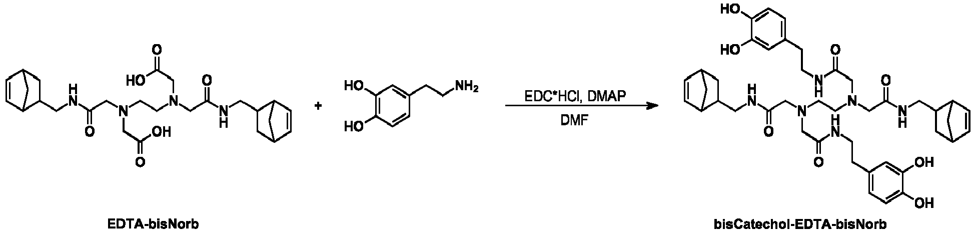

EDTA-bisNorb (1 Äqu., 7,5 g, 15 mmol), Dopaminhydrochlorid (5 Äqu., 11,5 g, 75 mmol) und DMAP (0,4 Äqu., 0,2 g, 6 mmol) wurden in 20 ml DMF gelöst und bei -10 °C unter Argon gerührt, wonach eine Suspension von EDC·HCl in 70 ml DMF zugetropft wurde, wobei die Temperatur unter 0 °C gehalten wurde. Nach vollständiger Zugabe wurde das Gemisch 48 h lang bei Raumtemperatur gerührt, wonach das Lösungsmittel abgedampft, das Produkt in entionisiertem Wasser ausgefällt und der Niederschlag im Vakuum getrocknet wurde, wobei 8,3 g (72 % d. Th.) des gewünschten EDTA-Tetraamids bisCatechol-EDTA-bisNorb als hellbrauner Feststoff erhalten wurden.
Fp.: 112,3-116,5 °C; ¹H-NMR (400 MHz, MeOD-d) δ (ppm): 7,90 (s, 2H, 2x NH norb), 6,79-6,75 (s, 2H, 2x NH dopa), 6,71-6,47 (m, 4H, 4x CH Ar), 6,48-6,34 (m, 2H, 2x CH Ar), 6,19-5,79 (m, 4H, 2x CH=CH), 3,52-3,44 (m, 2H, 2x CH₂NH), 3,40 (t, ³J_{HH} = 7,2 Hz, 4H, 2x NHCH₂CH₂ dopa), 3,35 (s, 8H, 4x NHCOCH₂N), 3,26-3,06 (m, 2H, 2x CH₂NH), 3,03-2,89 (m, 2H, CH₂NH), 2,88 (s, 4H, NCH₂CH₂N), 2,84-2,76 (m, 4H, 4x CH norb), 2,67 (t, ³J_{HH} = 7,2 Hz, 4H, 2x NHCH₂CH₂ dopa), 2,30-2,11 (m, 2H, 2x CHCH₂NH norb), 1,91-1,81 (m, 2H, 2x CH₂ norb), 1,49-1,27 (m, 2H, 2x CH₂ norb), 1,25-1,07 (m, 4H, 2x CH₂ Brücke); ¹³C-NMR (101 MHz, MeOD-d) δ (ppm): 146,28 (s, 2x C_{q}OH), 144,85 (s, 2x C_{q}OH), 138,54 (s, 2x CH=CH), 133,32 (s, 2x CH=CH), 131,84 (s, 2x C_{q}CH₂CH₂NH), 121,06 (s, CH Ar), 116,98 (s, CH Ar), 116,47 (s, CH Ar), 58,79 (s, NCH₂C=O), 57,54 (s, NCH₂C=0), 56,86 (s, NCH₂C=O), 56,39 (s, NCH₂C=0), 54,08 (s, NCH₂CH₂N), 49,66 (s, 2x CH₂ Brücke), 45,46 (s, CH norb), 44,75-44,32 (m, 2x NHCH₂CH), 43,70 (s, CH norb), 42,37-41,43 (m, C_{q}CH₂CH₂NH), 40,06-39,70 (m, CH norb), 36,03-35,45 (m, C_{q}CH₂CH₂NH), 31,55-30,87 (m, 2x CH₂ norb); Elementaranalyse: ber. für C₄₂H₅₆N₆O_{8:} C-65,26, H-7,30, N-10,87, 0-16,56; gef.: C-65,80, H-7,33, N-10,85, 0-16,29.

### Beispiel 9 - Herstellung von Diethylentriaminpentaessigsäure-bis(2-(5-norbornen-2-yl)ethyl)amid-tris(2-(3,4-dihydroxyphenyl)ethyl)amid (triCatechol-DTPA-bisNorb) (B9)

### Stufe 1: Herstellung von Ethylendiamintetraessigsäuredianhydrid (DTPA-Dianhydrid)

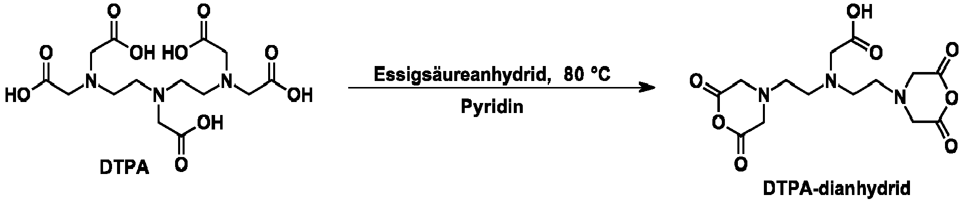

Die literaturbekannte Synthese erfolgte analog zu Beispiel 8, Stufe 1, nach der Vorschrift in Peng et al., Org. Biomol. Chem. 15(30), 6441-6446 (2017), durch Reaktion von Diethylentriaminpentaessigsäure (DTPA) mit Essigsäureanhydrid.
Fp.: 182,1-184,2 °C; ¹H-NMR (400 MHz, DMSO-d) δ (ppm): 3,71 (s, 8H, 4x (CH₂)₂N), 3,29 (s, 2H, NCH₂COOH), 2,75 (t, ³J_{HH} = 6,2 Hz, 4H, NCH₂CH₂N), 2,59 (t, ³J_{HH} = 5,8 Hz, 4H, NCH₂CH₂N); ¹³C-NMR (101 MHz, DMSO-d) δ (ppm): 171,99 (s, CH₂COOH), 165,81 (s, 4x C=O), 54,56 (s, CH₂COOH), 52,61 (s, 4x NCH₂COO), 51,79 (s, 2x NCH₂CH₂N), 50,74 (s, 2x NCH₂CH₂N).

### Stufe 2: Herstellung von Diethylentriaminpentaessigsäure-bis(2-(5-norbornen-2-yl)-ethyl)amid (DTPA-bisNorb)

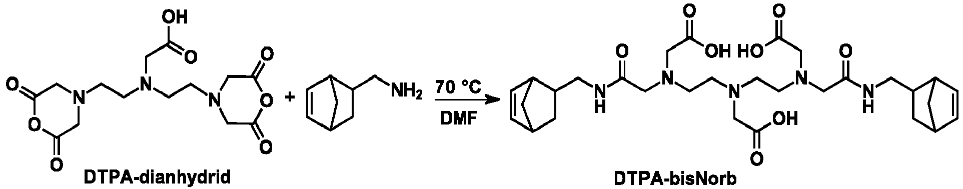

Die Synthese erfolgte analog zu Beispiel 8, Stufe 2, aus DTPA-Dianhydrid (1 Äqu., 23,4 g, 18 mmol) und 5-Norbornen-2-ylmethylamin (2,04 Äqu., 4,5 g, 36,7 mmol) in 70 ml DMF. Das Rohprodukt wurde mit 150 ml DCM und 200 ml Et₂O gewaschen und im Vakuum getrocknet, wobei 9,1 g (84% d. Th.) des gewünschten Diamids DTPA-bisNorb als brauner Feststoff isoliert wurden.
Fp.: 83,5-87,6 °C; ¹H-NMR (400 MHz, DMSO-d) δ (ppm): 8,05 (t, ³J_{HH} = 5,8 Hz, 2H, 2x NH), 6,18-5,93 (m, 4H, 2x CH=CH), 3,44 (s, 2H, NCH₂COOH), 3,37 (s, 4H, 2x CHCH₂NH), 3,23 (s, 4H, 2x COCH₂N), 3,20-3,04 (m, 4H, 2x NHCOCH₂), 2,99-2,94 (m, 4H, 2x NCH₂CH₂N), 2,89-2,81 (m, 4H, 2x NCH₂CH₂N), 2,78 (s, 2H, 2x CH norb), 2,75-2,67 (m, 2H, 2x CH norb), 2,26-2,17 (m, 2H, 2x CHCH₂NH norb), 1,80-1,73 (m, 2H, 2x CH₂ norb), 1,34-1,28 (m, 2H, 2x CH₂ norb), 1,24-1,12 (m, 4H, 2x CH₂ Brücke); ¹³C-NMR (101 MHz, DMSO-d) δ (ppm): 172,75 (s, 2x CH₂COOH), 171,99 (s, CH₂COOH), 169,82 (s, 2x NHCOCH₂N), 136,83 (s, 2x CH=CH), 132,62 (s, 2x CH=CH), 57,58 (s, 2x NCH₂COOH), 55,30 (s, NHCOCH₂N), 54,92 (s, NCH₂COOH), 52,27 (s, 2x NCH₂CH₂N), 51,09 (s, 2x NCH₂CH₂N), 48,95 (s, 2x CH₂ Brücke), 43,69 (s, 2x CH norb), 42,44 (s, 2x NHCH₂CH), 41,88 (s, 2x CH norb), 38,39 (s, 2x CH norb), 29,74 (s, 2x CH₂ norb); Elementaranalyse: ber. für C₃₀H₄₅N₅O₈: C-59,68, H-7,51, N-11,60, O-21,20; gef.: C-59,58, H-7,46, N-11,69, 0-21,35.

### Stufe 3: Herstellung von Diethylentriaminpentaessigsäure-bis(2-(5-norbornen-2-yl)-ethyl)amid-tris(2-(3,4-dihydroxyphenyl)ethyl)amid (triCatechol-DTPA-bisNorb) (B9)

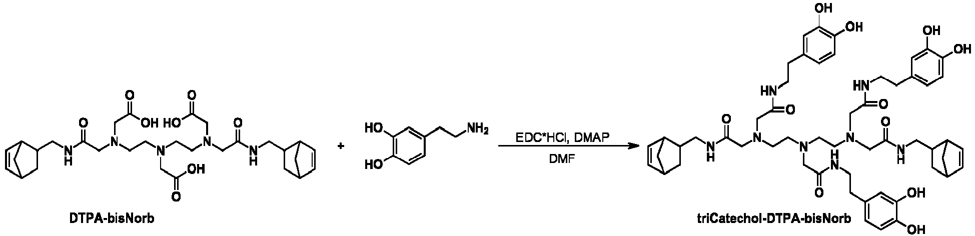

Die Synthese erfolgte analog zu Beispiel 8, Stufe 3, aus DTPA-bisNorb (1 Äqu., 6,9 g, 11,5 mmol), Dopaminhydrochlorid (4 Äqu., 8,7 g, 46 mmol) und DMAP (0,6 Äqu., 0,8 g, 6,9 mmol) in 80 ml DMF mit einer Suspension von EDC·HCl (3,5 Äqu., 7,7 g, 40,25 mmol) in 220 ml DMF. Das Rohprodukt wurde in Ethanol gelöst und in entionisiertem Wasser ausgefällt, wobei 2,9 g (25 % d. Th.) einer braunen, sehr viskosen Flüssigkeit erhalten wurden.
¹H-NMR (400 MHz, MeOD-d) δ (ppm): 7,88 (s, 2H, 2x NH norb), 6,89-6,26 (m, 9H, 9x CH Ar), 6,16-5,74 (m, 4H, 2x CH=CH), 3,59 (m, 2H, 2x CH₂NH), 3,30 (t, ³J_{HH} = 7,2 Hz, 6H, 3x NHCH₂CH₂ dopa), 3,16-2,93 (m, 12H, 5x NHCOCH₂N), 3,04-2,95 (m, 2H, 2x CH₂NH), 2,72-2,66 (m, 8H, 2x NCH₂CH₂N), 2,57 (t, , ³J_{HH} = 7,2 Hz, 6H, 3x NHCH₂CH₂ dopa), 2,25-2,12 (m, 2H, 2x CHCH₂NH norb), 1,87-1,67 (m, 2H, 2x CH₂ norb), 1,38-1,25 (m, 2H, 2x CH₂ norb), 1,24-1,01 (m, 4H, 2x CH₂ Brücke); ¹³C-NMR (101 MHz, MeOD-d) δ (ppm): 173,22 (s, 3x NHCOCH₂N dopa), 172,90 (s, 2x NHCOCH₂N norb), 146,27 (s, 3x C_{q}OH), 144,82 (s, 3x C_{q}OH), 138,50 (s, 2x CH=CH), 133,35 (s, 2x CH=CH), 131,86 (s, 2x C_{q}CH₂CH₂NH), 121,12 (s, 3x CH Ar), 117,00 (s, 3x CH Ar), 116,62 (s, 3x CH Ar), 59,78 (s, 3x NCH₂C=0), 58,33 (s, NCH₂CH₂N), 56,46 (s, NCH₂CH₂N), 50,37 (s, 2x CH₂ Brücke), 45,49 (s, CH norb), 44,41 (m, 3x NHCH₂CH), 43,69 (s, CH norb), 42,41-41,73 (m, C_{q}CH₂CH₂NH), 39,97 (m, CH norb), 37,30 (m, CH norb), 36,01-35,28 (m, 3x C_{q}CH₂CH₂NH), 31,14 (m, 2x CH₂ norb); Elementaranalyse: ber. für C₅₄H₇₂N₈O₁₁: C-64,27, H-7,19, N-11,10, O-17,44; gef.: C-64,27, H-7,86, N-11,64, O-18,27.

### Beispiele 10 bis 36, Vergleichsbeispiele 1 bis 30 - Herstellung und Testung von polymerisierbaren Zusammensetzungen

### Vorbereitung der zu beschichtenden Oberflächen

Für die Messung der Scherhaftfestigkeit (SBS) von polymerisierbaren Zusammensetzungen der vorliegenden Erfindung wurden drei verschiedene Substrate vorbereitet.

### 1) Hydroxylapatit

Dieses Mineral der chemischen Formel Ca₅[OH|(PO₄)₃] stellt den Hauptbestandteil der anorganischen Hartsubstanz in Knochen dar und wird daher in der Regel als Modellsubstanz für Testreihen mit Knochenklebern eingesetzt. Für die Zwecke hierin wurde Hydroxylapatit (HAP-) Pulver von der Lithoz GmbH (Wien, Österreich) bezogen und mit 400 kN 1 min lang kalt-isostatisch zu Pellets gepresst. Die so erhaltenen Pellets wurden anschließend in einem Laborofen der Carbolite Gero GmbH (Neuhausen, Deutschland) nach dem folgenden Temperaturprogramm gesintert: mit 2 K/min auf 500 °C, 30 min Wartezeit, mit 2 K/min auf 1250 °C, 2 h Wartezeit, anschließend Abkühlung auf Raumtemperatur mit 3 K/min.

### 2) Titandioxid

Titandioxid- (TiO₂-) Pulver (Ø 200 nm) von Cinkarna (Celje, Slowenien) wurde unter Verwendung eines Hammer Lab35 Metal 3D Printers der Incus GmbH (Wien, Österreich) zu TiO₂-Pellets laser-gesintert.

### 3) Rinderknochen

Zur Durchführung von Ex-vivo-Messungen an Knochenmaterial wurden Teile von Rinderhufen, die das distale Ende der zweiten Phalanx umgeben, mit einer Bandsäge in 1 cm dicke Scheiben gesägt. Die so erhaltenen Rinderzehenknochen-Scheiben wurden anschließend in kleinere Stücke geschnitten und in PBS-Puffer eingelegt bei -80 °C tiefgefroren bis zur Verwendung gelagert.

Alle drei Substrate wurden vor Beschichtung mit den polymerisierbaren Zusammensetzungen in Epoxidharz eingebettet und ihre Oberflächen mit SiC-Nassschleifpapier der Körnung 400 glattpoliert.

### Herstellung der polymerisierbaren Zusammensetzungen

Durch einfaches Vermischen eines der in den obigen Synthesebeispielen und Beispielen hergestellten haftvermittelnden Primer mit aus der Literatur zur Verwendung in Knochenklebern bekannten multifunktionellen Monomeren, multifunktionellen Thiolen und einem radikalischen Standard-Initiator wurden sowohl Ein- als auch Zwei-Komponenten-Zusammensetzungen hergestellt.

### Beispiele 10 bis 24, Vergleichsbeispiele 1 bis 18 - Zwei-Komponenten-Zusammensetzungen

Zur Untersuchung der Verträglichkeit der erfindungsgemäßen neuen Primer mit den übrigen Komponenten solcher Zusammensetzungen wurden zunächst auf Basis von Trimethylolpropan-tris(3-mercaptopropionat) (TMPMP) als Thiol und von Bis-(4-methoxybenzoyl)diethylgermanium, einem radikalischen Photoinitiator, der unter dem Markennamen Ivocerin^{®} von Ivoclar Vivadent (Schaan, Liechtenstein) im Handel erhältlich ist, mehrere Zwei-Komponenten-Zusammensetzungen hergestellt.

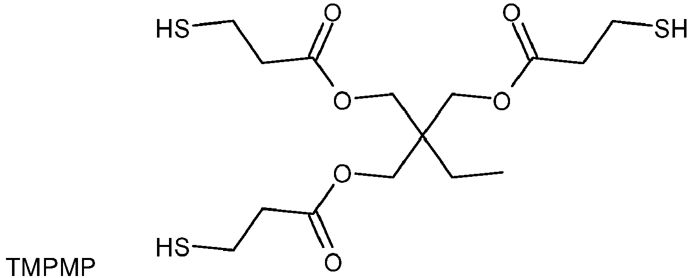

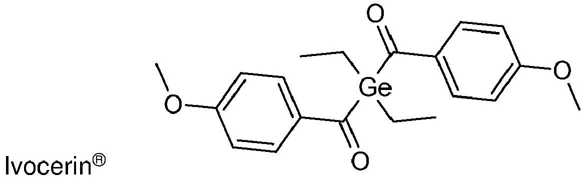

Als Monomere wurden das trifunktionelle, relativ starre Triallylisocyanurat 1,3,3-Tri-allyl-1,3,5-triazin-2,4,6-trion (TATATO) und der difunktionelle, biologisch abbaubare Vinylester O,O'-(Hexahydrofuro[3,2-b]furan-3,6-diyl)divinyladipat (Glucitoldivinyladipat, GDVA) untersucht.

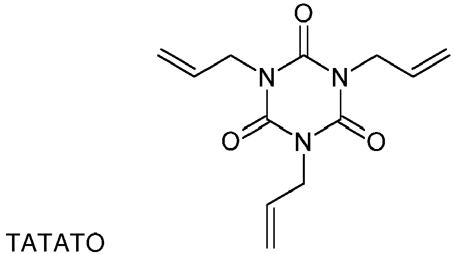

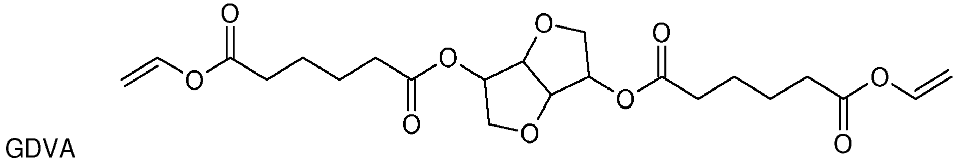

Gemische aus Monomer, Thiol, Initiator und Primer wurden als erste und solche ohne Primer als zweite Komponente hergestellt, die hierin als "Primerformulierung", PF, bzw. "Matrixformulierung", MF, bezeichnet werden. Als Primer wurde zunächst der herkömmliche Primer aus Synthesebeispiel 1, 2,2-Bis(allyloxymethyl)propanamido)-methyl)phosphonsäure (BAPA^{Phn}) (S1), eingesetzt.

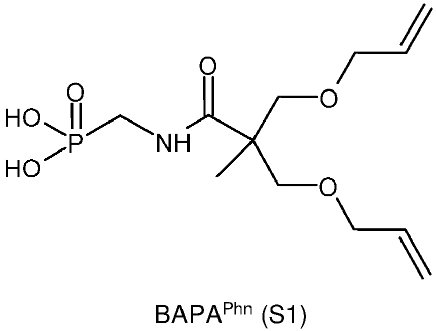

In der nachstehenden Tabelle 1 sind die Mengen der einzelnen Verbindungen in Gew.-%, bezogen auf das gesamte Gemisch, angegeben, wobei jede der Formulierungen zusätzlich noch 0,02 Gew.-% Pyrogallol (1,2,3-Trihydroxybenzol) als Thiol-En-Stabilisator enthielt.

**Tabelle 1 - erste (PF) und zweite (MF) Komponente von Zwei-Komponenten-Zusammensetzungen nach dem Stand der Technik unter Variation des Monomers**

| Formulierung | BAPA^{Phn} [Gew.-%] | Monomer [Gew.-%] | | TMPMP [Gew.-%] | Ivocerin [Gew.-%] |
|---|---|---|---|---|---|
| PF1 | 15 | 12,5 | TATATO | 71 | 1,5 |
| PF2 | 15 | 12,5 | GDVA | 71 | 1,5 |
| MF1 | - | 32 | TATATO | 67 | 1 |
| MF2 | - | 32 | GDVA | 67 | 1 |

Aus diesen vier Formulierungen wurden drei Kombinationen als Zwei-Komponenten-Zusammensetzungen zusammengemischt, womit in der Folge Substrate beschichtet, die Beschichtung gehärtet und auf ihre Scherhaftfestigkeit untersucht wurde:

| | |
|---|---|
| Vergleichsbeispiel 1 (V1): | PF1 + MF1 |
| Vergleichsbeispiel 2 (V2): | PF2 + MF1 |
| Vergleichsbeispiel 3 (V3): | PF1 + MF2 |

Zu diesem Zweck wurden wie oben beschrieben hergestellte und polierte Hydroxylapatit- bzw. Titandioxid-Scheiben zunächst mit der jeweiligen Primer-Formulierung PF beschichtet, indem das Gemisch auf die Oberfläche aufgebracht und mit einer Mikrobürste 20 s lang eingerieben, 10 s lang an der Luft antrocknen gelassen und danach mit einer LED-Polymerisationslampe Bluephase C8 von Ivoclar Vivadent (Schaan, Liechtenstein) mit einer Emission im Wellenlängenbereich von 385-515 nm und einer Leistung von 720 mW cm⁻² 10 s lang bestrahlt. Auf die so vorgehärtete erste Komponente wurde die jeweilige Matrixformulierung MF (unter Zuhilfenahme einer die Ränder der Oberflächen begrenzenden Form, um ein Abfließen der Formulierung zu verhindern) als zweite Komponente aufgebracht und durch Bestrahlung mit der LED-Lampe 20 s lang gehärtet.

Die Proben wurden anschließend in modifizierte simulierte Körperflüssigkeit ("modified simulated body fluid", m-SBF) eingelegt und bei 37 °C 24 h lang gelagert, wonach sie unter Verwendung des VTSYIQI SF-30 Digitalen Kraftmessgeräts mit einem V-förmigen Prüfstab auf die Scherhaftfestigkeit (SBS) der gehärteten Beschichtung geprüft wurden.

Wie aus den in Fig. 1A grafisch dargestellten Ergebnissen klar zu erkennen ist, lieferte von den drei Proben nur die Kombination aus PF1 und MF1 von Vergleichsbeispiel 1, bei der in beiden Komponenten TATATO als Monomer enthalten war, gute Ergebnisse bei den Scherhaftfestigkeitstests. Jene der Vergleichsbeispiele 2 und 3, in denen jeweils in einer der beiden Formulierungen GDVA als Monomer zum Einsatz kam, konnte hingegen keine auch nur annähernd zufriedenstellende Festigkeit erzielt werden. Aus diesem Grund wurde auf Tests mit einer Kombination beider GDVA-hältiger Formulierungen, PF2+MF2, verzichtet.

In der Folge wurde von den Erfindern eine erste polymerisierbare Zusammensetzung gemäß dem ersten Aspekt der Erfindung hergestellt, wobei zunächst in Analogie zum zuvor untersuchten Standard-Primer BAPA^{Phn} einer der neuen Primer mit einer ähnlichen Spacer-Länge und ebenfalls zwei polymerisierbaren Gruppen PG und einem Adhäsionsmotiv AM getestet wurde, nämlich 6-(2,3-Bis(5-norbornen-2-ylcarbonyloxy)-propylthio)hexylphosphonsäure (Phn-6C-bisNorb) (B5) aus Beispiel 5 mit zwei Norbornenyl- und einer Phosphonsäure-Gruppierung untersucht:

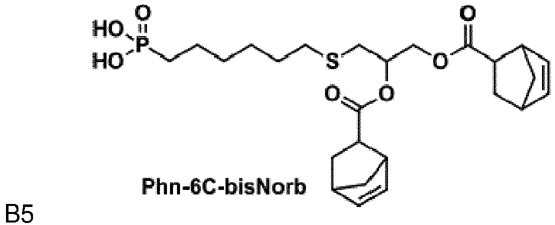

Die Herstellung von Formulierungen mit den beiden Monomeren TATATO und GDVA und Ivocerin als Initiator und deren Kombination zu Zwei-Komponenten-Zusammensetzungen erfolgte ebenfalls analog zu den obigen Vergleichsbeispielen 1 bis 3, wie nachstehend angegeben.

**Tabelle 2 - erste (PF) und zweite (MF) Komponente von Zwei-Komponenten-Zusammensetzungen gemäß vorliegender Erfindung unter Variation des Monomers**

| Formulierung | Phn-6C-bisNorb (B5) [Gew.-%] | Monomer [Gew.-%] | | TMPMP [Gew.-%] | Ivocerin [Gew.-%] |
|---|---|---|---|---|---|
| PF1 | 15 | 12,5 | TATATO | 71 | 1,5 |
| PF2 | 15 | 12,5 | GDVA | 71 | 1,5 |
| MF1 | - | 32 | TATATO | 67 | 1 |
| MF2 | - | 32 | GDVA | 67 | 1 |

Daraus wurden erneut drei Kombinationen als Zwei-Komponenten-Zusammensetzungen zusammengemischt:

| | |
|---|---|
| Beispiel 10 (B10): | PF1 + MF1 |
| Beispiel 11 (B11): | PF2 + MF1 |
| Beispiel 12 (B12): | PF1 + MF2 |

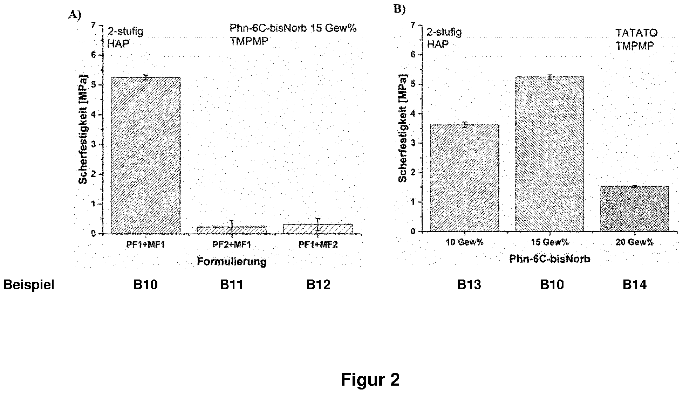

Diese Zusammensetzungen wurden auf identische Weise wie oben beschrieben nacheinander auf polierte Hydroxylapatit-Scheiben aufgebracht, (vor)gehärtet und die Scherhaftfestigkeit der Beschichtung getestet. Die Ergebnisse sind in Fig. 2A dargestellt.

Ein Vergleich zwischen den Ergebnissen der Vergleichsbeispiele 1 bis 3 in Fig. 1A und der Beispiele 10 bis 12 in Fig. 2A zeigt klar, dass zwar die beiden GDVA-hältigen Zusammensetzungen der Beispiele 11 und 12 nur unwesentlich bessere Werte für die Scherhaftfestigkeit ergeben hatten als jene der Vergleichsbeispiele 2 und 3, dafür aber mit der Zwei-Komponenten-Zusammensetzung von Beispiel 1 ohne GDVA mit Phn-6C-bisNorb als Primer mehr als die dreifache Scherhaftfestigkeit von jener mit BAPA^{Phn} in Vergleichsbeispiel 1 erzielt wurde als, nämlich 5,25 ± 0,08 MPa gemäß vorliegender Erfindung im Vergleich zu nur 1,64 ± 0,15 MPa nach dem Stand der Technik.

### Variation der Primer-Konzentration

In der Folge wurden sowohl mit dem herkömmlichen Primer BAPA^{Phn} als auch mit Phn-6C-bisNorb (B5) gemäß vorliegender Erfindung Versuche unter Variation der Primer-Konzentration in den Formulierungen unternommen. Dazu wurde in den obigen Primerformulierungen PF1, die jeweils 15 Gew.-% Primer enthielten, dessen Menge um jeweils 5 Gew.-% gesenkt bzw. erhöht und die Mengen an Monomer (TATATO) und Thiol (TMPMP) entsprechend angepasst. Diese je nach der Menge an Primer als PF10, PF15 und PF20 bezeichneten Primerformulierungen wurden jeweils mit der TATATO-hältigen Matrixformulierung MF1 zu Zwei-Komponenten-Zusammensetzungen kombiniert. Die genauen Zusammensetzungen der einzelnen Formulierungen sind in der umseitigen Tabelle 3 angegeben.

**Tabelle 3 - erste (PF) und zweite (MF) Komponente von Zwei-Komponenten-Zusammensetzungen nach dem Stand der Technik und gemäß vorliegender Erfindung unter Variation der Menge an Primer**

| **Beispiel** | **Formulierung** | **BAPA^{Phn} [Gew.-%]** | **Phn-6C-bisNorb (B5) [Gew.-%]** | **TATATO [Gew.-%]** | **TMPMP [Gew.-%]** | **Ivocerin [Gew.-%]** |
|---|---|---|---|---|---|---|
| Vergleichsbeispiel 1 (V1) | PF₁₅ | 15 | - | 12,5 | 71,0 | 1,5 |
| Vergleichsbeispiel 4 (V4) | PF₁₀ | 10 | - | 15,0 | 73,5 | 1,5 |
| Vergleichsbeispiel 5 (V5) | PF₂₀ | 20 | - | 10,0 | 68,5 | 1,5 |
| Beispiel 10 (B10) | PF₁₅ | - | 15 | 12,5 | 71,0 | 1,5 |
| Beispiel 13 (B13) | PF₁₀ | - | 10 | 15,0 | 73,5 | 1,5 |
| Beispiel 14 (B14) | PF₂₀ | - | 20 | 10,0 | 68,5 | 1,5 |
| | | | | | | |
| alle obigen Beispiele | MF1 | - | - | 32,0 | 67,0 | 1,0 |

Die vier neuen Zwei-Komponenten-Zusammensetzungen der Vergleichsbeispiele 4 und 5 sowie der Beispiele 13 und 14 wurden auf identische Weise wie zuvor auf Hydroxylapatit-Oberflächen aufgebracht, (vor)gehärtet und auf die Scherhaftfestigkeit der Beschichtung untersucht. Die dabei erzielten Ergebnisse sind zusammen mit den beiden zuvor für die jeweils 15 Gew.-% Primer enthaltenden Zusammensetzungen von Vergleichsbeispiel 1 und Beispiel 10 erhaltenen in Fig. 1B und 2B dargestellt.

Ein Vergleich dieser beiden Grafiken zeigt erneut ähnliche Tendenzen sowohl für die Vergleichsbeispiele als auch die Beispiele gemäß vorliegender Erfindung: In beiden Fällen ergaben die neuen Zusammensetzungen mit weniger (10 Gew.-%) oder mehr (20 Gew.-%) Primer in den Primerformulierungen eine geringere Scherfestigkeit als zuvor mit jeweils 15 Gew.-% des jeweiligen Primers, wobei mit 10 Gew.-% jeweils noch bessere Werte als mit 20 Gew.-% Primer erzielt wurden. Ähnlich wie zuvor im erfindungsgemäßen Beispiel 10 mit 15 Gew.-% Phn-6C-bisNorb in der Primerformulierung beträgt auch für Beispiel 13 der Erfindung mit 10 Gew.-% Primer die Scherhaftfestigkeit mehr als das Dreifache jener der entsprechenden Zusammensetzung nach dem Stand der Technik mit 10 bzw. 15 Gew.-% BAPA^{Phn} (3,62 ± 0,09 MPa zu 0,98 ± 0,26 MPa). Und auch für Beispiel 14 beträgt der Wert mehr als das Doppelte jenes von Vergleichsbeispiel 5, die jeweils 20 Gew.-% Primer enthielten (1,64 ± 0,15 MPa zu 0,75 ± 0.21 MPa).

Zusammenfassend kann daher einerseits festgestellt werden, dass ein relativ starres, trifunktionelles und daher stärker vernetzend wirkendes Monomer wie TATATO gegenüber einem difunktionellen und deutlich flexibleren Monomer wie GDVA klar zu bevorzugen ist. Und andererseits, dass rund 15 Gew.-% Primer in einer Primerformulierung als erste Komponente einer Zwei-Komponenten-Zusammensetzung am besten geeignet zu sein scheinen, um hohe Scherhaftfestigkeiten der gehärteten Zusammensetzungen zu erzielen.

Allerdings hat auch ein Gehalt von 20 und vor allem von nur 10 Gew.-% Primer eine gute oder zumindest akzeptable Scherhaftfestigkeit ergeben, so dass generell ein Anteil von 5 bis 25 Gew.-% der erfindungsgemäßen Primer in der Primerformulierung von Zwei-Komponenten-Zusammensetzungen zu bevorzugen zu sein scheint, wovon der Anteil noch bevorzugter etwa 7 bis etwa 17 Gew.-%, insbesondere etwa 12 bis 15 Gew.-%, beträgt.

### Variation des Thiols

Unter erneuter Verwendung von 15 Gew.-% Primer in der Primerformulierung einer Zwei-Komponenten-Zusammensetzung wurde in der Folge das in beiden Komponenten enthaltene Thiol variiert.

Zu diesem Zweck wurde jeweils in beiden der zu kombinierenden Formulierungen PF und MF der Zusammensetzungen von Vergleichsbeispiel 1 und Beispiel 10 das Thiol TMPMP durch gleiche Gewichtsmengen an entweder Tris[2-(3-mercaptopropionyl-oxy)ethyl]isocyanurat (TEMPIC) oder Dipentaerythrit-hexakis(3-mercaptopropionat) (DiPETMP) ersetzt.

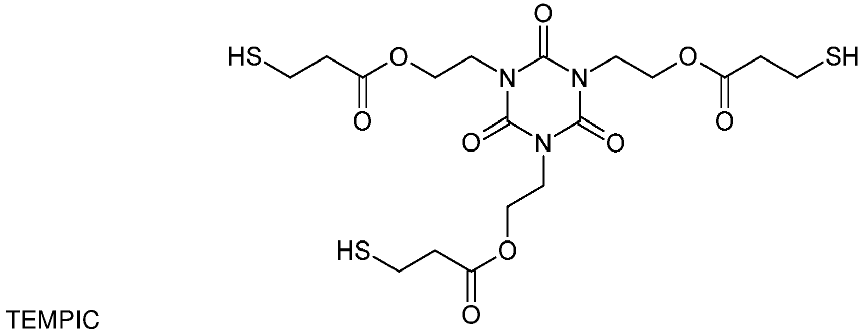

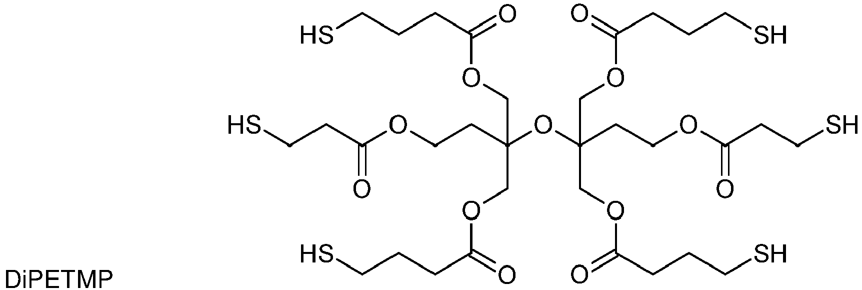

Die genauen Zusammensetzungen der einzelnen Formulierungen sind in der umseitigen Tabelle 4 angegeben.

**Tabelle 4 - erste (PF) und zweite (MF) Komponente von Zwei-Komponenten-Zusammensetzungen nach dem Stand der Technik und gemäß vorliegender Erfindung unter Variation des Thiols**

| **Beispiel** | **Formulierung** | **BAPA^{Phn} [Gew.-%]** | **Phn-6C-bisNorb (B5) [Gew.-%]** | **TATATO [Gew.-%]** | **TEMPMP [Gew.-%]** | **TEMPIC [Gew.-%]** | **DiPETMP [Gew.-%]** | **Ivocerin [Gew.-%]** |
|---|---|---|---|---|---|---|---|---|
| Vergleichsbeispiel 1 (V1) | PF1 | 15 | - | 12,5 | 71,0 | - | - | 1,5 |
| Vergleichsbeispiel 6 (V6) | PF2 | 15 | - | 12,5 | | 71,0 | - | 1,5 |
| Vergleichsbeispiel 7 (V7) | PF3 | 15 | - | 12,5 | | - | 71,0 | 1,5 |
| Beispiel 10 (B10) | PF1 | - | 15 | 12,5 | 71,0 | - | - | 1,5 |
| Beispiel 15 (B15) | PF2 | - | 15 | 12,5 | | 71,0 | - | 1,5 |
| Beispiel 16 (B16) | PF3 | - | 15 | 12,5 | | - | 71,0 | 1,5 |
| | | | | | | | | |
| V1 und B10 | MF1 | - | - | 32,0 | 67,0 | - | - | 1,0 |
| V6 und B15 | MF2 | - | - | 32,0 | - | 67,0 | - | 1,0 |
| V7 und B16 | MF3 | - | - | 32,0 | - | - | 67,0 | 1,0 |

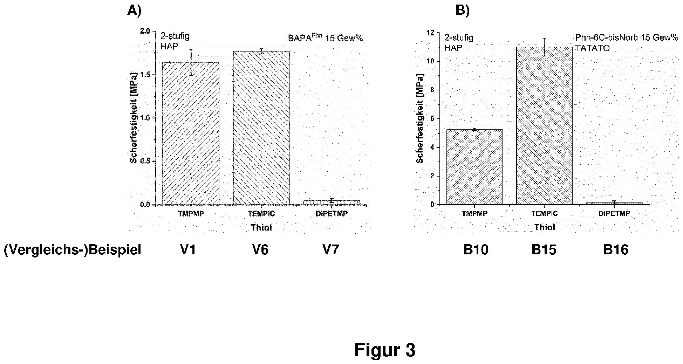

Die vier neuen Zwei-Komponenten-Zusammensetzungen der Vergleichsbeispiele 6 und 7 sowie der Beispiele 15 und 16 wurden auf identische Weise wie zuvor auf Hydroxylapatit-Oberflächen aufgebracht, (vor)gehärtet und auf die Scherhaftfestigkeit der Beschichtung untersucht. Die dabei erzielten Ergebnisse sind zusammen mit den zuvor für die TMPMP enthaltenden Zusammensetzungen von Vergleichsbeispiel 1 und Beispiel 1 erhaltenen in Fig. 3A und 3B dargestellt.

In Fig. 3 fällt sofort auf, dass unter Verwendung von DiPETMP als Thiol sowohl mit der Zusammensetzung nach dem Stand der Technik als auch mit jener gemäß vorliegender Erfindung nur eine sehr geringe Scherhaftfestigkeit erzielt werden konnte. Ohne sich darauf festlegen zu wollen, nehmen die Erfinder an, dass dies einerseits auf den mit dem sechswertigen Thiol erzielten (zu) hohen Vernetzungsgrad zurückzuführen ist, aufgrund dessen nur unzureichende Mengen der als Adhäsionsmotive der Primer dienenden Phosphonsäure-Gruppen mit der Substratoberfläche in Kontakt kommen und bleiben können. Es ist daher anzunehmen, dass bei Einsatz von stark vernetzenden Thiolen wie DiPETMP die Verwendung eines Primers mit größerer Spacer-Kettenlänge und/oder einer größeren Anzahl an Adhäsionsmotiven AM bessere Ergebnisse liefern wird.

Während allerdings bei den Versuchen mit BAPA^{Phn} als Primer durch den Austausch des Thiols von TMPMP gegen TEMPIC nur eine geringfügige Verbesserung der Werte von rund 5 % erzielt wurde, führte dieser Wechsel unter Verwendung des erfindungsgemäßen Primers Phn-6C-bisNorb (B5) in Beispiel 15 zu einem im Vergleich zu Beispiel 1 mehr als doppelt so hohen Scherhaftfestigkeitswert (11,00 ± 0,64 MPa zu 5,25 ± 0,08 MPa). Dies wird von den Erfindern, ohne sich darauf festlegen zu wollen, ähnlich wie beim Vergleich zwischen TATATO und GDVA als Monomer, auf die größere Starrheit von TEMPIC im Vergleich zu TMPMP zurückgeführt.

In der Folge wurde eine Reihe von weiteren Vergleichsbeispielen und Beispielen für Zwei-Komponenten-Zusammensetzungen als Kombinationen von TATATO als Monomer, TEMPIC als Thiol und Ivocerin als Initiator mit den in den umseitigen Tabellen 5 und 6 angeführten Primern hergestellt.

**Tabelle 5 - erste (PF) und zweite (MF) Komponente von Zwei-Komponenten-Zusammensetzungen nach dem Stand der Technik und gemäß vorliegender Erfindung unter Variation des Primers in den Primerformulierungen PF als erste Komponente**

| **Beispiel** | **Formulierung** | **Primer 15 Gew.-%** | **TATATO [Gew.-%]** | **TEMPIC [Gew.-%]** | **Ivocerin [Gew.-%]** | **SBS HAP [MPa]** |
|---|---|---|---|---|---|---|
| Vergleichsbeispiel 6 (V6) | PF | BAPA^{Phn} (S1) | 12,5 | 71,0 | 1,5 | 1,8 |
| Vergleichsbeispiel 8 (V8) | PF | BAPA^{bisPhn} (S2) * | 11,5 | 70,0 | 1,5 | 3,9 |
| Vergleichsbeispiel 9 (V9) | PF | Phn-1C-Allo (S3) | 12,5 | 71,0 | 1,5 | 3,1 |
| Vergleichsbeispiel 10 (V10) | PF | Phn-6C-Allo (S4) | 12,5 | 71,0 | 1,5 | 3,5 |
| Vergleichsbeispiel 11 (V11) | PF | Phn-6C-bisAllo (S5) | 12,5 | 71,0 | 1,5 | 5,2 |
| Vergleichsbeispiel 12 (V12) | PF | Phn-1C-Allcarb (S6) | 12,5 | 71,0 | 1,5 | 2,7 |
| Vergleichsbeispiel 13 (V13) | PF | Phn-6C-Allcarb (S7) | 12,5 | 71,0 | 1,5 | 3,1 |
| Vergleichsbeispiel 14 (V14) | PF | Phn-6C-bisAllcarb (S8) | 12,5 | 71,0 | 1,5 | 4,1 |
| Vergleichsbeispiel 15 (V15) | PF | Phn-6C-Enyl (S9) * | 11,5 | 70,0 | 1,5 | 2,9 |
| Vergleichsbeispiel 16 (V16) | PF | Phn-11C-Enyl (S10) * | 11,5 | 70,0 | 1,5 | 4,1 |
| Beispiel 15 (B15) | PF | Phn-6C-bisNorb (B5) | 12,5 | 71,0 | 1,5 | 11,0 |
| Beispiel 17 (B17) | PF | Alendronat-Norb (B1) * | 11,5 | 70,0 | 1,5 | 3,7 |
| Beispiel 18 (B18) | PF | Phn-1C-Norb (B3) | 12,5 | 71,0 | 1,5 | 3,0 |
| Beispiel 19 (B19) | PF | Phn-6C-Norb (B4) | 12,5 | 71,0 | 1,5 | 3,8 |
| Beispiel 20 (B20) | PF | bisPhn-6C-bisNorb (B6) * | 12,5 | 71,0 | 1,5 | 6,8 |
| | | | | | | |
| alle obigen Beispiele | MF | - | 32,0 | 67,0 | 1,0 | |

| | | | | | | |
|---|---|---|---|---|---|---|
| *: Zur Verbesserung der Löslichkeit in der Formulierung wurden 1 Gew.-% Wasser und 1 Gew.-% Ethanol auf Kosten der Mengen an Monomer und Thiol zugesetzt. | | | | | | |

**Tabelle 6 - erste (PF) und zweite (MF) Komponente von Zwei-Komponenten-Zusammensetzungen nach dem Stand der Technik und gemäß vorliegender Erfindung unter Variation des Primers in den Primerformulierungen PF als erste Komponente**

| **Beispiel** | **Formulierung** | **Primer 15 Gew.-%** | **TATATO [Gew.-%]** | **TEMPIC [Gew.-%]** | **Ivocerin [Gew.-%]** | **SBS TiO₂ [MPa]** |
|---|---|---|---|---|---|---|
| Vergleichsbeispiel 6 (V6) | PF | BAPA^{Phn} (S1) | 12,5 | 71,0 | 1,5 | 3,4 |
| Vergleichsbeispiel 8 (V8) | PF | BAPA^{bisPhn} (S2) * | 11,5 | 70,0 | 1,5 | 4,8 |
| Vergleichsbeispiel 9 (V9) | PF | Phn-1C-Allo (S3) | 12,5 | 71,0 | 1,5 | 1,7 |
| Vergleichsbeispiel 10 (V10) | PF | Phn-6C-Allo (S4) | 12,5 | 71,0 | 1,5 | 2,3 |
| Vergleichsbeispiel 11 (V11) | PF | Phn-6C-bisAllo (S5) | 12,5 | 71,0 | 1,5 | 2,3 |
| Vergleichsbeispiel 12 (V12) | PF | Phn-1C-Allcarb (S6) | 12,5 | 71,0 | 1,5 | 1,8 |
| Vergleichsbeispiel 13 (V13) | PF | Phn-6C-Allcarb (S7) | 12,5 | 71,0 | 1,5 | 2,2 |
| Vergleichsbeispiel 14 (V14) | PF | Phn-6C-bisAllcarb (S8) | 12,5 | 71,0 | 1,5 | 2,4 |
| Vergleichsbeispiel 15 (V15) | PF | Phn-6C-Enyl (S9) * | 11,5 | 70,0 | 1,5 | 2,0 |
| Vergleichsbeispiel 16 (V16) | PF | Phn-11C-Enyl (S10) * | 11,5 | 70,0 | 1,5 | 2,3 |
| Beispiel 15 (B15) | PF | Phn-6C-bisNorb (B5) | 12,5 | 71,0 | 1,5 | 7,9 |
| Beispiel 17 (B17) | PF | Alendronat-Norb (B1) * | 11,5 | 70,0 | 1,5 | 7,5 |
| Beispiel 18 (B18) | PF | Phn-1C-Norb (B3) | 12,5 | 71,0 | 1,5 | 2,8 |
| Beispiel 19 (B19) | PF | Phn-6C-Norb (B4) | 12,5 | 71,0 | 1,5 | 2,7 |
| Beispiel 20 (B20) | PF | bisPhn-6C-bisNorb (B6) * | 12,5 | 71,0 | 1,5 | 4,5 |
| | | | | | | |
| alle obigen Beispiele | MF | - | 32,0 | 67,0 | 1,0 | |

| | | | | | | |
|---|---|---|---|---|---|---|
| *: Zur Verbesserung der Löslichkeit in der Formulierung wurden 1 Gew.-% Wasser und 1 Gew.-% Ethanol auf Kosten der Mengen an Monomer und Thiol zugesetzt. | | | | | | |

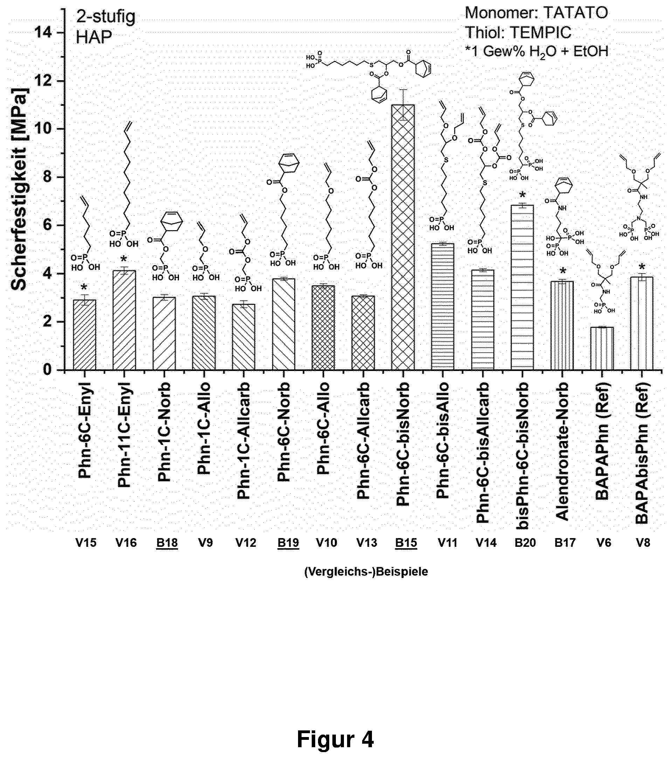

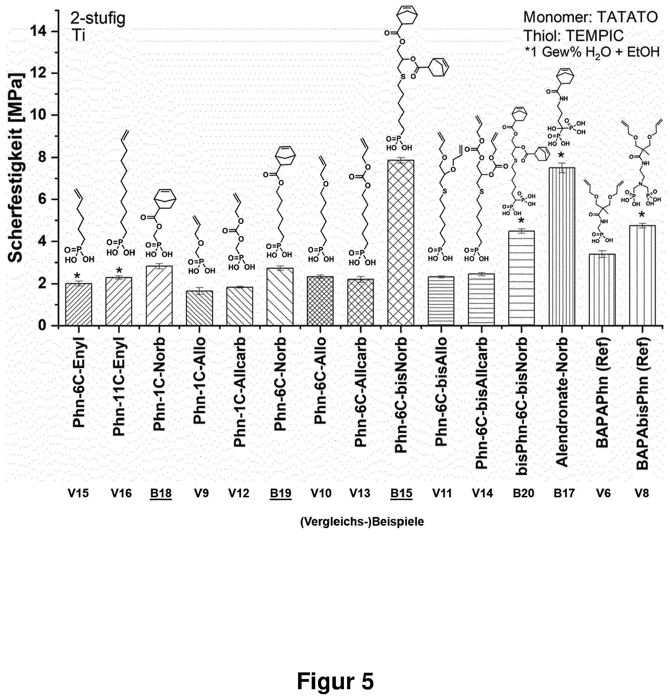

Diese neuen Zwei-Komponenten-Zusammensetzungen der Vergleichsbeispiele 8 bis 16 sowie der Beispiele 17 bis 20 wurden auf identische Weise wie zuvor auf Hydroxylapatit-Oberflächen, nun aber auch - genau wie jene von Vergleichsbeispiel 6 und Beispiel 15 - auf Titandioxid-Oberflächen aufgebracht, (vor)gehärtet und erneut auf die Scherhaftfestigkeit (SBS) der jeweiligen Beschichtung untersucht. Die dabei erzielten Ergebnisse sind zusammen mit den zuvor für Vergleichsbeispiel 6 und Beispiel 15 erhaltenen in Tabelle 5 und Fig. 4 für Hydroxylapatit ("HAP") und in Tabelle 6 und in Fig. 5 für Titandioxid ("Ti") dargestellt.

Aus Tabelle 5 und Fig. 4 ist zunächst zu erkennen, dass Primer mit einer Kettenlänge des Spacers zwischen den polymerisierbaren Gruppen PG (Vinyl, Allyl oder erfindungsgemäß Norbornenyl) und den Adhäsionsmotiven AM (hier durchwegs Phosphonsäure-Gruppen) von zumindest 6 Kohlenstoff- bzw. Heteroatomen auf Hydroxylapatit tendenziell bessere Scherhaftfestigkeiten ergaben als die kürzerkettigen Spacer in den Vergleichsbeispielen 15, 9, 12 und 6 und in Beispiel 18 (Phn-1C-Norb). Weiters zeigte sich, dass Primer mit mehr als einer PG und/oder mehr als einem AM ebenfalls tendenziell besser abschnitten als solche mit nur jeweils einer entsprechenden Gruppierung (d.s. in Fig. 4 alle Ergebnisse links von dem Top-Wert für Beispiel 15).

Weiters fällt auf, dass der Referenz-Primer BAPA^{Phn} aus Vergleichsbeispiel 6 - trotz der Gegenwart zweier Allylgruppen als PG und einer Spacer-Länge von 6 Atomen zwischen der Phosphonsäure-Gruppierung und jeder der beiden Allylgruppen - mit Abstand das schlechteste Ergebnis geliefert hat. Im Gegensatz dazu wurden für die Vergleichsbeispiele 11, 14 und 8, d.h. Primer mit 9 bis 11 Atomen zwischen PG und AM, zwei- bis dreimal so hohe Scherhaftfestigkeiten gemessen, wovon die beiden Erstgenannten ebenfalls nur ein AM und zwei Allylgruppen als PG enthalten, während BAPA^{bisPhn} eine zusätzliche Phosphonsäure-Gruppierung (HO)₂P(=O)- aufweist.

Nichtsdestotrotz schneiden die erfindungsgemäßen Primer mit Norbornenyl als PG im direkten Vergleich mit diesen drei besten Primern nach dem Stand der Technik ebenfalls besser ab: Alendronat-Norb aus Beispiel 17 mit nur einer PG und zwei AM war etwa gleich effektiv wie BAPA^{bisPhn} mit zwei PG und zwei AM und kaum schlechter als Phn-6C-bisAllcarb mit einer AM und zwei Allylgruppen als PG. Und Phn-6C-bisNorb aus Beispiel 15 sowie bisPhn-6C-bisNorb aus Beispiel 20 mit jeweils zwei Norbornenyl-PG und einer bzw. zwei Phosphonsäure-Gruppierungen ergaben deutlich die besten Scherhaftfestigkeiten SBS der gehärteten Beschichtungen auf Hydroxylapatit, wobei erstaunlicherweise Phn-6C-bisNorb aus Beispiel 15 mit nur einem AM sogar noch bessere Werte lieferte. Offenbar hatten die Erfinder zufällig den besten der erfindungsgemäßen neuen Primer von Anfang an für ihre Untersuchungen ausgewählt.

Etwas anders stellt sich die Situation bei den Versuchen auf Titandioxid-Oberflächen dar. Wie Tabelle 6 und Fig. 5 zu entnehmen ist, sind zwar wiederum längerkettige Spacer mit zumindest 6 Atomen zwischen PG- und AM-Gruppierungen zu bevorzugen, allerdings lieferten in diesen Fällen die beiden BAPA-hältigen Primer der Vergleichsbeispiele 6 und 8 die besten Ergebnisse aller Primer nach dem Stand der Technik, wobei BAPA^{bisPhn} mit zwei AM und zwei PG erneut bessere Scherhaftfestigkeiten ergab als BAPA^{Phn} mit nur einer Phosphonsäure-Gruppierung. Der erfindungsgemäße Primer bisPhn-6C-bisNorb aus Beispiel 20 mit ebenfalls zwei AM und zwei Norbornenyl-PG liegt jedoch nahezu gleichauf mit BAPA^{bisPhn}, während Alendronat-Norb aus Beispiel 17 mit nur einer Norbornenyl-Gruppe und zwei AM, das zuvor auf Hydroxylapatit etwa gleich mit BAPA^{bisPhn} abgeschnitten hatte, dieses nun deutlich übertrifft und seinerseits nur von Phn-6C-bisNorb mit zwei Norbornenyl-PG und nur einer Phosphonsäure-AM aus Beispiel 15 übertroffen wird, das somit erneut die besten Werte lieferte.

Allerdings haben auf Titandioxid auch die Primer der Beispiele 18 und 19 mit jeweils nur einer Norbornenyl-PG und einer Phosphonsäure-Gruppierung bessere Werte ergeben als alle Primer nach dem Stand der Technik mit Ausnahme der beiden BAPA-hältigen - und somit sogar besser als die Primer der Vergleichsbeispiele 11 und 14 mit jeweils zwei Allyl-PG; und das, obwohl Phn-1C-Norb aus Beispiel 18 einen Spacer mit einer Kettenlänge von nur 3 Atomen umfasst.

Zusammenfassend kann festgestellt werden, dass die erfindungsgemäße Kombination aus 5-Norbornen-2-yl-Gruppen als polymerisierbare Gruppen PG und Phosphonsäure-Gruppen (HO)₂P(=O)- als Adhäsionsmotive AM in Primern für polymerisierbare Zwei-Komponenten-Zusammensetzungen auf Thiol-En-Basis jenen nach dem Stand der Technik deutlich überlegen sind. Dabei überwiegen offenbar die Reaktivität der Norbornenyl-Gruppen bei der Polymerisation der Primerformulierung und der daraus resultierende rasche Einbau der Primer in die Monomermatrix gegenüber der durch die Adhäsionsmotive hervorgerufenen Haftwirkung auf der beschichteten Oberfläche.

In der Folge wurden weitere Zwei-Komponenten-Zusammensetzungen unter Verwendung der zuvor hergestellten Primer mit Brenzcatechin- (Catechol-) Gruppen als Adhäsionsmotive AM hergestellt und auf identische Weise wie oben auf die Scherhaftfestigkeit (SBS) der daraus erhaltenen Beschichtungen untersucht, wobei zu Vergleichszwecken auch die Werte der für die BAPA-hältigen Primer der Vergleichsbeispiele 6 und 8 und für den besten erfindungsgemäßen Primer Phn-6C-bisNorb aus Beispiel 15 angegeben werden. Als neue Brenzcatechin-hältige Primer-Zusammensetzungen wurden wiederum Kombinationen von TATATO als Monomer, TEMPIC als Thiol und Ivocerin als Initiator nun mit den in den umseitigen Tabellen 7 und 8 angeführten Primern hergestellt, wobei es sich bei Vergleichsbeispiel 18 um einen Literaturwert gemäß Olofsson et al., s.o., handelt. Dieser wurde allerdings nicht auf Hydroxylapatit, sondern auf Knochen bestimmt, und zwar mit einer Zwei-Komponenten-Zusammensetzung, die ebenfalls TATATO als Monomer und TEMPIC als Thiol, als Initiator jedoch nicht Ivocerin, sondern Campherchinon enthielt. Als Primer kam dabei ein ein 1:1-Gemisch aus DOPA-Allyl und DOPA-Thiol zum Einsatz:

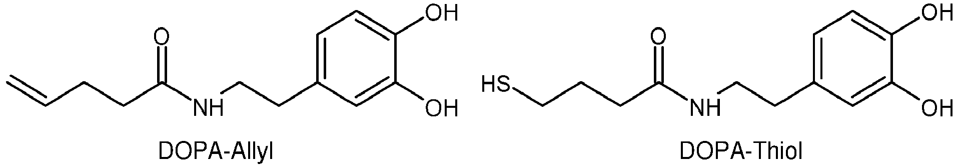

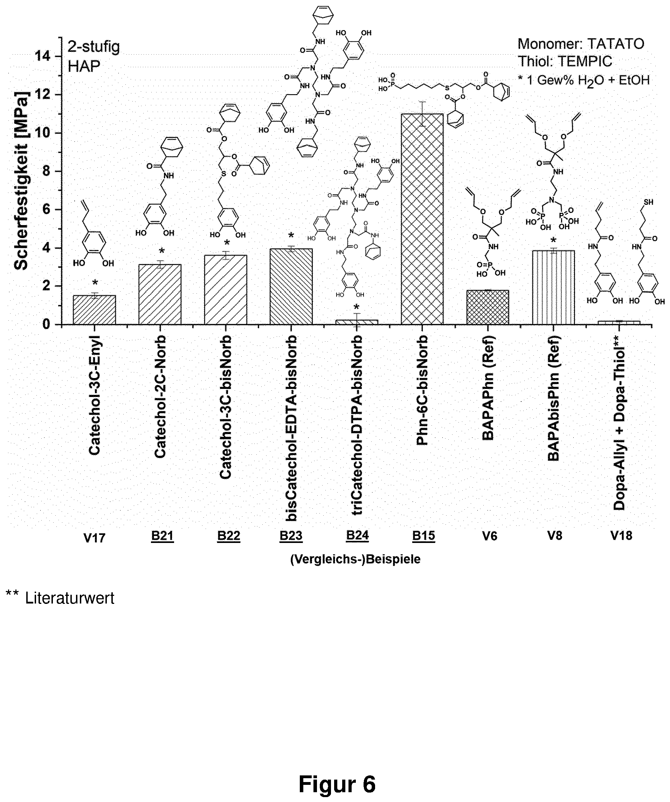

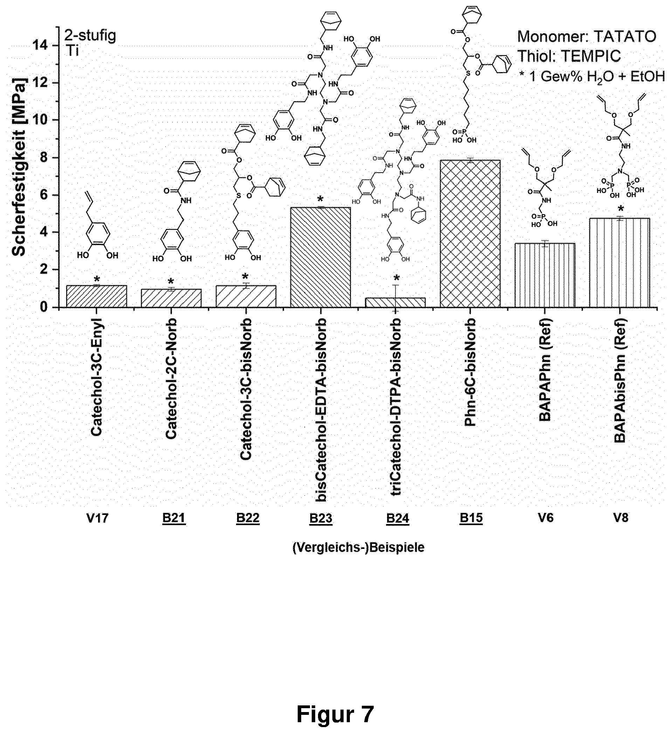

Die Ergebnisse sind ebenfalls in den Tabellen 7 und 8 angegeben und in den Fig. 6 und 7 dargestellt.

**Tabelle 7 - erste (PF) und zweite (MF) Komponente von Zwei-Komponenten-Zusammensetzungen nach dem Stand der Technik und gemäß vorliegender Erfindung unter Variation des Primers in den Primerformulierungen PF als erste Komponente**

| **Beispiel** | **Formulierung** | **Primer 15 Gew.-%** | **TATATO [Gew.-%]** | **TEMPIC [Gew.-%]** | **Ivocerin [Gew.-%]** | **SBS HAP [MPa]** |
|---|---|---|---|---|---|---|
| Vergleichsbeispiel 6 (V6) | PF | BAPA^{Phn} (S1) | 12,5 | 71,0 | 1,5 | 1,8 |
| Vergleichsbeispiel 8 (V8) | PF | BAPA^{bisPhn} (S2) * | 11,5 | 70,0 | 1,5 | 3,9 |
| Vergleichsbeispiel 17 (V17) | PF | Catechol-3C-Enyl (S11) * | 11,5 | 70,0 | 1,5 | 1,5 |
| Vergleichsbeispiel 18 (V18) | PF | DOPA-Allyl + DOPA-Thiol ** | - | - | - | 0,29 |
| Beispiel 15 (B15) | PF | Phn-6C-bisNorb (B5) | 12,5 | 71,0 | 1,5 | 11,0 |
| Beispiel 21 (B21) | PF | Catechol-2C-Norb (B2) * | 11,5 | 70,0 | 1,5 | 3,1 |
| Beispiel 22 (B22) | PF | Catechol-3C-bisNorb (B7) * | 11,5 | 70,0 | 1,5 | 3,6 |
| Beispiel 23 (B23) | PF | bisCatechol-EDTA-bisNorb (B8) * | 11,5 | 70,0 | 1,5 | 4,0 |
| Beispiel 24 (B24) | PF | triCatechol-DTPA-bisNorb (B9) * | 11,5 | 70,0 | 1,5 | 0,4 |
| | | | | | | |
| obige Beispiele außer V18 | MF | - | 32,0 | 67,0 | 1,0 | |
| Vergleichsbeispiel 18 (V18) | MF | - | 32,1 | 67,8 | 0,1 *** | |

| | | | | | | |
|---|---|---|---|---|---|---|
| *: Zur Verbesserung der Löslichkeit in der Formulierung wurden 1 Gew.-% Wasser und 1 Gew.-% Ethanol auf Kosten der Mengen an Monomer und Thiol zugesetzt. **: SBS-Wert aus der Literatur; Olofsson et al. (s.o.) ***: Campherchinon als Initiator | | | | | | |

**Tabelle 8 - erste (PF) und zweite (MF) Komponente von Zwei-Komponenten-Zusammensetzungen nach dem Stand der Technik und gemäß vorliegender Erfindung unter Variation des Primers in den Primerformulierungen PF als erste Komponente**

| **Beispiel** | **Formulierung** | **Primer 15 Gew.-%** | **TATATO [Gew.-%]** | **TEMPIC [Gew.-%]** | **Ivocerin [Gew.-%]** | **SBS TiO₂ [MPa]** |
|---|---|---|---|---|---|---|
| Vergleichsbeispiel 6 (V6) | PF | BAPA^{Phn} (S1) | 12,5 | 71,0 | 1,5 | 3,4 |
| Vergleichsbeispiel 8 (V8) | PF | BAPA^{bisPhn} (S2) * | 11,5 | 70,0 | 1,5 | 4,8 |
| Vergleichsbeispiel 17 (V17) | PF | Catechol-3C-Enyl (S11) * | 11,5 | 70,0 | 1,5 | 1,1 |
| Beispiel 15 (B15) | PF | Phn-6C-bisNorb (B5) | 12,5 | 71,0 | 1,5 | 7,9 |
| Beispiel 21 (B21) | PF | Catechol-2C-Norb (B2) * | 11,5 | 70,0 | 1,5 | 0,9 |
| Beispiel 22 (B22) | PF | Catechol-3C-bisNorb (B7) * | 11,5 | 70,0 | 1,5 | 1,1 |
| Beispiel 23 (B23) | PF | bisCatechol-EDTA-bisNorb (B8) * | 11,5 | 70,0 | 1,5 | 5,3 |
| Beispiel 24 (B24) | PF | triCatechol-DTPA-bisNorb (B9) * | 11,5 | 70,0 | 1,5 | 0,5 |
| | | | | | | |
| alle obigen Beispiele | MF | - | 32,0 | 67,0 | 1,0 | |

| | | | | | | |
|---|---|---|---|---|---|---|
| *: Zur Verbesserung der Löslichkeit in der Formulierung wurden 1 Gew.-% Wasser und 1 Gew.-% Ethanol auf Kosten der Mengen an Monomer und Thiol zugesetzt. | | | | | | |

Aus den SBS-Ergebnissen in Tabelle 7 und Fig. 6 ist zunächst zu erkennen, dass der erfindungsgemäße Primer triCatechol-DTPA-bisNorb aus Beispiel 24 eine Scherhaftfestigkeit ergeben hatte, die nur geringfügig besser war als der in der Literatur zitierte Wert für das Gemisch aus DOPA-Allyl und DOPA-Thiol, aber deutlich schlechter als die übrigen Werte für Brenzcatechin-hältige Primer. Dies ist jedoch zum Teil ebenso darauf zurückzuführen, dass es sich um gewichtsbezogene Primeranteile in den Formulierungen handelte, wie die Tatsache, dass die drei erfindungsgemäßen Primer der Beispiele 21 bis 23 nicht besser abgeschnitten hatten als der äußerst kurzkettige Primer aus Vergleichsbeispiel 17 mit einer Spacerlänge von nur einem einzigen Kohlenstoffatom. Berücksichtigt man die stark unterschiedlichen Molekulargewichte, enthielten die Primerformulierungen der Beispiele 21 bis 24 nur rund die Hälfte (B21), ein Drittel (B22), ein Fünftel (B23) bzw. ein Siebentel (B24) der molaren Menge an Primer in Vergleichsbeispiel 17 (Catechol-3C-Enyl). Allerdings dürfte der vergleichsweise schlechte Wert von Beispiel 24 nach Ansicht der Erfinder vor allem auf dem Umstand beruhen, dass dieser triCatechol-DTPA-bisNorb-Primer im Monomer-Thiol-Gemisch trotz des Zusatzes von Wasser und Ethanol nicht vollständig löslich war. Weitere Tests mit diesem Primer sind daher Gegenstand derzeitiger Untersuchungen der Erfinder.

Die Wirksamkeit der drei erfindungsgemäßen Primer der Beispiele 21 bis 23 ist jedoch aufgrund der hohen Molekulargewichtsdifferenzen zu jenem aus Vergleichsbeispiel 17, aber auch zu den beiden BAPA-hältigen Primern der Vergleichsbeispiele 6 und 8 noch deutlich höher einzuschätzen, als es die Werte in Tabelle 8 und die Grafik in Fig. 6 widerspiegeln. Dies gilt sinngemäß natürlich auch für die obigen Beispiele mit Phosphonsäure-Gruppierungen als Adhäsionsmotiv.

Im direkten Vergleich zwischen den Primern mit zwei polymerisierbaren Gruppen PG und einem Adhäsionsmotiv AM, d.h. Catechol-3C-bisNorb (B22) und BAPA^{Phn} (V6), bewirkte der erfindungsgemäße Primer einen doppelt so hohen Scherhaftfestigkeitswert. Bei Vergleich zwischen den Primern mit je zwei PG und zwei AM, d.h. bisCatechol-EDTA-bisNorb (aus B23) und BAPA^{bisPhn} (aus V8), schneidet der erfindungsgemäße Primer zwar nur geringfügig besser ab, allerdings ist auch das einerseits wieder auf sein deutlich höheres Molekulargewicht, andererseits aber auf den Umstand zurückzuführen, dass Phosphonsäure-Gruppen auf Hydroxylapatit-Oberflächen wirksamere Adhäsionsmotive sind als Brenzcatechyl-Reste, wie der hier zu Vergleichszwecken erneut angeführte Spitzenwert des Primers aus Beispiel 15 klar belegt. Dieser beträgt rund das Dreifache jenes von Beispiel 22, obwohl beide Primer analog aufgebaut sind (je 2 PG und 1 AM) und vergleichbare Molekulargewichte aufweisen. Speziell dieser letztere Effekt wirkt sich auf Titandioxid-Oberflächen noch stärker aus, wie die Werte in Tabelle 8 und die Grafik in Fig. 7 belegen. Hier beträgt der Spitzenwert des erfindungsgemäßen Primers aus Beispiel 15 sogar rund das Siebenfache jenes von Beispiel 22, woraus folgt, dass Phosphonsäure-Gruppen speziell auf Titandioxid als AM zu bevorzugen sind. Ohne sich darauf festlegen zu wollen, nehmen die Erfinder an, dass dies zum Teil auf gewisse inhibierende Wirkung der phenolischen OH-Gruppen während der radikalischen Polymerisation zurückzuführen sein könnte.

Neben dem löslichkeitsbedingt wiederum schlechten Wert für den triCatechol-DTPA-bisNorb-Primer aus Beispiel 24 fällt in diesem Fall jedoch auch auf, dass der erfindungsgemäße Primer aus Beispiel 23 mit zwei Norbonenyl-PG und zwei Brenzcatechyl-AM hier im Vergleich zum Spitzenwert aus Beispiel 15 besser abschnitt als zuvor auf Hydroxylapatit, was erneut die erfindungsgemäße Bevorzugung von zumindest zwei Norbonenyl-Gruppen als PG und zumindest zwei Phosphonsäure- oder Brenzcatechyl-Gruppen als AM belegt. Außerdem ist der Wert für Beispiel 23 in diesem Fall sogar rund 10% besser als jener für den analog aufgebauten BAPA-Primer aus Vergleichsbeispiel 8 - obwohl Letzterer zwei Phosphonsäure-PG aufweist, die eine höhere Haftung verleihen als die beiden Brenzcatechyl-Gruppen von bisCatechol-EDTA-bisNorb aus Beispiel 23.

### Beispiele 25 bis 36, Vergleichsbeispiele 19 bis 30 - Ein-Komponenten-Zusammensetzungen

In analoger Weise zu oben wurden in dieser Gruppe von Beispielen Ein-Komponenten-Zusammensetzungen durch Vermischen von Monomeren, Thiolen, Initiator und je einem erfindungsgemäßen oder aus dem Stand der Technik bekannten Primer hergestellt, auf eine polierte Substrat-Oberfläche aus Hydroxylapatit oder Titandioxid aufgebracht, 20 s lang bestrahlt und somit gehärtet, in modifizierte simulierte Körperflüssigkeit eingelegt, bei 37 °C 24 h lang gelagert und anschließend unter Verwendung des VTSYIQI SF-30 Digitalen Kraftmessgeräts mit einem V-förmigen Prüfstab auf die Scherhaftfestigkeit (SBS) der gehärteten Beschichtung geprüft.

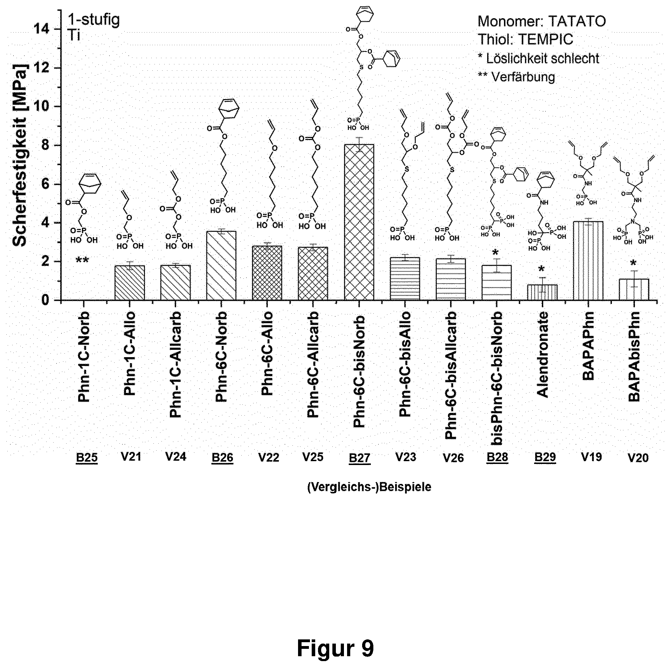

Sämtliche Formulierungen der Ein-Komponenten-Zusammensetzungen enthielten als Monomer TATATO (27,0 Gew.-%), als Thiol TEMPIC (57,0 Gew.-%) und als Initiator Ivocerin (1,0 Gew.-%). Als Primer wurden nur Phosphonsäure-Gruppen als AM enthaltende Verbindungen (15 Gew.-%) eingesetzt. In Tabelle 9 sind die so hergestellten Formulierungen, die erneut jeweils zusätzlich 0,02 Gew.-% Pyrogallol als Oxidationsstabilisator enthielten, sowie die so erzielten Scherhaftfestigkeitswerte auf Hydroxylapatit- (HAP) und Titandioxid- (TiO₂) Oberflächen angegeben, wobei diese Werte auch in Fig. 8 bzw. Fig. 9 grafisch dargestellt sind.

**Tabelle 9 - Ein-Komponenten-Zusammensetzungen nach dem Stand der Technik und gemäß vorliegender Erfindung unter Variation des Primers**

| **Beispiel** | **Primer** | **SBS HAP [MPa]** | **SBS TiO₂ [MPa]** |
|---|---|---|---|
| Vergleichsbeispiel 19 (V19) | BAPA^{Phn} (S1) | 8,1 | 4,1 |
| Vergleichsbeispiel 20 (V20) | BAPA^{bisPhn} (S2) * | 1,0 | 1,1 |
| Vergleichsbeispiel 21 (V21) | Phn-1C-Allo (S3) | 1,6 | 1,8 |
| Vergleichsbeispiel 22 (V22) | Phn-6C-Allo (S4) | 3,6 | 2,8 |
| Vergleichsbeispiel 23 (V23) | Phn-6C-bisAllo (S5) | 3,5 | 2,2 |
| Vergleichsbeispiel 24 (V24) | Phn-1C-Allcarb (S6) | 1,8 | 1,8 |
| Vergleichsbeispiel 25 (V25) | Phn-6C-Allcarb (S7) | 3,6 | 2,7 |
| Vergleichsbeispiel 26 (V26) | Phn-6C-bisAllcarb (S8) | 3,5 | 2,1 |
| Beispiel 25 (B25) | Phn-1C-Norb (B3) ** | 0,0 | 0,0 |
| Beispiel 26 (B26) | Phn-6C-Norb (B4) | 8,0 | 3,6 |
| Beispiel 27 (B27) | Phn-6C-bisNorb (B5) | 15,4 | 8,0 |
| Beispiel 28 (B28) | bisPhn-6C-bisNorb (B6) * | 0,5 | 1,8 |
| Beispiel 29 (B29) | Alendronat-Norb (B1) * | 1,1 | 0,8 |

| | | | |
|---|---|---|---|
| *: nicht vollständig löslich **: verfärbt | | | |

Die mit* gekennzeichneten Primer waren in den Formulierungen nicht vollständig löslich, und der mit ** gekennzeichnete Primer aus Beispiel 25 führte zu einer starken Braunfärbung der Formulierung, was bei Belichtung das Eindringen der Strahlung stark behinderte und die Durchhärtungstiefe deutlich reduzierte, so dass nahezu keine Haftung am Substrat feststellbar war. Weitere Tests mit diesen Primern, unter anderem unter erneutem Zusatz von H₂O und Ethanol zur Verbesserung der Löslichkeit, sind daher Gegenstand derzeitiger Untersuchungen der Erfinder.

Wie aus Tabelle 9 und Fig. 8 zu erkennen, waren jedoch die beiden anderen erfindungsgemäßen Primer der Beispiele 26 und 27, bei denen keine Probleme aufgetreten waren, jenen der Vergleichsbeispiele erneut überlegen, von denen BAPA^{Phn} (S1) aus Vergleichsbeispiel 19 mit Abstand am besten abgeschnitten hatte, da er mehr als das Doppelte der Scherhaftfestigkeit aller übrigen Vergleichsprimer erreicht hatte. Dennoch hatte Phn-6C-Norb (B4) aus Beispiel 26 auf Hydroxylapatit (HAP) nahezu denselben Scherhaftfestigkeitswert ergeben, obwohl dieser neue Primer nur eine polymerisierbare Norbornenyl-Gruppe enthält, während BAPA^{Phn} (S1) zwei Allylgruppen aufweist. Der beste Primer gemäß vorliegender Erfindung war erwartungsgemäß ein weiteres Mal der zu BAPA^{Phn} (S1) strukturanaloge Phn-6C-bisNorb (B5) aus Beispiel 27 mit seinen zwei Norbornenyl-Gruppen und einer Phosphonsäure-Gruppierung als Adhäsionsmotiv, der sogar nahezu den doppelten Scherhaftfestigkeitswert von BAPA^{Phn} (S1) erreicht hatte - und das sowohl auf Hydroxylapatit als auch auf Titandioxid, wie in Fig. 9 gut zu erkennen ist. Auch auf TiO₂ hatte der Primer aus Beispiel 26 mit nur einer polymerisierbare Gruppe besser als alle Vergleichsprimer mit Ausnahme von BAPA^{Phn} (S1) abgeschnitten, dessen Wert hier rund 10% höher liegt.

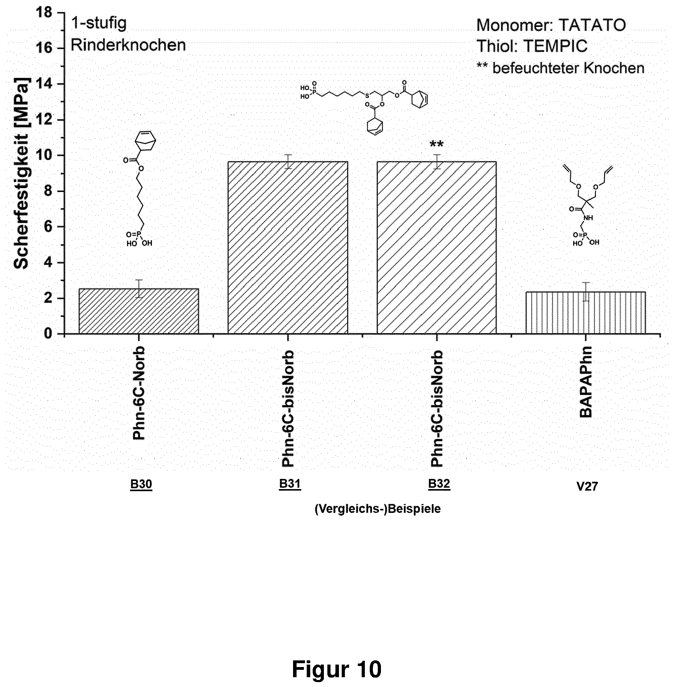

In der Folge wurden die drei Primer BAPA^{Phn} (S1), Phn-6C-Norb (B4) und Phn-6C-bisNorb (B5) auch auf den wie zuvor beschrieben präparierten Rinderzehenknochen-Scheiben in Form von analogen Ein-Komponenten-Zusammensetzungen untersucht, die neben 15 Gew.-% Primer wiederum 27,0 Gew.-% TATATO als Monomer, 57,0 Gew.-% TEMPIC als Thiol und 1,0 Gew.-% Ivocerin als Initiator enthielten. Die Aufbringung, Härtung und Messung erfolgten wiederum analog zu oben. Der beste erfindungsgemäße Primer Phn-6C-bisNorb (B5) wurde zudem ein zweites Mal auf einer solchen Knochenscheibe getestet, wobei die Scheibe allerdings vor der Aufbringung mit befeuchtet wurde, indem destilliertes Wasser mit einer Mikrobürste 20 s lang eingerieben und die Oberfläche danach 10 s lang an der Luft antrocknen gelassen wurde. Die Zusammensetzung der Formulierungen und die damit erzielten Ergebnisse sind in der nachstehenden Tabelle 10 angegeben und Letztere in Fig. 10 auch grafisch dargestellt.

**Tabelle 10 - Ein-Komponenten-Zusammensetzungen nach dem Stand der Technik und gemäß vorliegender Erfindung auf Rinderknochen**

| **Beispiel** | **Primer** | **SBS Knochen [MPa]** |
|---|---|---|
| Vergleichsbeispiel 27 (V27) | BAPA^{Phn} (S1) | 2,4 |
| Beispiel 30 (B30) | Phn-6C-Norb (B4) | 2,5 |
| Beispiel 31 (B31) | Phn-6C-bisNorb (B5) | 9,6 |
| Beispiel 32 (B32) | Phn-6C-bisNorb (B5) ** | 9,6 |

| | | |
|---|---|---|
| **: befeuchteter Knochen | | |

Die Verhältnisse der gemessenen Werte waren erwartungsgemäß ähnlich wie zuvor auf Hydroxylapatit, das ja schließlich als Modellsubstanz für Versuche mit Knochenklebern eingesetzt wird, auch wenn die Scherfestigkeit auf glattpolierten, homogenen Hydroxylapatit-Oberflächen jene auf Knochenscheiben nachvollziehbarerweise höher gelegen hatte. Allerdings übertrafen die beiden erfindungsgemäßen Primer auf Knochenmaterial den bekannten Primer BAPA^{Phn} (S1) aus Vergleichsbeispiel 27 noch deutlicher als zuvor. In diesem Fall ist der mit Phn-6C-Norb (B4) in Beispiel 30 erzielte Scherhaftfestigkeitswert sogar etwas höher als der Vergleichswert für den Primer mit zwei polymerisierbaren Gruppen, jener für den strukturanalogen Primer Phn-6C-bisNorb (B5) beträgt hier allerdings sogar nahezu das Fünffache des Vergleichs - und das sowohl auf trockenem (Beispiel 31) als auch auf zuvor befeuchtetem und kurz antrocknen gelassenem Rinderknochen (Beispiel 32).

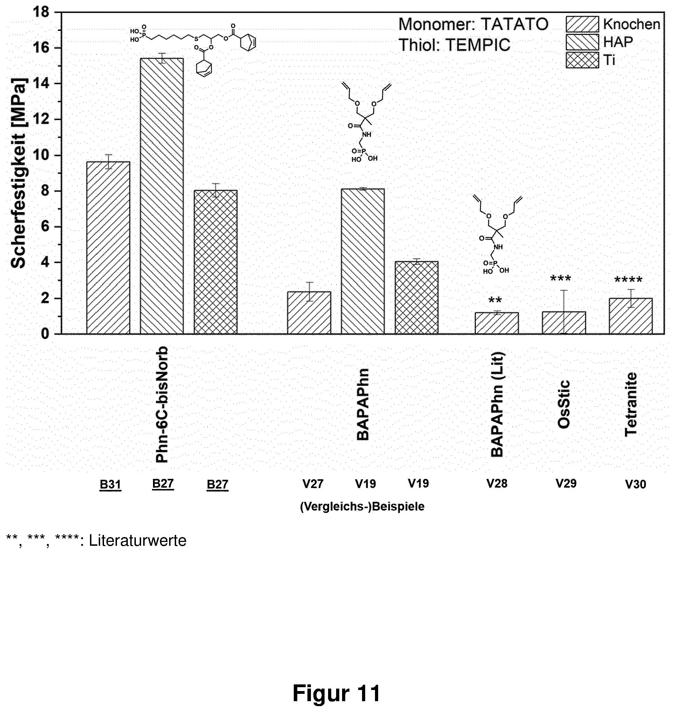

In der nachstehenden Tabelle 11 sind die Ergebnisse für den besten erfindungsgemäßen Primer Phn-6C-bisNorb (B5) und den strukturanalogen Vergleichsprimer BAPA^{Phn} (S1) auf allen drei untersuchten Oberflächen, Rinderknochen ("Knochen"), Hydroxylapatit (HAP) und Titandioxid ("Ti") erneut angeführt, wobei als zusätzliche Vergleichsbeispiele Literaturwerte für BAPA^{Phn} (aus Granskog et al., s.o.) sowie für die eingangs erwähnten, unter den Markennamen OsStic^{™} von PBC Biomed und Tetranite^{™} von LaunchPad Medical erhältlichen Knochenkleber bzw. -zemente (aus Pujari-Palmer et al., s.o.; bzw. Kirillova et al., s.o.), jeweils auf Hydroxylapatit-Oberflächen, angegeben sind. In Fig. 11 sind diese Werte auch grafisch dargestellt.

**Tabelle 11 - Ein-Komponenten-Zusammensetzungen nach dem Stand der Technik und gemäß vorliegender Erfindung**

| **Beispiel** | **Primer** | **SBS K [MPa]** | **SBS HAP [MPa]** | **SBS TiO₂ [MPa]** |
|---|---|---|---|---|
| Vergleichsbeispiel 27 (V27) | BAPA^{Phn} (S1) | 2,4 | | |
| Vergleichsbeispiel 19 (V19) | BAPA^{Phn} (S1) | | 8,1 | 4,1 |
| Vergleichsbeispiel 28 (V28) | BAPA^{Phn} ** | 1,2 | | |
| Vergleichsbeispiel 29 (V29) | OsStic^{™} *** | 1,5 | | |
| Vergleichsbeispiel 30 (V30) | Tetranite^{™} **** | 1,9 | | 3,3 |
| Beispiel 31 (B31) | Phn-6C-bisNorb (B5) | 9,6 | | |
| Beispiel 27 (B27) | Phn-6C-bisNorb (B5) | | 15,4 | 8,0 |

| | | | | |
|---|---|---|---|---|
| **: Granskog et al. (s.o.) ***: Pujari-Palmer et al. (s.o.) ****: Kirillova et al. (s.o.) | | | | |

Ein-Komponenten-Zusammensetzungen mit dem besten erfindungsgemäßen Primer Phn-6C-bisNorb (B5) zeigen somit auf allen untersuchten Oberflächen deutlich bessere Ergebnisse als bisher bekannte Systeme. Aufgrund der, wie zuvor erwähnt, generell höheren Scherfestigkeit auf Hydroxylapatit-Oberflächen als auf Knochenscheiben, sind für die bekannten Systeme der Vergleichsbeispiele 28 bis 30 für Rinderknochen- , aber wohl auch für Titandioxid-Oberflächen noch deutlich niedrigere Werte als die in der Literatur für Hydroxylapatit angegebenen zu erwarten.

Abschließend wurden von den Erfindern noch Versuche mit gefüllten Ein-Komponenten-Zusammensetzungen unter Verwendung von Phn-6C-bisNorb (B5) durchgeführt, wobei die erneut 15 Gew.-% des Primers, 27,0 Gew.-% TATATO, 57,0 Gew.-% TEMPIC und 1,0 Gew.-% Ivocerin enthaltende Formulierung jeweils mit unterschiedlichen Mengen an Hydroxylapatit oder Titandioxid als Füllstoff vermischt wurde.

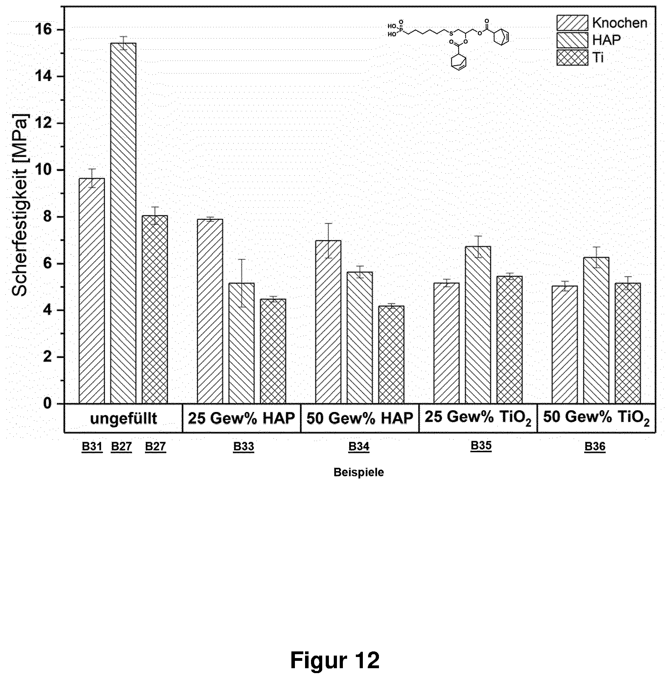

Um Probleme in Bezug auf die Eindringtiefe der Strahlung zu vermeiden, wurden die Füllstoffe mit dem erfindungsgemäßen Bisphosphonsäure-Primer bisPhn-6C-bisNorb (B6) funktionalisiert. Dazu wurden jeweils 4 g Füllstoff-Pulver mit 0,5 g des Primers in 10 ml MeOH:H₂O (1:4 Vol.) dispergiert, 24 h gerührt, abfiltriert, mit MeOH (3 x 50 ml) gewaschen und im Hochvakuum getrocknet. Die so funktionalisierten Füllstoffe wurden jeweils in den angegebenen Gewichtsteilen, bezogen auf 100 Gewichtsteile der Formulierung, vermischt. Die jeweiligen Zusammensetzungen und die damit auf den unterschiedlichen Oberflächen (Knochen, K, Hydroxylapatit, HAP, und Titandioxid, TiO₂) erzielten Ergebnisse sind in der nachstehenden Tabelle 12 angegeben und Letztere auch in Fig. 12 grafisch dargestellt. In Tabelle 12 sind darüber hinaus auch erneut die Werte der Vergleichsbeispiele mit BAPA^{Phn} als Primer ohne Füllstoff angeführt.

**Tabelle 12 - gefüllte Ein-Komponenten-Zusammensetzungen gemäß vorliegender Erfindung mit Phn-6C-bisNorb (B5) als Primer**

| **Beispiel** | **Füllstoff (Gewichtsteile)** | **SBS K [MPa]** | **SBS HAP [MPa]** | **SBS TiO₂ [MPa]** |
|---|---|---|---|---|
| Vergleichsbeispiel 27 (V27) | - | 2,4 | | |
| Vergleichsbeispiel 19 (V19) | - | | 8,1 | 4,1 |
| Beispiel 31 (B31) | - | 9,6 | | |
| Beispiel 27 (B27) | - | | 15,4 | 8,0 |
| Beispiel 33 (B33) | Hydroxylapatit (25) | 7,9 | 5,2 | 4,5 |
| Beispiel 34 (B34) | Hydroxylapatit (50) | 7,0 | 5,6 | 4,2 |
| Beispiel 35 (B35) | Titandioxid (25) | 5,2 | 6,7 | 5,5 |
| Beispiel 36 (B36) | Titandioxid (50) | 5,0 | 6,3 | 5,2 |

Dabei zu erkennen, dass die mit den gefüllten Formulierungen erzielten Scherhaftfestigkeiten im Vergleich zu den jeweiligen ungefüllten Beispielen zwar abgenommen haben, auf Rinderknochen und Titandioxid aber dennoch über den Werten der ungefüllten Vergleichsbeispiele mit BAPA^{Phn} als Primer liegen. Unter der Annahme einer ähnlich starken Abnahme der Scherhaftfestigkeit der Vergleichsbeispiele bei entsprechendem Zusatz von Füllstoff, d.h. etwa um einen Faktor von 2,5 bis 3, würden die erfindungsgemäßen Beispiele jene mit BAPA^{Phn} als Primer mit an Sicherheit grenzender Wahrscheinlichkeit auch auf Hydroxylapatit übertreffen. Entsprechende Versuche sind Gegenstand derzeitiger Untersuchungen der Erfinder.

Die obigen Beispiele und Vergleichsbeispiele belegen somit eindrucksvoll die Überlegenheit der Primer und der diese enthaltenden polymerisierbaren Zusammensetzungen auf Thiol-En-Basis gemäß vorliegender Erfindung gegenüber dem Stand der Technik.

## Patentansprüche

1. Polymerisierbare Zusammensetzung auf Thiol-En-Basis, die zumindest einen radikalischen Initiator, Monomere mit zumindest zwei polymerisierbaren C-C-Doppelbindungen pro Molekül, Polythiole mit zumindest zwei SH-Gruppen pro Molekül sowie Primer zur Verbesserung der Haftung auf einem mit der Zusammensetzung zu beschichtenden Substrat umfasst, wobei die Primer pro Molekül jeweils zumindest eine polymerisierbare C-C-Doppelbindung, zumindest eine haftungsvermittelnde Gruppierung und eine dazwischen liegende, zumindest zweiwertige Kohlenwasserstoff-Gruppierung als Spacer aufweisen,
**dadurch gekennzeichnet, dass**
a) die polymerisierbaren C-C-Doppelbindungen der Primer 5-Norbornen-2-yl-Gruppen sind; und
b) die haftungsvermittelnden Gruppierungen der Primer aus Phosphonsäure-Gruppen (HO)₂P(=O)- und 3,4-Dihydroxyphenyl-Gruppen ausgewählt sind.

2. Verwendung von Verbindungen, die pro Molekül jeweils zumindest eine polymerisierbare C-C-Doppelbindung, zumindest eine haftungsvermittelnde Gruppierung und eine dazwischen liegende, zumindest zweiwertige Kohlenwasserstoff-Gruppierung als Spacer aufweisen, als Primer in einer polymerisierbaren Zusammensetzung auf Thiol-En-Basis zur Verbesserung der Haftung der Zusammensetzung auf einem damit zu beschichtenden Substrat, wobei die Zusammensetzung weiters zumindest einen radikalischen Initiator, Monomere mit zumindest zwei polymerisierbaren C-C-Doppelbindungen pro Molekül und Polythiole mit zumindest zwei SH-Gruppen pro Molekül umfasst,
**dadurch gekennzeichnet, dass**
die Primer
a) als polymerisierbare C-C-Doppelbindungen 5-Norbornen-2-yl-Gruppen und
b) aus Phosphonsäure-Gruppen und 3,4-Dihydroxyphenyl-Gruppen ausgewählte Gruppen als haftungsvermittelnde Gruppierungen
umfassen.

3. Zusammensetzung nach Anspruch 1 oder Verwendung nach Anspruch 2, **dadurch gekennzeichnet, dass** die Primer jeweils zumindest zwei 5-Norbornen-2-yl-Gruppen und/oder zumindest zwei haftungsvermittelnde Gruppierungen umfassen, wobei die Primer gegebenenfalls jeweils zwei 5-Norbornen-2-yl-Gruppen und eine oder zwei haftungsvermittelnde Gruppierungen umfassen.

4. Zusammensetzung oder Verwendung nach einem der Ansprüche 1 bis 3, **dadurch gekennzeichnet, dass** die haftungsvermittelnden Gruppierungen der Primer Phosphonsäure-Gruppen sind.

5. Zusammensetzung oder Verwendung nach einem der Ansprüche 1 bis 4, **dadurch gekennzeichnet, dass** die Spacer-Gruppierung der Primer eine Kettenlänge von zumindest 3 oder zumindest 6 Kohlenstoffatomen aufweist, von denen eines oder mehrere gegebenenfalls durch jeweils ein aus O, S und N ausgewähltes Heteroatom ersetzt sind.

6. Zusammensetzung oder Verwendung nach einem der Ansprüche 1 bis 5, **dadurch gekennzeichnet, dass** die Spacer-Gruppierung der Primer eine Kettenlänge von nicht mehr als 12 oder nicht mehr als 10 Kohlenstoffatomen aufweist, von denen eines oder mehrere gegebenenfalls durch jeweils ein aus O, S und N ausgewähltes Heteroatom ersetzt sind.

7. Zusammensetzung oder Verwendung nach einem der Ansprüche 1 bis 6, **dadurch gekennzeichnet, dass** die Monomere aus Verbindungen mit zumindest zwei Allyl- oder Vinylester-Gruppen, gegebenenfalls aus 1,3,3-Triallyl-1,3,5-triazin-2,4,6-trion (TATATO) und O,O'-(Hexahydrofuro[3,2-b]furan-3,6-diyl)divinyladipat (Glucitoldivinyladipat, GDVA), ausgewählt sind.

8. Zusammensetzung oder Verwendung nach Anspruch 7, **dadurch gekennzeichnet, dass** die Monomere 1,3,3-Triallyl-1,3,5-triazin-2,4,6-trion (TATATO) sind oder umfassen.

9. Zusammensetzung oder Verwendung nach einem der Ansprüche 1 bis 8, **dadurch gekennzeichnet, dass** die Polythiole aus Verbindungen mit zumindest drei SH-Gruppen pro Molekül, gegebenenfalls aus Tris[2-(3-mercaptopropionyloxy)ethyl]-isocyanurat (TEMPIC) und Trimethylolpropan-tris(3-mercaptopropionat) (TMPMP), ausgewählt sind.

10. Zusammensetzung oder Verwendung nach Anspruch 9, **dadurch gekennzeichnet, dass** die Polythiole Tris[2-(3-mercaptopropionyloxy)ethyl]isocyanurat (TEMPIC) sind oder umfassen.

11. Zusammensetzung oder Verwendung nach einem der Ansprüche 1 bis 10, **dadurch gekennzeichnet, dass** die Zusammensetzung als Ein-Komponenten-Zusammensetzung vorliegt.

12. Zusammensetzung oder Verwendung nach einem der Ansprüche 1 bis 10, **dadurch gekennzeichnet, dass** die Zusammensetzung als Zwei-Komponenten-Zusammensetzung vorliegt, wovon nur die erste Komponente die Primer umfasst.

13. Zusammensetzung oder Verwendung nach einem der Ansprüche 1 bis 12, **dadurch gekennzeichnet, dass** die Zusammensetzung etwa 0,1 bis etwa 5 Gew.-% radikalischen Initiator, etwa 10 bis etwa 50 Gew.-% Monomere, etwa 40 bis etwa 80 Gew.-% Polythiole und etwa 5 bis etwa 25 Gew.-% Primer jeweils in solchen Mengen umfasst, dass die Summe 100 Gew.-% ergibt.

14. Zusammensetzung oder Verwendung nach Anspruch 13 in Abhängigkeit von Anspruch 11, **dadurch gekennzeichnet, dass** die Zusammensetzung etwa 0,5 bis etwa 2 Gew.-% radikalischen Initiator, etwa 40 bis etwa 60 Gew.-% Monomere, etwa 60 bis etwa 80 Gew.-% Polythiole und etwa 10 bis etwa 20 Gew.-% Primer jeweils in solchen Mengen umfasst, dass die Summe 100 Gew.-% ergibt.

15. Zusammensetzung oder Verwendung nach einem der Ansprüche 1 bis 14, **dadurch gekennzeichnet, dass** der radikalische Initiator ein radikalischer Photoinitiator ist und die Zusammensetzung photopolymerisierbar ist.

16. Zusammensetzung oder Verwendung nach Anspruch 15 in Abhängigkeit von Anspruch 11, **dadurch gekennzeichnet, dass** die Zusammensetzung in einem Schritt auf das zu beschichtende Substrat aufbringbar und anschließend durch Bestrahlung härtbar ist.

17. Zusammensetzung nach einem der Ansprüche 1 bis 16 zur Verwendung als Knochenkleber.

18. Norbornen-Derivat der nachstehenden Formel
(Norb)ₙ-Sp-(AM)ₘ
worin
Norb für einen 5-Norbornen-2-yl-Rest steht;
AM jeweils unabhängig für eine Phosphonsäure-Gruppe (HO)₂P(=O)- oder eine 3,4-Dihydroxyphenyl-Gruppe steht;
n und m jeweils unabhängig für eine ganze Zahl ≥ 1 stehen; und
Sp für eine Spacer-Gruppe steht, die aus (n+m)-wertigen Kohlenwasserstoff-Resten mit 1 bis 40 Kohlenstoffatomen ausgewählt ist, von denen eines oder mehrere gegebenenfalls durch jeweils ein aus O, S und N ausgewähltes Heteroatom ersetzt sind.

19. Norbornen-Derivat nach Anspruch 18, **dadurch gekennzeichnet, dass**
a) n und m jeweils unabhängig für 1 oder 2 stehen; und/oder
b) S für einen (n+m)-wertigen Kohlenwasserstoff-Rest mit 3 bis 20 oder 6 bis 16 Kohlenstoffatomen steht, von denen eines oder mehrere gegebenenfalls durch jeweils ein aus O, S und N ausgewähltes Heteroatom ersetzt sind; und/oder
c) AM jeweils für eine Phosphonsäure-Gruppe (HO)₂P(=O)- steht.

20. Norbornen-Derivat nach Anspruch 18 oder 19, **dadurch gekennzeichnet, dass** es aus den folgenden Verbindungen ausgewählt ist:
N-(4-Hydroxy-4,4-diphosphonobutyl)-5-norbornen-2-carbonsäureamid-Natriumsalz (N-(4-Hydroxy-4,4-diphosphonobutyl)alendronsäureamid-Natriumsalz) (Alendronat-Norb) (B1): 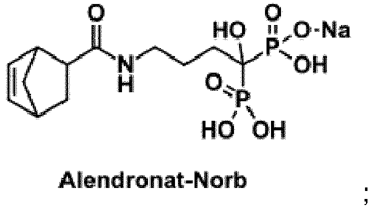
N-[2-(3,4-Dihydroxyphenyl)ethyl]-5-norbornen-2-carbonsäureamid (Catechol-2C-Norb) (B2): 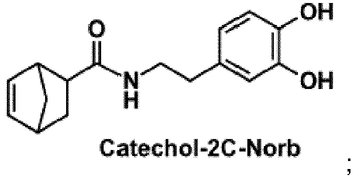
5-Norbornen-2-ylcarbonyloxymethylphosphonsäure (Phn-1C-Norb) (B3): 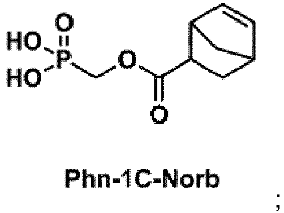
6-(5-Norbornen-2-ylcarbonyloxy)hexylphosphonsäure (Phn-6C-Norb) (B4): 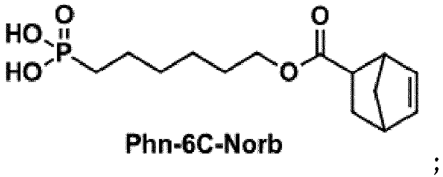
6-(2,3-Bis(5-norbornen-2-ylcarbonyloxy)propylthio)hexylphosphonsäure (Phn-6C-bisNorb) (B5): 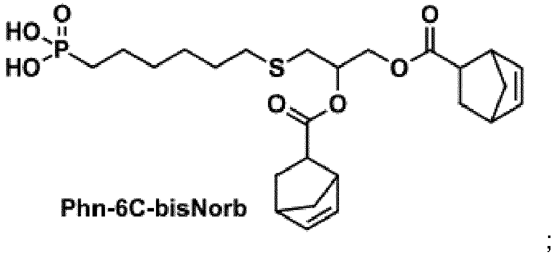
6-(2,3-Bis(5-norbornen-2-ylcarbonyloxy)propylthio)hexan-1,1-diyl-bis(phosphonsäure) (bisPhn-6C-bisNorb) (B6): 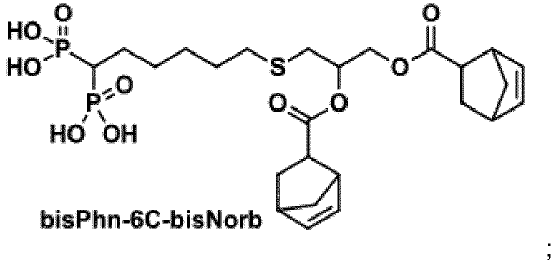
3-(3-(3,4-Dihydroxyphenyl)propylthio)propan-1,2-diyl-bis(norbornen-2-carboxylat) (Catechol-3C-bisNorb) (B7): 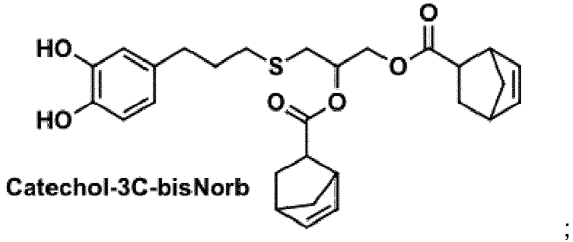
Ethylendiamintetraessigsäure-bis(2-(3,4-dihydroxyphenyl)ethyl)amid-bis(2-(5-norbornen-2-yl)ethyl)amid (bisCatechol-EDTA-bisNorb) (B8): 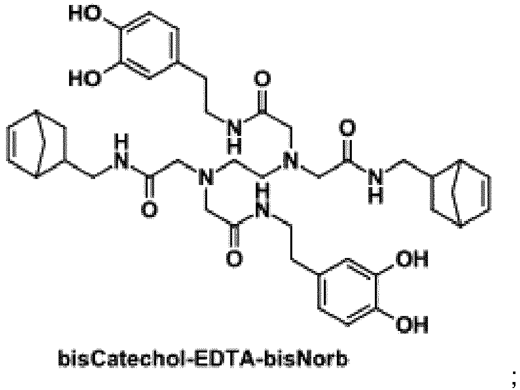
Diethylentriaminpentaessigsäure-bis(2-(5-norbornen-2-yl)ethyl)amid-tris(2-(3,4-dihydroxyphenyl)ethyl)amid (triCatechol-DTPA-bisNorb) (B9):

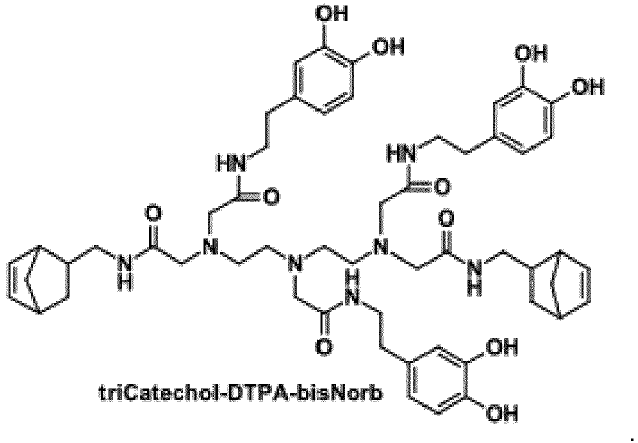

21. Verwendung von Norbornen-Derivaten nach einem der Ansprüche 18 bis 20 als Primer in polymerisierbaren Zusammensetzungen auf Thiol-En-Basis nach einem der Ansprüche 1 bis 17.
